# EUROPEAN PATENT SPECIFICATION

(11) **EP 3 288 594 B1**
(45) Date of publication and mention of the grant of the patent: **29.06.2022**
(21) Application number: 16720663.0
(22) Date of filing: 26.04.2016
(51) Int. Cl.: A61K 48/00, C12N 15/11, A61P 1/16, C12N 15/90, C12N 15/86, C12N 9/22, C12N 15/113

(54) **DUAL AAV VECTOR SYSTEM FOR CRISPR/CAS9 MEDIATED CORRECTION OF HUMAN DISEASE**
DUALES AAV-VEKTORSYSTEM FÜR CRISPR-/CAS9-VERMITTELTE KORREKTUR VON MENSCHLICHEN ERKRANKUNGEN
SYSTÈME DE VECTEUR AAV DOUBLE POUR LA CORRECTION MÉDIÉE PAR CRISPR/CAS9 D'UNE MALADIE HUMAINE

(30) Priority: 27.04.2015 US 201562153470 P; 24.06.2015 US 201562183825 P; 12.11.2015 US 201562254225 P; 27.01.2016 US 201662287511 P
(43) Date of publication of application: 07.03.2018
(73) Proprietor: The Trustees of The University of Pennsylvania, Philadelphia, PA 19104 (US)
(72) Inventor: WILSON, James, M., Philadelphia, PA 19103 (US); WANG, Lili, Phoenixville, PA 19460 (US); YANG, Yang, Qingyang District, Chengdu, Sichuan 610091 (CN)
(74) Representative: Appleyard Lees IP LLP
(86) International application number: PCT/US2016/029330
(87) International publication number: WO 2016/176191

(56) References cited:
- WO-A1-98/42727
- WO-A1-2014/204726
- WO-A2-2015/191693
- LI H ET AL: "In vivo genome editing restores haemostasis in a mouse model of haemophilia", NATURE, NATURE PUBLISHING GROUP, UNITED KINGDOM, vol. 475, no. 7355, 14 July 2011 (2011-07-14), pages 217-221, XP002688791, ISSN: 0028-0836, DOI: 10.1038/NATURE10177 [retrieved on 2011-06-26]
- SENÍS E ET AL: "CRISPR/Cas9-mediated genome engineering: an adeno-associated viral (AAV) vector toolbox", BIOTECHNOLOGY JOURNAL, WILEY-VCH VERLAG, WEINHEIM, DE, vol. 9, no. 11, Sp. Iss. SI, November 2014 (2014-11), pages 1402-1412, XP002737562, ISSN: 1860-6768, DOI: 10.1002/BIOT.201400046 [retrieved on 2014-10-06]
- SANDS MARK S: "AAV-mediated liver-directed gene therapy", METHODS IN MOLECULAR BIOLOGY (CLIFTON, N.J.)UNITED STATES2010,, vol. 807, 2011, pages 141-157, XP009167845, ISSN: 1940-6029, DOI: 10.1007/978-1-61779-370-7_6
- DAVID COX ET AL: "Therapeutic genome editing: prospects and challenges", NATURE MEDICINE., vol. 21, no. 2, February 2015 (2015-02), pages 121-131, XP055285107, US ISSN: 1078-8956, DOI: 10.1038/nm.3793
- YANG YANG ET AL: "A dual AAV system enables the Cas9-mediated correction of a metabolic liver disease in newborn mice", NATURE BIOTECHNOLOGY, vol. 34, no. 3, February 2016 (2016-02), pages 334-338, XP055284932, US ISSN: 1087-0156, DOI: 10.1038/nbt.3469

## Description

### STATEMENT REGARDING FEDERALLY SPONSORED RESEARCH OR DEVELOPMENT

This invention was made with government support under a grant from the National Institutes of Health, NICHD P01-HD057247. The US government has certain rights in this invention.

### BACKGROUND OF THE INVENTION

Early intervention and therapy is crucial in many inherited diseases. Various types of therapies have been described in the literature. One technique which has been described as having potential in correction of diseases associated with a genetic mutation or a specific phenotype is the Clustered Regularly Interspaced Short Palindromic Repeats (CRISPR) and CRISPR-associated protein (Cas9) system. CRISPR-Cas was derived from an adaptive immune response defense mechanism used by archaea and bacteria for the degradation of foreign genetic material [Van der Oost, J., et al. 2014. Nat. Rev. Microbiol. 7: 479-492; Hsu, P., et al. 2014. Development and applications of CRISPR-Cas9 for genome editing. Cell 157: 1262-1278]. This mechanism can be repurposed for other functions, including genomic engineering for mammalian systems, such as gene knockout (KO) [Cong, L., et al. 2013. Multiplex genome engineering using CRISPR/Cas systems. Science 339: 819-823; Mali, P., et al. 2013. RNA-guided human genome engineering via Cas9. Science 339: 823-826; Ran, F.A., et al. 2013. Genome engineering using the CRISPR-Cas9 system. Nat. Protoc. 8: 2281-2308; Shalem, O., et al. 2014. Genome-scale CRISPR-Cas9 knockout screening in human cells. Science 343: 84-87]. The CRISPR Type II system is currently the most commonly used RNA-guided endonuclease technology for genome engineering. There are two distinct components to this system: (1) a guide RNA and (2) an endonuclease, in this case the CRISPR associated (Cas) nuclease, Cas9. The guide RNA is a combination of the endogenous bacterial crRNA (CRISPR RNA) and tracrRNA (transactivating crRNA) into a single chimeric guide RNA (gRNA) transcript. The gRNA combines the targeting specificity of crRNA with the scaffolding properties of tracrRNA into a single transcript. When the gRNA and the Cas9 are expressed in the cell, the genomic target sequence can be modified or permanently disrupted.

Adeno-associated viruses have been described as being useful vectors for gene therapy. Such uses include those involving the CRISPR-Cas system. See, *e.g.,* Yin et al, "Biotechnology, 32: 551-3 (2014) and "In vivo interrogation of gene function in the mammalian brain using CRISPR-Cas", Nature Biotechnology, 33: 102-6 (2015).

As stated above, some inherited disorders require very early intervention, *i.e.,* often within hours or days, in order to avoid infant mortality or significant morbidity. However, AAV vectors have been described as being unstable and therefore ineffective when delivered to neonates or infants; this has been attributed to the rapid proliferation of the liver in this stage of life, resulting in loss of the AAV vector by the proliferating cells and/or dilution of AAV-containing cells resulting in inadequate therapeutic outcomes.

What are needed are effective compositions and techniques for treating genetic disorders, and particularly those associated with neonate or infant mortality or significant morbidity in children and adults.

### SUMMARY OF THE INVENTION

The invention is described in the appended claims.

The present invention provides a CRISPR-Cas system delivered via dual AAV vectors to newborn and infant subjects for targeted treatment of a genetic disorder. Advantageously, the system uses the rapid proliferation of the cells in the neonatal stage to populate tissues with the cells corrected by the AAV-mediated treatment while simultaneously diluting the AAV-mediated Cas elements. However, the method is also useful for treatment in older children and in adults for treatment of a variety of disorders, e.g., through targeting of proliferating, progenitor and/or stem cells in developing and adult tissues.

In one aspect, the invention provides a dual vector system for treating disorders, wherein the system comprises: (a) a gene editing AAV vector comprising a Cas9 gene under control of regulatory sequences which direct its expression in a target cell (e.g., a hepatocyte) comprising a targeted gene which has one or more mutations resulting in a disease or disorder (e.g., a liver metabolic disorder); and (b) a targeting AAV vector comprising one or more of sgRNAs and donor template, wherein the sgRNA comprises at least 20 nucleotides which specifically bind to a selected site in the targeted genes and is 5' to a protospacer-adjacent motif (PAM) which is specifically recognized by the Cas9, and wherein the donor template comprises nucleic acid sequences which replaces at least one of the mutations in the targeted gene; wherein the ratio of gene editing vector of (a) to the vector containing template (b) is such that (b) is in excess of (a) and wherein the target cells are hepatocytes or liver cells. In one embodiment, the disorder is a metabolic disorder. In another embodiment, the disorder is a liver metabolic disorder. In one example, both the gene editing AAV vector and the targeting AAV vector have the same capsid.

In one aspect, disclosed herein is a method of treating a liver metabolic disorder in neonates, comprising: co-administering to the subject (a) a gene editing AAV vector comprising a Cas9 gene under control of regulatory sequences which direct its expression in a hepatocyte comprising a targeted gene which has one or more mutations resulting in a liver metabolic disorder; and (b) an AAV targeting vector comprising sgRNA and donor template, wherein the sgRNA comprises at least 20 nucleotides which specifically bind to a selected site in the targeted genes, and wherein the donor template comprises nucleic acid sequences which replaces at least one of the mutations in the targeted gene and which is 5' to a PAM which is specifically recognized by the Cas9; wherein the ratio of gene editing AAV vector of (a) to (b) is such that (b) is in excess of (a). In one example, the liver metabolic disorder is ornithine transcarbamylase deficiency. In another aspect, the invention provides use of these AAV vectors to treat a liver metabolic disorder, or for the preparation of a medicament for the treatment of a liver metabolic disorder.

Also disclosed, is that the method is useful for treating genetic diseases in non-liver tissues by correcting point mutations by gene editing. This method allows for a single gene editing vector that simultaneously targets to different sites in order to increase the efficiency of the disease correction. This gene editing vector is co-administered to a subject with Cas9, and allows correction point mutations, as well as small deletions and insertions in the targeted cells. Suitable target tissues may include, e.g., gut epithelium, lung epithelium, hepatocytes, retina (e.g., retina epithelia), muscle and central nervous system progenitor cells.

In yet another aspect, method is useful for treating genetic diseases by inserting an entire gene cassette upstream of the targeted intron in a site-specific manner and downstream of the natural regulatory elements. This method is advantageous because it is independent from the specific location of a mutation. An exon or a large deletion or insertion can also be corrected by this method if an insertion cassette is followed by a donor site and is targeted to a donor site of the defective exon, so that gene correction will be achieved at the level of spliced mRNA. Coding sequences, as well as splice donor and acceptor sites can be corrected by the methods described herein.

Still other aspects and advantages of the invention will be readily apparent from the following detailed description of the invention.

### BRIEF DESCRIPTION OF THE DRAWINGS

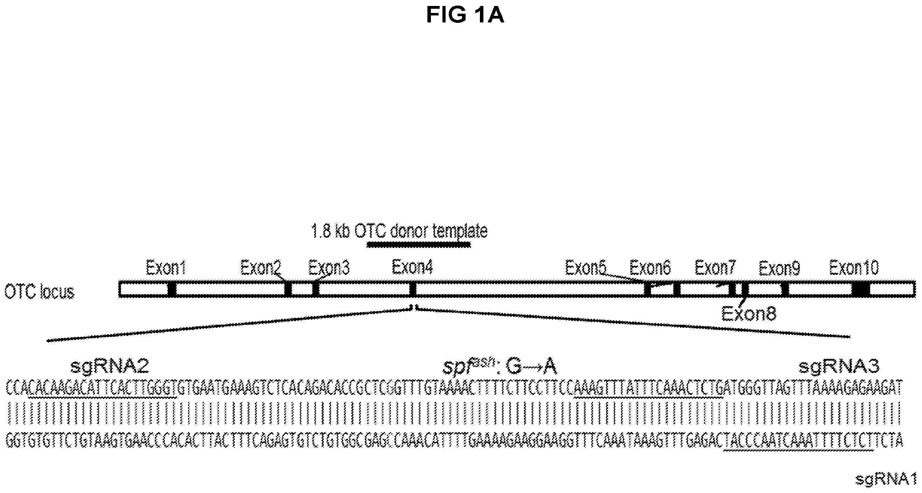

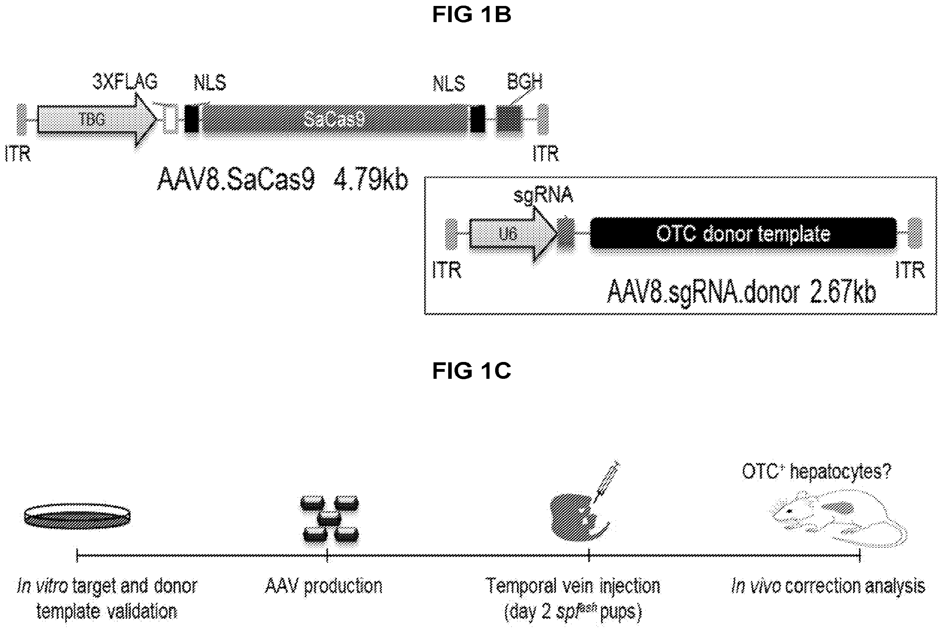

FIGs 1A - 1C illustrate the results of a study showing *in vivo* gene correction of the *OTC* locus in the *spf^{ash}* mouse liver by AAV.CRISPR-SaCas9. FIG. 1A is a schematic diagram of the mouse OTC locus showing the *spf^{ash}* mutation and three SaCas9 targets. *spf^{ash}* has a G to A mutation at the donor splice site at the end of exon 4 indicated on the top strand. The nucleotide sequence reflected is SEQ ID NO: 1. The three selected SaCas9-targeted genomic loci (20 bp each) are in lighter gray and underlined with the PAM sequences marked. The black line above exon 4 indicates the 1.8 kb OTC donor template. FIG. 1B is a cartoon showing the dual AAV vector system for liver-directed and SaCas9-mediated gene correction. The AAV8.sgRNA1.donor vector contains a 1.8 kb murine OTC donor template sequence as shown in FIG. 1A with the corresponding PAM sequence mutated. FIG. 1C is a flowchart showing key steps of AAV8.CRISPR-SaCas9-mediated gene correction in the neonatal OTC *spf^{ash}* model.

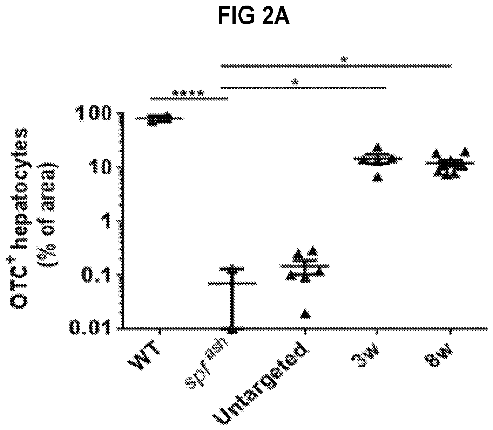

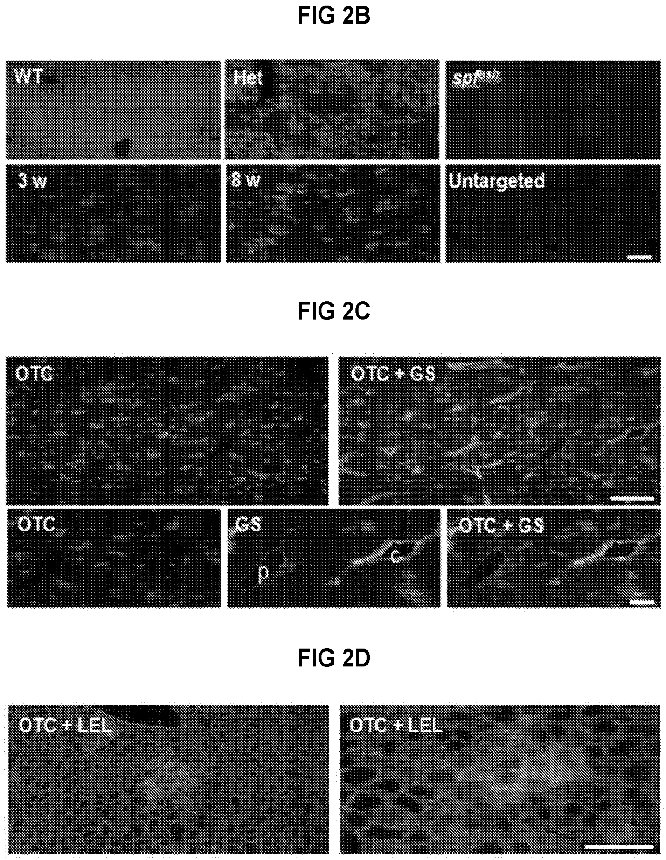

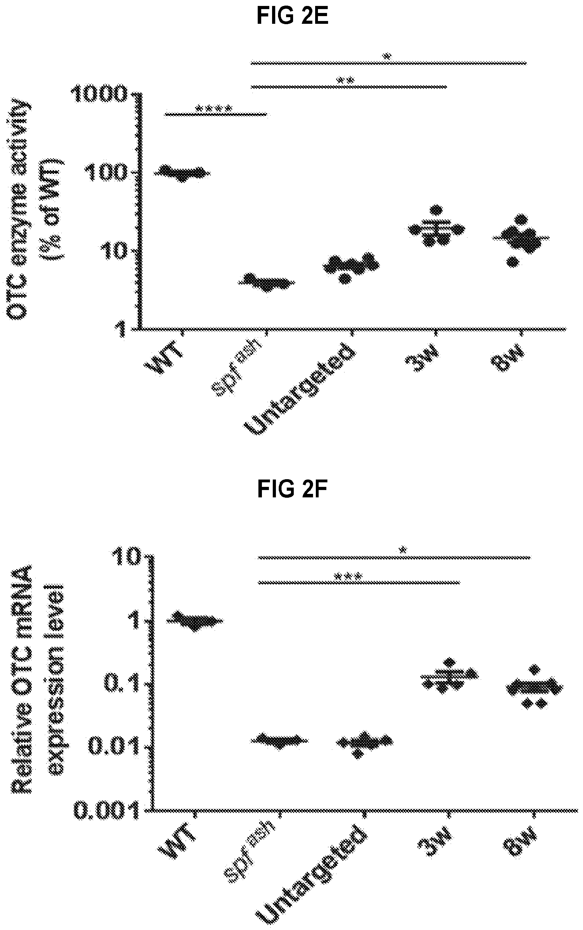

FIG. 2A - 2F illustrate efficient restoration of OTC expression in the liver of *spf^{ash}* mice by AAV8.CRISPR-SaCas9-mediated gene correction. AAV8.SaCas9 (5×10¹⁰ GC/pup) and AAV8.sgRNA1.donor (5×10¹¹ GC/pup) were administered to postnatal day 2 (p2) *spf^{ash}* pups via the temporal vein. *spf^{ash}* mice were sacrificed at 3 (n=5) or 8 weeks (n=8) after treatment. Untargeted *spf^{ash}* mice received AAV8.SaCas9 (5×10¹⁰ GC/pup) and AAV8.control.donor (5×10¹¹ GC/pup) at p2, and livers were harvested 8 weeks post treatment (n=6). FIG 2A provides the quantification of gene correction based on the percentage of area on liver sections expressing OTC by immunostaining. FIG 2B illustrates immunofluorescence staining with antibodies against OTC on liver sections from *spf^{ash}* mice treated with the dual AAV vectors for CRISPR-SaCas9-mediated gene correction at 3 weeks and 8 weeks. Stained areas typically represent clusters of corrected hepatocytes. Untreated controls show livers from wild type (wt), *spf^{ash}* heterozygous (het), and *spf^{ash}* hemizygous mice. Scale bar, 100µm. FIG 2C illustrates random distribution of clusters of corrected hepatocytes along the portal-central axis shown by double immunostaining against OTC and glutamine synthetase (GS) as a pericentral marker (p, portal vein; c, central vein). Scale bars, 300 µm (upper panel) and 100 µm (lower panel). FIG 2D shows groups of corrected hepatocytes expressing OTC shown by immunofluorescence on sections counterstained with fluorescein-labeled tomato lectin (Lycopersicon esculentum lectin, LEL) which outlines individual hepatocytes. Scale bar, 50µm. FIG 2E shows OTC enzyme activity in the liver lysate of *spf^{ash}* mice at 3 and 8 weeks following dual vector treatment. FIG 2F shows quantification of OTC mRNA levels in the liver by RT-qPCR using primers spanning exons 4-5 to amplify wild-type OTC. Mean ± SEM are shown. ^{∗} *P* < 0.05, ^{∗∗} *P* < 0.01, ^{∗∗∗∗} *P* < 0.0001, Dunnett's test.

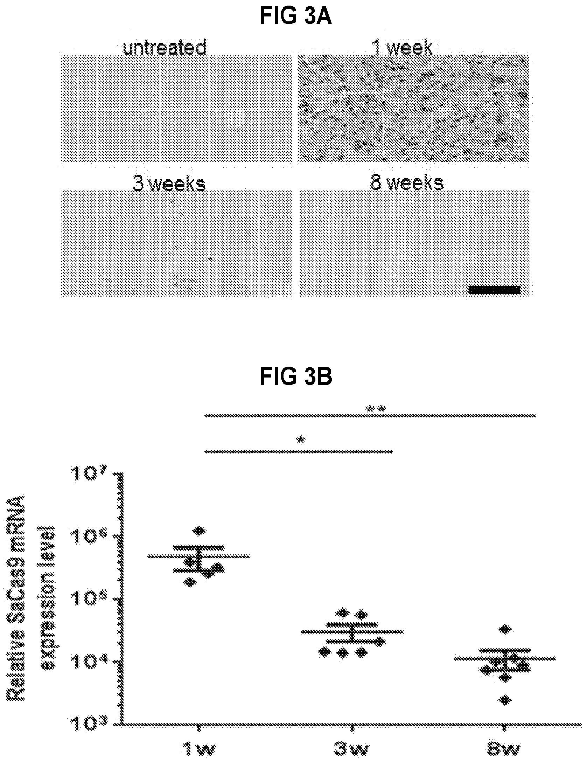

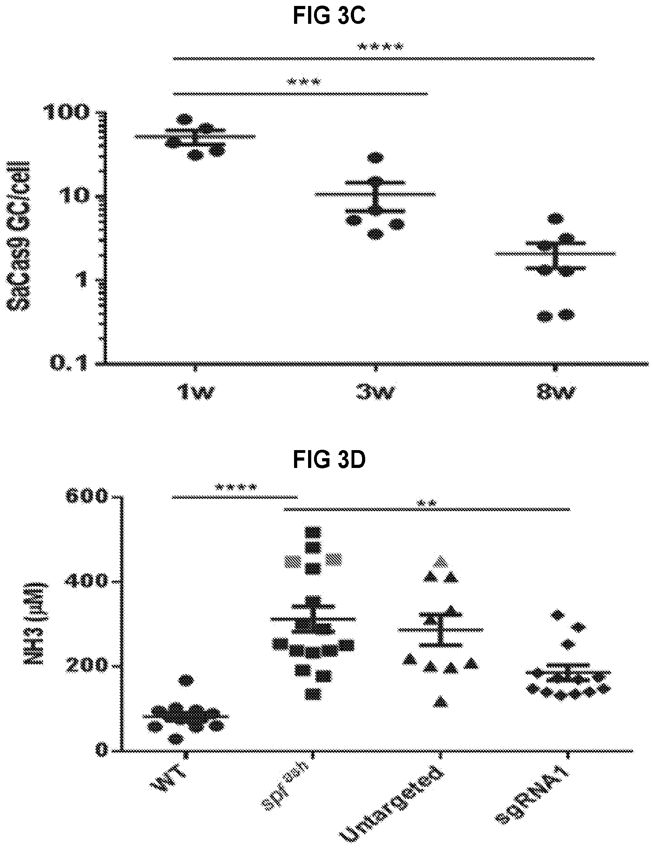

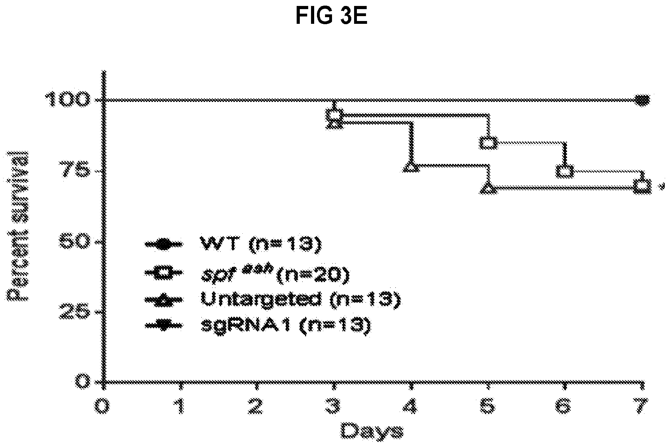

FIGS 3A-3E shows the time course of SaCas9 expression following neonatal vector administration and functional improvement following high protein diet challenge. FIG 3A shows the results of immunostaining with antibodies against Flag on liver sections from an untreated mouse or treated *spf^{ash}* mice at 1, 3, or 8 weeks following neonatal injection of the dual AAV vectors for CRISPR-SaCas9-mediated gene correction. AAV8.SaCas9 (5×10¹⁰ GC/pup) and AAV8.sgRNA1.donor (5×10¹¹ GC/pup) were administered to p2 *spf^{ash}* pups via the temporal vein. Nuclear staining of Flag-tagged SaCas9 were abundant at 1 week (n=5) but dramatically reduced at 3 weeks (n=6) and became scarce at 8 weeks (n=7) after vector injection. Scale bar, 100 µm. FIG 3B shows the results of quantification of SaCas9 mRNA levels in liver by RT-qPCR. Mean ± SEM are shown. ^{∗} *P* < 0.05, ^{∗∗} *P* < 0.01, ^{***} *P* < 0.001, ^{∗∗∗∗} *P* < 0.0001, Dunnett's test. FIG 3C provides quantification of SaCas9 vector genome in liver by QPCR. FIG. 3D shows plasma ammonia levels in control or dual AAV vector-treated *spf^{ash}* mice after a one-week course of high protein diet. Seven weeks following neonatal treatment with the dual AAV vectors, mice were given high protein diet for 7 days. Plasma ammonia levels were measured 7 days after the high protein diet. Plasma ammonia levels in WT mice (n=13) and AAV8.SaCas9 + AAV8.sgRNA1.donor-treated *spf^{ash}* mice (n=13) were significantly lower than untreated *spf^{ash}* mice (n=16) after a 7-day high protein diet. Shaded quares indicate samples obtained from moribund untreated *spf^{ash}* mice 6 days after high protein diet; shaded triangle indicates sample obtained from a moribund *spf^{ash}* mouse treated with untargeted vector (AAV8.control.donor with no sgRNA1, n=10) 5 days after high protein diet. ^{∗∗} *P*< 0.01, ^{∗∗∗∗} *P*< 0.0001, Dunnett's test. FIG. 3E shows survival curves in control or dual AAV vector-treated spfash mice after a one-week course of high protein diet. Untreated *spf^{ash}* mice (n=20) or *spf^{ash}* mice treated with untargeted vectors (AAV8.control.donor, n=13) started to die 3 days after high protein diet. All WT (n=13) and AAV8.SaCas9 + AAV8.sgRNA1.donor-treated mice (n=13) survived. ^{∗} *P*< 0.05, Mantel-Cox test.

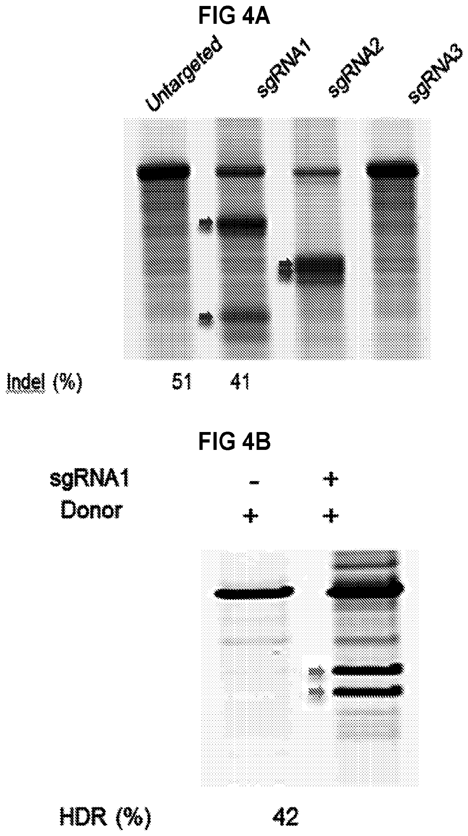

FIGs 4A and 4B show *in vitro* validation of OTC sgRNAs and donor template. FIG 4A shows *in vitro* validation of sgRNAs targeted to OTC in MC57G mouse cell line by transient transfection followed by 4-day puromycin enrichment and SURVEYOR^{®} nuclease assay. sgRNA1 showed the highest efficiency in inducing indels in the targeted loci and was therefore chosen for subsequent studies. Arrows denote SURVEYOR^{®} nuclease cleaved fragments of the OTC PCR products. Results were replicated in 2 independent experiments. FIG 4B shows *in vitro* validation of OTC donor template. MC57G cells were transiently transfected with a plasmid co-expressing OTC sgRNA1, SaCas9, and an *Age*I restriction site tagged OTC donor plasmid followed by 4-day puromycin enrichment. RFLP analysis was performed following *Age*I digestion to detect HDR *in vitro.* Co-transfection of the *Age*I-tagged OTC donor template with an SaCas9 plasmid without OTC sgRNA1 did not result in detectable HDR. Arrows denote *Age*I-sensitive cleavage products resulting from HDR. Results were replicated in 2 independent experiments. Indel and HDR frequency were calculated based on band intensities [L. Wang et al, Hum Gene Ther, 23: 533-539 (2012)].

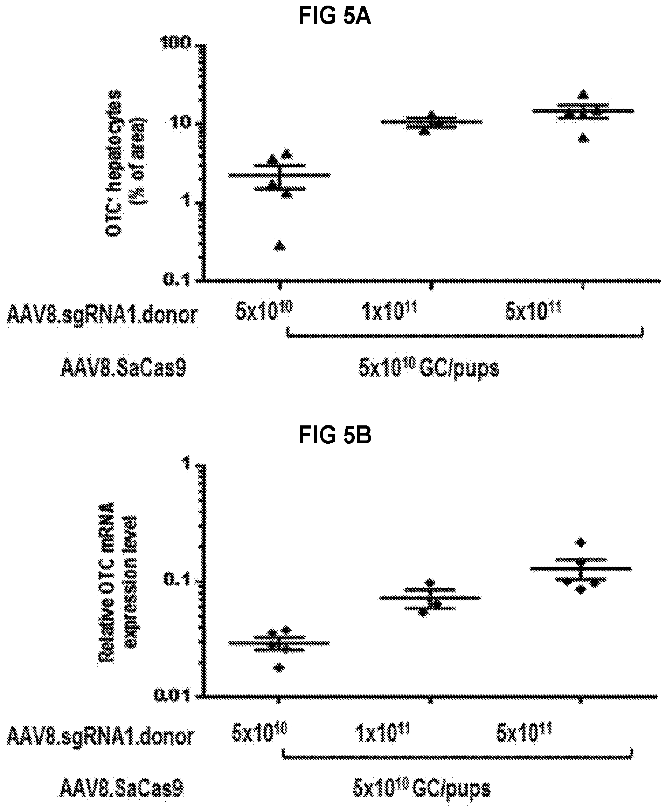

FIGS 5A and 5B show vector dose optimization to improve *in vivo* gene correction. Postnatal day 2 *spf^{ash}* pups received temporal vein injection of 5×10¹⁰ GC AAV8.SaCas9 and either 5×10¹⁰ (n=5), 1×10¹¹ (n=3), or 5×10¹¹ (n=5) GC of AAV8.sgRNA1.donor vector. Liver samples were collected 3 weeks post vector treatment for analysis. FIG 5A shows quantification of gene correction based on the percentage of area on liver sections expressing OTC by immunostaining. FIG 5B shows quantification of OTC mRNA levels in the liver by RT-qPCR using primers spanning exons 4-5 to amplify wild-type OTC. Mean ± SEM are shown. ^{∗∗} *P*<0.01, Dunnett's test.

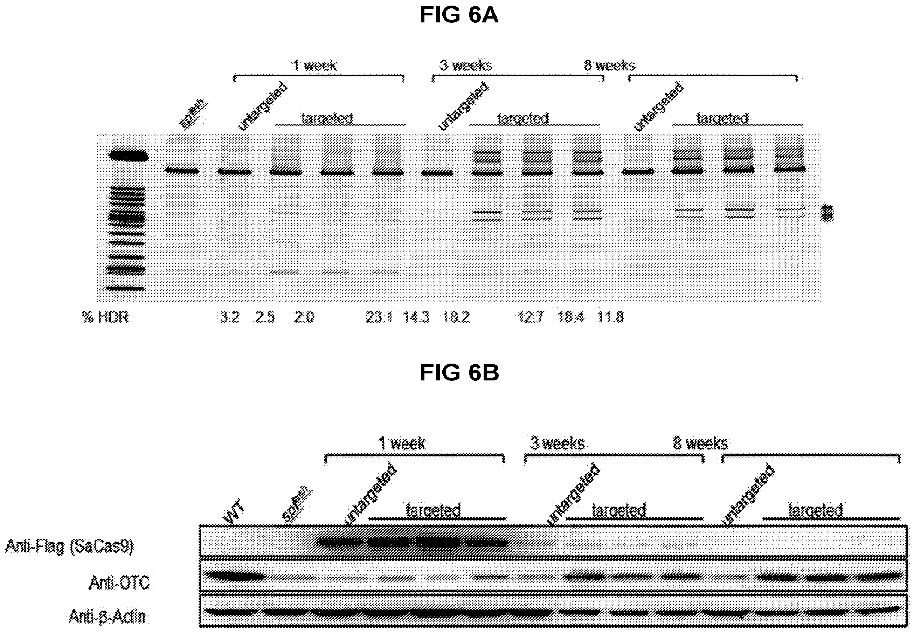

FIGs. 6A and 6B show the time course of gene expression by Western analysis and HDR analysis by RFLP. FIG 6A provides HDR analysis by RFLP. OTC target region was PCR amplified from the liver genomic DNA isolated from untreated WT and *spf^{ash}* mice or *spf^{ash}* mice treated with the dual AAV vectors. Untreated WT and *spf^{ash}* control samples were collected at 8 weeks of age; samples from the treated *spf^{ash}* mice were collected at 1, 3, and 8 weeks (n=3 animals per time point) following neonatal injection of the dual AAV8 vectors. Targeted animals received AAV8.SaCas9 (5×10¹⁰ GC/pup) and AAV8.sgRNA1.donor (5×10¹¹ GC/pup); untargeted animals received AAV8.SaCas9 (5×10¹⁰ GC/pup) and AAV8.control.donor (5×10¹¹ GC/pup). *AgeI* digestion was performed and estimated HDR percentages are shown. FIG 6B shows western analysis. Liver lysates were prepared from untreated WT and *spf^{ash}* mice or *spf^{ash}* mice treated with the dual AAV vectors for detection of Flag-SaCas9 and OTC protein.

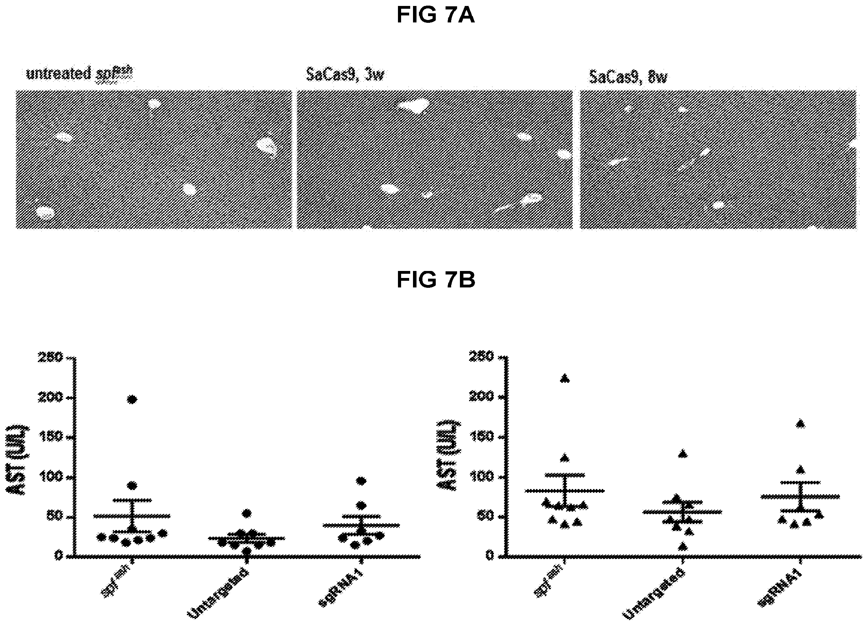

FIGs. 7A and 7B show the examination of liver toxicity in animals treated with AAV8.CRISPR-SaCas9 dual vectors. FIG 7A shows results of histological analysis on livers harvested 3 and 8 weeks following the dual vector treatment. FIG 7B shows liver transaminase levels in untreated *spf^{ash}* mice (n=9) or 8 weeks following dual vector treatment. Untargeted mice received 5×10¹⁰ GC AAV8.SaCas9 and 5×10¹¹ of AAV8.control.donor vectors (n=8), while gene-targeted mice received 5×10¹⁰ GC AAV8.SaCas9 and 5×10¹¹ GC of AAV8.sgRNA1.donor (n=7). Mean ± SEM are shown. There were no statistically significant differences between groups, Dunnett's test.

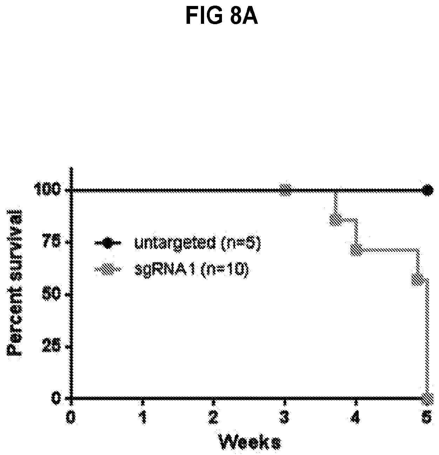

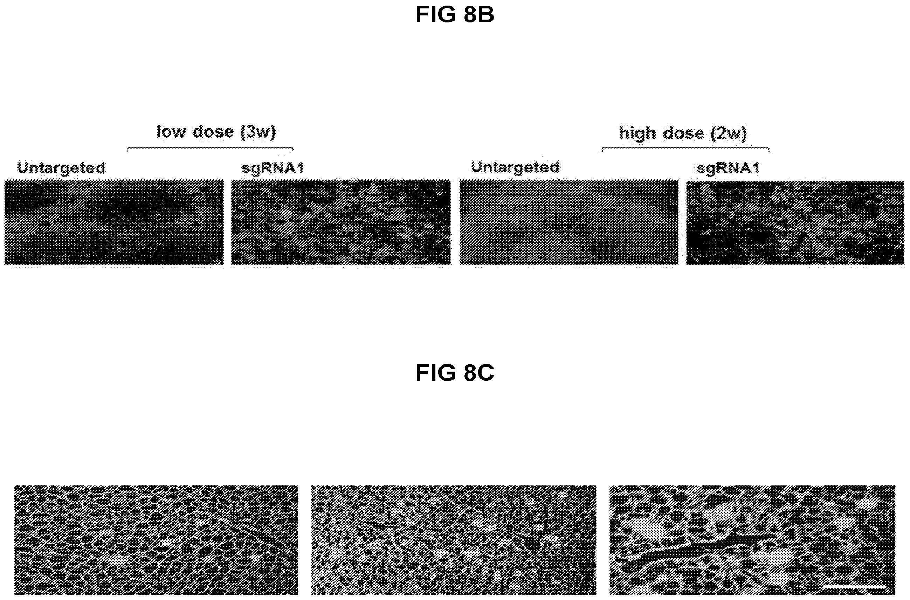

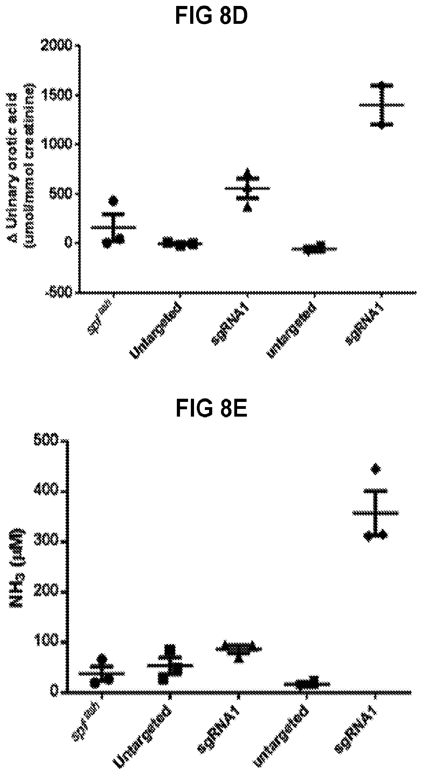

FIG 8A - 8E illustrate gene targeting/correction in the liver of spfash mice treated as adults by AAV8.CRISPR-SaCas9 vectors. Adult spf^{ash} mice (8-10 weeks old) received an intravenous injection of AAV8.SaCas9 (1×10¹¹ GC) and AAV8.sgRNA1.donor (1×10¹² GC), or higher dose of AAV8.SaCas9 (1×10¹² GC) and AAV8.sgRNA1.donor (5×10¹² GC), or untargeted vectors at the equivalent doses. 8A. Survival curve of the low-dose cohorts: sgRNA1 (n=10) or untargeted vector at the same dose (n=5). 8B. Immunofluorescence staining with antibodies against OTC on liver sections collected at 3 (low-dose, n=3) or 2 weeks (high-dose, n=3) after injection. Stained cells typically showed as single corrected hepatocytes. 8C. Isolated corrected hepatocytes expressing OTC shown by immunofluorescence on sections co-stained with fluorescein-labeled tomato lectin (LEL) which outlines individual hepatocytes. Scale bar, 50µm. 8D. Change of urine orotic acid levels in adult spf^{ash} mice after treatment with high-dose gene targeting vectors (n=3 for untreated spf^{ash} and low-dose groups; n=2 for high-dose groups). 8E. Elevation of plasma NH₃ levels in adult spf^{ash} mice after treatment with high-dose gene targeting vectors (n=3 for each group). Mean ± SEM are shown. ^{∗∗∗} P < 0.001, ^{∗∗∗∗} P < 0.0001, Dunnett's test.

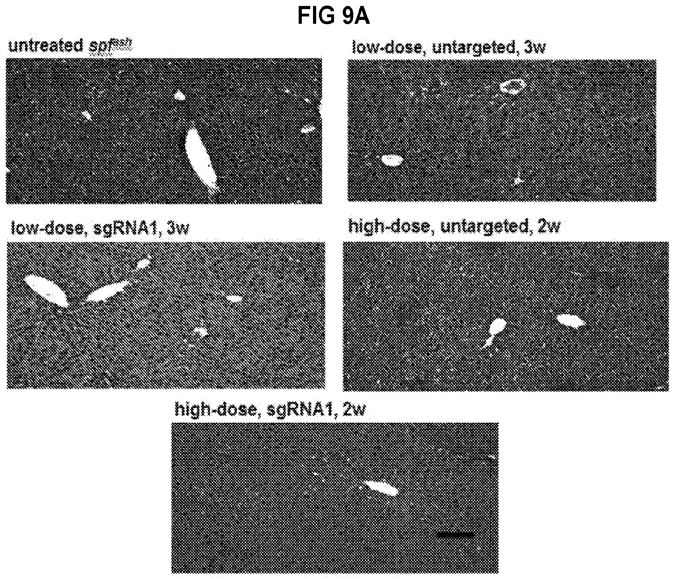

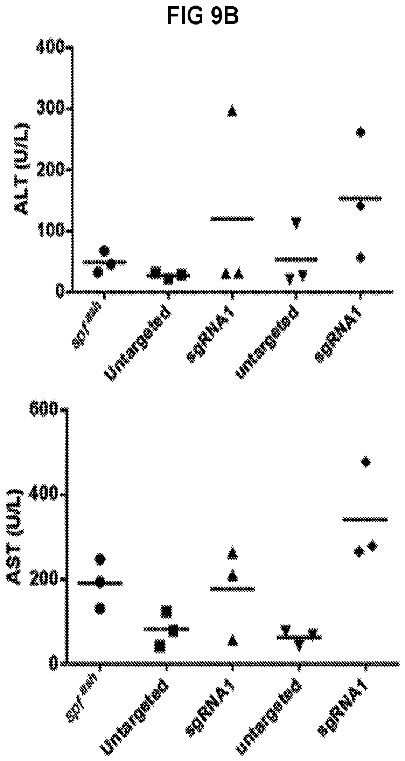

FIGS 9A and 9B illustrate examination of liver toxicity in adult animals treated with AAV8.CRISPR-SaCas9 dual vectors. FIG 9A shows the histological analysis on livers harvested 3 weeks (low-dose) or 2 weeks (high-dose) following dual vector treatment. Scale bar, 100 µm. FIG 9B shows liver transaminase levels in untreated *spf^{ash}* mice, or 3 weeks following low-dose dual vector treatment, or 2 weeks following high-dose dual vector treatment (n=3 for each group). Low-dose, untargeted mice received 1×10¹¹ GC AAV8.SaCas9 and 1×10¹² GC of AAV8.control.donor vectors, while low-dose, gene-targeted mice received 1×10¹¹ GC AAV8.SaCas9 and 1×10¹² GC of AAV8.sgRNA1.donor. High-dose, untargeted mice received 1×10¹² GC AAV8.SaCas9 and 5×10¹² GC of AAV8.control.donor vectors, while high-dose, gene-targeted mice received 1×10¹² GC AAV8.SaCas9 and 5×10¹² GC of AAV8.sgRNA1.donor. Mean ± SEM are shown. Adult animals received high-dose, gene-targeted vectors showed a trend of elevated ALT and AST levels, although not statistically different when compared with other groups (Dunnett's test).

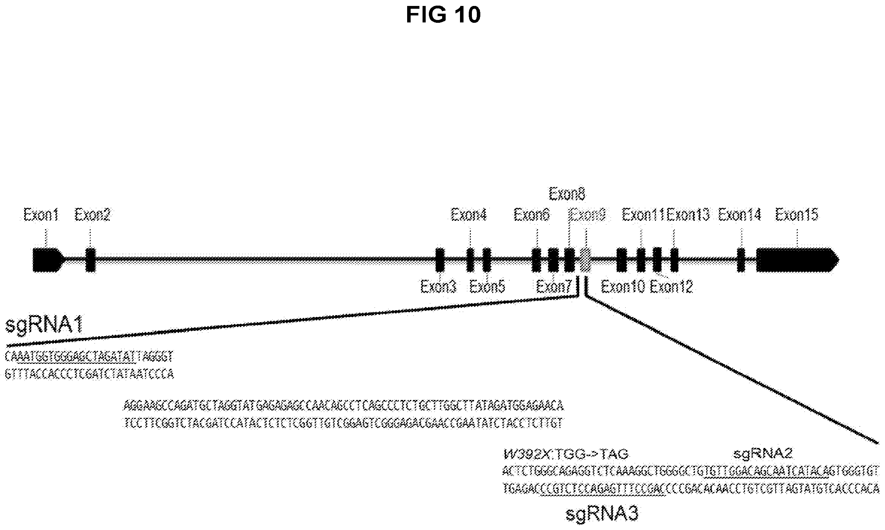

FIG 10 is a cartoon showing the location of sgRNA1, sgRNA2 and sgRNA3 assessed for correction of MPSI as described in Example 2. The nucleotide sequence reflected is SEQ ID NO: 2.

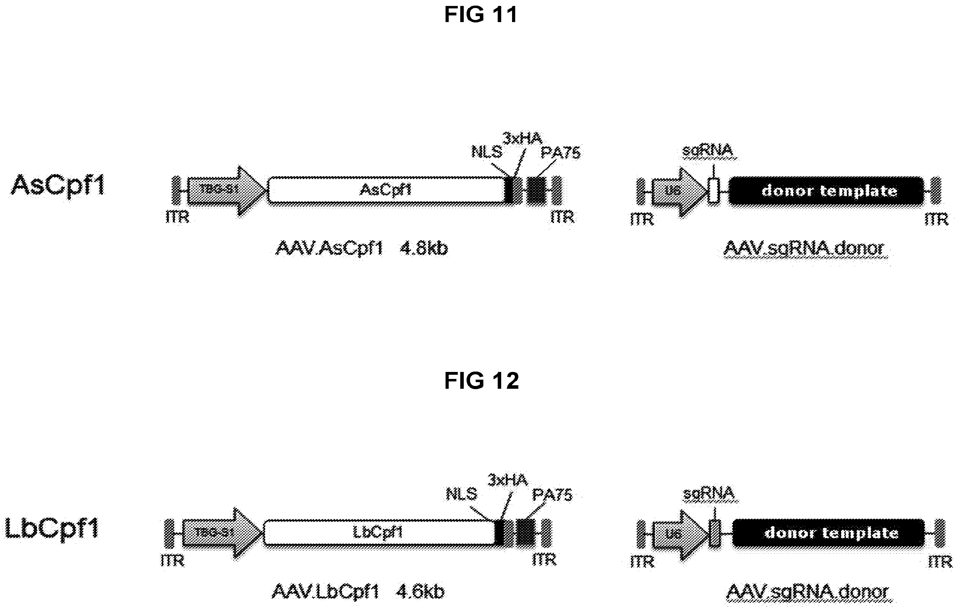

FIG 11 is a cartoon illustrating the dual AAV vector system for liver-directed and AsCpf1-mediated gene correction. The AAV8.sgRNA.donor vector contains a 1.8 kb donor template sequence with the corresponding PAM sequence mutated..
FIG 12 is a cartoon illustrating the dual AAV vector system for liver-directed and LbCpf1-mediated gene correction. The AAV8.sgRNA.donor vector contains a 1.8 kb donor template sequence with the corresponding PAM sequence mutated.

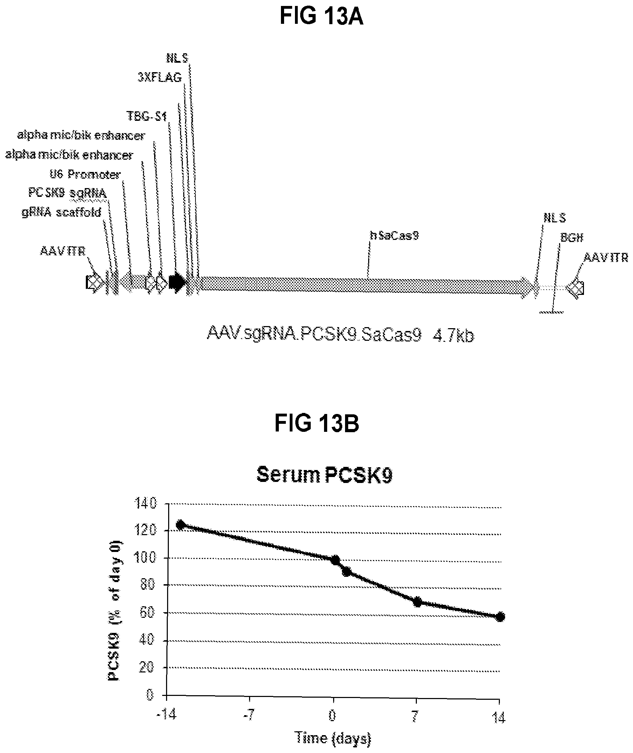

FIG 13A is a cartoon showing the AAV.sgRNA.PCSK9.ScCas9 plasmid. FIG 13B illustrates the serum PCSK9 levels in a rhesus monkey following administration of AAV8.sgRNA.PCSK9.TBG-S1.SaCas9 vector (3×10¹³ GC/kg). Approximately ~40% reduction at day 14 post vector administration is observed compared to day 0.

### DETAILED DESCRIPTION OF THE INVENTION

The disclosure provides a dual vector system to express sgRNA and donor template RNA for precise *in vivo* gene correction of a disease, disorder or condition characterized by a genetic abnormality. This system is particularly well suited for delivery to hepatocytes during the neonatal stage and/or infancy, but may be used in the pre-natal stage, or in older children or adults for targeting other cell types. Examples of other cell types are proliferating, progenitor and/or stem cells in young and adult patients, and optionally, post-mitotic cells. Such cells may include, e.g., epithelial cells (gut, lung, retina, etc), central nervous system (CNS) progenitor cells, among others.

The term "proliferating cells" as used herein refers to cells which multiply or reproduce, as a result of cell growth and cell division. Cells may be naturally proliferating at a desired rate, e.g., epithelial cells, stem cells, blood cells, hepatocytes; in such embodiments, the invention takes advantage of the natural proliferation rate of the cells as described herein. In another embodiment, cells may be proliferating at an abnormal or undesirable rate, e.g., as in cancer cells, or the excessive hyperplastic cell growth associated with the occlusive vascular lesions of atherosclerosis, restenosis post-angioplasty, and graft atherosclerosis after coronary artery bypass. In this embodiment, the invention may either use the proliferation rate of these cells and/or make seek to alter the proliferation rate (e.g., by correcting a genetic abnormality which results in a high growth rate).

As used herein, the term "disorder" or "genetic disorder" is used throughout this specific to refer to any diseases, disorders, or condition associated with an insertion, change or deletion in the amino acid sequence of the wild-type protein. Unless otherwise specified such disorders include inherited and/or non-inherited genetic disorders, as well as diseases and conditions which may not manifest physical symptoms during infancy or childhood.

In general, a neonate in humans may refer to infants from birth to under about 28 days of age; and infants may include neonates and span up to about 1 year of age to up to 2 years of age. The term "young children" may span to up to about 11-12 years of age.

The dual CRISP-Cas vector system provided herein utilizes a combination of two different vector populations co-administered to a subject. These vectors may be formulated together or separately and delivered essentially simultaneously, preferably by the same route. The working examples below describe use of AAV vectors. While the following discussion focuses on AAW vectors, it will be understood that a different, partially or wholly integrating virus (*e.g*., another parvovirus or a lentivirus) may be used in the system in place of the gene editing vector and/or the vector carrying template.

In one example, the dual vector system comprises (a) a gene editing vector which comprises a gene for an editing enzyme under control of regulatory sequences which direct its expression in a target cell (*e.g.,* a hepatocyte) comprising a targeted gene which has one or more mutations resulting in a disorder (*e.g.,* a liver metabolic disease) and (b) a targeting vector comprising a sequence specifically recognized by the editing enzyme and donor template, wherein the donor template comprises nucleic acid sequences which replaces at least one of the mutations in the targeted gene.

In one embodiment, the gene editing vector comprises a Cas9 gene as the editing enzyme and the targeting vector comprises sgRNA which is at least 20 nucleotides in length which specifically bind to a selected site in the targeted genes and is 5' to a protospacer-adjacent motif (PAM) which is specifically recognized by the Cas9. Typically, the PAM sequence to the corresponding sgRNA is mutated on the donor template. However, in another embodiment, the gene editing vector may contain a different Crispr.

"Cas9" (CRISPR associated protein 9) refers to family of RNA-guided DNA endonucleases which is characterized by two signature nuclease domains, RuvC (cleaves non-coding strand) and HNH (coding strand). Suitable bacterial sources of Cas9 include *Staphylococcus aureus* (SaCas9), *Streptococcus pyogenes* (SpCas9), and Neisseria meningitides [KM Estelt et al, Nat Meth, 10: 1116-1121 (2013)]. The wild-type coding sequences may be utilized in the constructs described herein. Alternatively, these bacterial codons are optimized for expression in humans, *e.g*., using any of a variety of known human codon optimizing algorithms. Alternatively, these sequences may be produced synthetically, either in full or in part. In the examples below, the *Staphylococcus aureus* (SaCas9) and the *Streptococcus pyogenes* (SpCas9) versions of cas9 were compared. SaCas9 has a shorter sequence. Other endonucleases with similar properties may optionally be substituted. See, e.g., the public CRISPR database (db) accessible at http://crispr.u-psud.fr/crispr.

In another embodiment, the CRISPR system selected may be Cpfl (CRISPR from Prevotella and Francisella), which may be substituted for Class 2 CRISPR, type II Cas9-based system in the methods described herein. In contrast, Cpf1's preferred PAM is 5'-TTN; this contrasts with that of SpCas9 (5'-NGG) and SaCas9 (5'-NNGRRT; N=any nucleotide; R=adenine or guanine) in both genomic location and GC-content. While at least 16 Cpfl nucleases, two humanized nucleases (AsCpf1 and LbCpf1) are particularly useful. See, http://www.addgene.org/69982/sequences/#depositor-full (AsCpf1 sequences; and http://www.addgene.org/69988/sequences/#depositor-full (LbCpf1 sequences), which are incorporated herein by reference. Further, Cpfl does not require a tracrRNA; allowing use of shorter guide RNAs (about 42 nucleotides) as compared to Cas9. Plasmids may be obtained from Addgene, a public plasmid database.

While the system can be effective if the ratio of gene editing vector to template vector is about 1 to about 1, it is desirable for the template vector to be present in excess of the gene editing vector. In one embodiment, the ratio of editing vector (a) to targeting vector (b) is about 1:3 to about 1:100, or about 1:10. This ratio of gene editing enzyme (e.g., Cas9 or Cpf) to donor template may be maintained even if the enzyme is additionally or alternatively supplied by a source other than the AAV vector. Such embodiments are discussed in more detail below.

A variety of conventional vector elements may be used for delivery of the editing vector to the target cells and expression of the enzyme (Cas9 or Cpfl). However, for the dual vector system designed for treatment of metabolic disorders characterized by a mutation or phenotype in hepatocytes, the gene editing vector may designed such that the enzyme is expressed under the control of a liver-specific promoter. The illustrative plasmid and vector described herein uses the liver-specific promoter thyroxin binding globulin (TBG) or a novel shortened version of TBG, a variant termed herein TBG-S1, which is characterized by the sequence:

Alternatively, other liver-specific promoters may be used [*see, e.g.,* The Liver Specific Gene Promoter Database, Cold Spring Harbor, http://rulai.schl.edu/LSPD, alpha 1 anti-trypsin (A1AT); human albumin Miyatake et al., J. Virol., 71:5124 32 (1997), humAlb; and hepatitis B virus core promoter, Sandig et al., Gene Ther., 3:1002 9 (1996)]. TTR minimal enhancer/promoter, alpha-antitrypsin promoter, LSP (845 nt). For a different target tissue (*e.g*., epithelial cells, CNS), a different tissue specific promoter may be selected. Examples of promoters specific for endothelial cells include, but are not limited to, endothelin-I (ET-I), Flt-I, FoxJ1 (that targets ciliated cells), and T3^{b} [H Aihara et al, FEBS Letters, Vol. 463 (Issues 1-2), p. 185-188 (10 Dec 1999) (targeting intestinal epithelial cells), E-cadherin promoter (J. Behrens et al, Proc Natl Acad Sci USA, Vol. 88: 11495-11499 (Dec 1991)], CEA promoter. Examples of neuron-specific promoters include, e.g., synapsin I (SYN), calcium/calmodulin-dependent protein kinase III, tubulin alpha I, microtubulin-associated protein 1B (MAPIB), neuron-specific enolase (Andersen et al., Cell. Mol. Neurobiol., 13:503-15 (1993)), and platelet-derived growth factor beta chain promoters, neurofilament light- chain gene promoter (Piccioli et al., Proc. Natl. Acad. Sci. USA, 88:5611-5 (1991)), and the neuron-specific vgf gene promoter (Piccioli et al., Neuron, 15:373-84 (1995)), neuronal nuclei (NeuN), glial fibrillary acidic protein (GFAP), adenomatous polyposis coli (APC), and ionized calcium-binding adapter molecule 1 (Iba-1), minimal promoter for HB9 [S Pfaff, Neuron (1999) 23: 675-687; Nature Genetics (1999) 23: 71-75]. Non-tissue specific promoters can also be used. Optionally, such a promoter may be an enhancer (e.g., cytomegalovirus). Alternatively, other promoters, such as constitutive promoters, regulatable promoters [*see, e.g.,* WO 2011/126808 and WO 2013/049493, or a promoter responsive to physiologic cues may be utilized in the vectors described herein. Alternatively, for a different target tissue, Optionally, if a regulatable system is selected, a third vector may be required in order to provide the regulatory function.

Among other conventional vector elements which may be used in the gene editing vector are expression enhancer elements. One desirable enhancer is the novel ABPS2 (2 repeats of shortened ABP enhancer element):
gttaatttttaaactgtttgctctggttaataatctcaggaggttaatttttaaactgtttgctctggttaataatctca (SEQ ID NO: 4).

However, other suitable enhancers may include, *e.g*., the alpha fetoprotein enhancer, the TTR minimal promoter/enhancer, LSP (TH-binding globulin promoter/alpha1-microglobulin/bikunin enhancer), amongst others. Yet other promoters and enhancers can be used to target liver and/or other tissues. Other suitable vector elements may also be included in this gene editing vector. However, the size of the enzyme (Cas9 or Cpfl) gene and packaging limitations of AAV does make it desirable to select truncated or shortened versions of such elements. Thus, while conventional polyA sequences may be selected including, *e.g*., SV40 and bovine growth hormone (bGH), shortened and/or synthetic polyAs may also be desired.

In addition to the gene editing vector, the dual AAV vector system utilizes a second type of vector which is an AAV targeting vector comprising sgRNA and donor template. Optionally, more than 1 sgRNA can be used to improve the rates of gene correction. The term "sgRNA" refers to a "single-guide RNA". sgRNA has at least a 20 base sequence (or about 24 - 28 bases) for specific DNA binding (homologous to the target DNA). Transcription of sgRNAs should start precisely at its 5' end. When targeting the template DNA strand, the base-pairing region of the sgRNA has the same sequence identity as the transcribed sequence. When targeting the nontemplate DNA strand, the base-pairing region of the sgRNA is the reverse-complement of the transcribed sequence. Optionally, the targeting vector may contain more than one sgRNA. The sgRNA is 5' to a protospacer-adjacent motif (PAM) which is specifically recognized by the Cas9 (or Cpfl) enzyme. Typically, the sgRNA is "immediately" 5' to the PAM sequence, *i.e.,* there are no spacer or intervening sequences. Examples of sgRNA and PAM sequences designed for correcting a mutation in the OTC gene which causes OTC deficiency are illustrated below. More particularly, the following target sequences are designed to correct the G/A mutation associated with OTC deficiency in the position corresponding to nt 243 of wt OTC by inserting (or knocking-in) a fragment containing the correct sequence [*see, e.g.,* Genbank entry D00230.2, for genomic DNA sequence and identification of introns and exons, http://www.ncbi.nlm.nih.gov/nuccore/-D00230.2].

In general, a PAM sequence for SaCas9 has an NNGRRT motif. Once a selected target sequence is selected, an sgRNA comprising the target and PAM sequence may be generated synthetically, or using conventional site-directed mutagenesis. In the examples below, illustrating correction of the ornithine transcarbamylase (OTC) gene, the target DNA is within intron 4, which is 3' to the G/A mutation site. However, other suitable target sites may be selected for other mutations targeted for correction. See, *e.g.,* http://omim.org/entry/311250.

**Table 1**

| Allelic Variants (29 Selected Examples) | | | | |
|---|---|---|---|---|
| Number | Phenotype | Mutation | dbSNP | ClinVar |
| .0001 | ORNITHINE TRANSCARBAMYLASE DEFICIENCY | OTC, DEL | | RCV000011733 |
| .0002 | ORNITHINE TRANSCARBAMYLASE DEFICIENCY | OTC, ARG109GLN | rs68026851 | RCV000083434, RCV000011734 |
| .0003 | ORNITHINE TRANSCARBAMYLASE DEFICIENCY | OTC, ARG109TER | rs67960011 | RCV000083433, RCV000011735 |
| .0004 | ORNITHINE TRANSCARBAMYLASE DEFICIENCY | OTC, LEU111PRO | rs1800324 | RCV000011736 |
| .0005 | ORNITHINE TRANSCARBAMYLASE DEFICIENCY | OTC, GLN216GLU | rs72558423 | RCV000011737, RCV000083523 |
| .0006 | ORNITHINE TRANSCARBAMYLASE DEFICIENCY | OTC, GLU154TER | rs72556267 | RCV000011738, RCV000083446 |
| .0007 | ORNITHINE TRANSCARBAMYLASE DEFICIENCY | OTC, LEU45PRO | rs72554312 | RCV000083338, RCV000011739 |
| .0008 | ORNITHINE TRANSCARBAMYLASE DEFICIENCY | OTC, ARG26GLN | rs68031618 | RCV000011740, RCV000083565 |
| .0009 | ORNITHINE TRANSCARBAMYLASE POLYMORPHISM | OTC, LYS46ARG | rs1800321 | RCV000079082, RCV000011741 |
| .0010 | ORNITHINE TRANSCARBAMYLASE DEFICIENCY | OTC, ARG245TRP | | RCV000011742 |
| .0011 | ORNITHINE TRANSCARBAMYLASE DEFICIENCY | OTC, GT-GC, INTRON 7 | rs72558431 | RCV000011743, RCV000083544 |
| .0012 | ORNITHINE TRANSCARBAMYLASE DEFICIENCY | OTC, GTA-GTG, INTRON 7 | | RCV000011744 |
| .0013 | ORNITHINE TRANSCARBAMYLASE DEFICIENCY | OTC, IVS4, A-T, -2 | rs66556380 | RCV000083419, RCV000011745 |
| .0014 | ORNITHINE TRANSCARBAMYLASE DEFICIENCY | OTC, ARG277TRP | rs72558454 | RCV000083586, RCV000011746 |
| .0015 | ORNITHINE TRANSCARBAMYLASE DEFICIENCY | OTC, PRO225LEU | rs67120076 | RCV000083536, RCV000011747 |
| .0016 | ORNITHINE TRANSCARBAMYLASE DEFICIENCY | OTC, GLU87LYS | rs72554338 | RCV000011748, RCV000083376 |
| .0017 | ORNITHINE TRANSCARBAMYLASE DEFICIENCY | OTC, GLY50TER | rs67486158 | RCV000083346, RCV000011749 |
| .0018 | ORNITHINE TRANSCARBAMYLASE DEFICIENCY | OTC, GLY162ARG | rs66626662 | RCV000083456, RCV000011750 |
| .0019 | ORNITHINE TRANSCARBAMYLASE DEFICIENCY | OTC, 1-BP DEL, 403G | | RCV000011751 |
| .0020 | ORNITHINE TRANSCARBAMYLASE DEFICIENCY | OTC, IVS2, G-A, -1 | | RCV000011752 |
| .0021 | ORNITHINE TRANSCARBAMYLASE DEFICIENCY | OTC, GLY47GLU | rs72554331 | RCV000011753, RCV000083369 |
| .0022 | ORNITHINE TRANSCARBAMYLASE DEFICIENCY | OTC, ARG62THR | rs72554345 | RCV000083388, RCV000011754 |
| .0023 | ORNITHINE TRANSCARBAMYLASE DEFICIENCY | OTC, LEU272PHE | rs72558465 | RCV000011755, RCV000083607 |
| .0024 | ORNITHINE TRANSCARBAMYLASE DEFICIENCY | OTC, TYR313ASP | rs66469337 | RCV000011756, RCV000083325 |
| .0025 | ORNITHINE TRANSCARBAMYLASE DEFICIENCY | OTC, ARG129HIS | rs66656800 | RCV000011757, RCV000083414 |
| .0026 | ORNITHINE TRANSCARBAMYLASE DEFICIENCY | OTC, LEU148PHE | rs66741318 | RCV000083441, RCV000011758 |
| .0027 | ORNITHINE TRANSCARBAMYLASE DEFICIENCY | OTC, MET206ARG | rs72558412 | RCV000011759, RCV000083513 |
| .0028 | ORNITHINE TRANSCARBAMYLASE DEFICIENCY | OTC, ARG40CYS | rs72554307 | RCV000083332, RCV000011760 |
| .0029 | ORNITHINE TRANSCARBAMYLASE DEFICIENCY | OTC, ARG40HIS | rs72554308 | RCV000083333, RCV000011761 |

A more expansive list of the mutations, insertions and/or deletions associated with OTCD is provided, *e.g*., at http://www.uniprot.org/uniprot/P00480, Suitable target sites for correcting other errors in this and/or other disorders may be selected. For example, www.uniprot.org/uniprot provides a list of mutations associated with other diseases, *e.g*., cystic fibrosis [www.uniprot.org/uniprot/P13569; also OMIM: 219700], MPSIH [http://www.uniprot.org/uniprot/P35475; OMIM:607014]; hemophilia B [Factor IX, http://www.uniprot.org/uniprot/P00451]; hemophilia A [Factor VIII, http://www.uniprot.org/uniprot/P00451]. Still other diseases and associated mutations, insertions and/or deletions can be obtained from reference to this database. Other suitable sources may include, *e.g*., http://www.genome.gov/10001200; http://www.kumc.edu/gec/support/; http://www.ncbi.nlm.nih.gov/books/NBK22183/.

The target sites are selected such that they do not disrupt expression of functional portions of the gene. Optionally, more than one correction may be made to a target gene using the system described herein. Suitably, the vectors delivering donor template which are gene fragments are designed such that the donor template is inserted upstream of the gene mutation or phenotype to be corrected.

As an alternative, a full-length functioning gene may be inserted into the genome to replace the defective gene. Thus, in one embodiment, the inserted sequence may be a full-length gene, or a gene encoding a functional protein or enzyme. Where a full-length gene is being delivered, there is more flexibility within the target gene for targeting. As another alternative, a single exon may be inserted upstream of the defective exon. In another alternative, gene deletion or insertion can be corrected.

In still another embodiment, the compositions described herein are used to reduce expression of a gene having undesirably high expression levels. Such a gene may be a PCSK9 which binds to the receptor for low-density lipoprotein (LDL) cholesterol; reducing PCSK9 expression can be used to increase circulating LDL cholesterol levels. Still other genes for targeting cancer-associated genes.(e.g., BRCA1, BRCA2). See, also, http://www.eupedia.com/genetics/cancer_related_snp.shtml.

The dual AAV vector system described in detail herein expresses sgRNA and donor template RNA for precise *in vivo* gene correction of a metabolic liver disorder characterized by a genetic abnormality in the liver (hepatocytes). This system is particularly well suited for neonatal vector delivery. In one embodiment, neonatal treatment is defined as delivering treatment within 8 hours, the first 12 hours, the first 24 hours, or the first 48 hours following delivery, or up about 28 days. In another embodiment, particularly for a primate, neonatal delivery is within the period of about 12 hours to about 1 week, 2 weeks, 3 weeks, or about 1 month, or after about 24 hours to about 48 hours. Optionally, the system may be used for pre-natal delivery, delivery in infants, older children and/or adults. The same system can also be adapted to correct a variety of disorders when appropriate donor sequences and sgRNAs are incorporated into the system. Corresponding changes may also be made to the selection of vector elements, including the selection of the AAV capsid and the selection of a different type of vector system.

In one study performed to date, a dual AAV8 vector system has been used to express sgRNA and donor template DNA for precise *in vivo* gene correction of an OTC mutation about 25% of hepatocytes following neonatal vector delivery. This is believed to be the first time that efficient gene delivery and CRISPR mediated gene correction in hepatocytes has been demonstrated using an AAV vector system.

Although AAV8 is described herein in the working examples, a variety of different AAV capsids have been described and may be used, although AAV which preferentially target the liver and/or deliver genes with high efficiency are particularly desired. The sequences of the AAV8 have been described in, *e.g.,* US Patent 7790449; US Patent 7282199, and are available from a variety of databases. While the examples utilize AAV vectors having the same capsid, the capsid of the gene editing vector and the AAV targeting vector are the same AAV capsid. Another suitable AAV may be, *e.g.,* rh10 [WO 2003/042397]. Still other AAV sources may include, e.g., AAV9 [US 7,906,111; US 2011-0236353-A1], and/or hu37 [*see, e.g.,* US 7,906,111; US 2011-0236353-A1], AAV1, AAV2, AAV3, AAV4, AAV5, AAV6, AAV6.2, AAV7, AAV8, [US Patent 7790449; US Patent 7282199] and others. *See, e.g.,* WO 2003/042397; WO 2005/033321, WO 2006/110689; US Patent 7790449; US Patent 7282199; US 7588772B2 for sequences of these and other suitable AAV, as well as for methods for generating AAV vectors. Still other AAV may be selected, optionally taking into consideration tissue preferences of the selected AAV capsid.

A recombinant AAV vector (AAV viral particle) may comprise, packaged within an AAV capsid, a nucleic acid molecule containing a 5' AAV ITR, the expression cassettes described herein and a 3' AAV ITR. As described herein, an expression cassette may contain regulatory elements for an open reading frame(s) within each expression cassette and the nucleic acid molecule may optionally contain additional regulatory elements.

The AAV vector may contain a full-length AAV 5' inverted terminal repeat (ITR) and a full-length 3' ITR. A shortened version of the 5' ITR, termed ΔITR, has been described in which the D-sequence and terminal resolution site (trs) are deleted. The abbreviation "sc" refers to self-complementary. "Self-complementary AAV" refers a construct in which a coding region carried by a recombinant AAV nucleic acid sequence has been designed to form an intra-molecular double-stranded DNA template. Upon infection, rather than waiting for cell mediated synthesis of the second strand, the two complementary halves of scAAV will associate to form one double stranded DNA (dsDNA) unit that is ready for immediate replication and transcription. *See, e.g.,* D M McCarty et al, "Self-complementary recombinant adeno-associated virus (scAAV) vectors promote efficient transduction independently of DNA synthesis", Gene Therapy, (August 2001), Vol 8, Number 16, Pages 1248-1254. Self-complementary AAVs are described in, *e.g.,* U.S. Patent Nos. 6,596,535; 7,125,717; and 7,456,683,

Where a pseudotyped AAV is to be produced, the ITRs are selected from a source which differs from the AAV source of the capsid. For example, AAV2 ITRs may be selected for use with an AAV capsid having a particular efficiency for a selected cellular receptor, target tissue or viral target. In one embodiment, the ITR sequences from AAV2, or the deleted version thereof (ΔITR), are used for convenience and to accelerate regulatory approval. However, ITRs from other AAV sources may be selected. Where the source of the ITRs is from AAV2 and the AAV capsid is from another AAV source, the resulting vector may be termed pseudotyped. However, other sources of AAV ITRs may be utilized.

A single-stranded AAV viral vector may be used. Methods for generating and isolating AAV viral vectors suitable for delivery to a subject are known in the art. *See, e.g.,* US Patent 7790449; US Patent 7282199; WO 2003/042397; WO 2005/033321, WO 2006/110689; and US 7588772 B2]. In one system, a producer cell line is transiently transfected with a construct that encodes the transgene flanked by ITRs and a construct(s) that encodes rep and cap. In a second system, a packaging cell line that stably supplies rep and cap is transfected (transiently or stably) with a construct encoding the transgene flanked by ITRs. In each of these systems, AAV virions are produced in response to infection with helper adenovirus or herpesvirus, requiring the separation of the rAAVs from contaminating virus. More recently, systems have been developed that do not require infection with helper virus to recover the AAV - the required helper functions (*i.e.,* adenovirus E1, E2a, VA, and E4 or herpesvirus UL5, UL8, UL52, and UL29, and herpesvirus polymerase) are also supplied, *in trans,* by the system. In these newer systems, the helper functions can be supplied by transient transfection of the cells with constructs that encode the required helper functions, or the cells can be engineered to stably contain genes encoding the helper functions, the expression of which can be controlled at the transcriptional or posttranscriptional level. In yet another system, the transgene flanked by ITRs and rep/cap genes are introduced into insect cells by infection with baculovirus-based vectors. For reviews on these production systems, see generally, *e.g.,* Zhang et al., 2009, "Adenovirus-adeno-associated virus hybrid for large-scale recombinant adeno-associated virus production," Human Gene Therapy 20:922-929, Methods of making and using these and other AAV production systems are also described in the following U.S. patents, 5,139,941; 5,741,683; 6,057,152; 6,204,059; 6,268,213; 6,491,907; 6,660,514; 6,951,753; 7,094,604; 7,172,893; 7,201,898; 7,229,823; and 7,439,065.

In another embodiment, other viral vectors may be used, including integrating viruses, *e.g*., herpesvirus or lentivirus, although other viruses may be selected. Suitably, where one of these other vectors is generated, it is produced as a replication-defective viral vector. A "replication-defective virus" or "viral vector" refers to a synthetic or artificial viral particle in which an expression cassette containing a gene of interest is packaged in a viral capsid or envelope, where any viral genomic sequences also packaged within the viral capsid or envelope are replication-deficient; *i.e.,* they cannot generate progeny virions but retain the ability to infect target cells. In one embodiment, the genome of the viral vector does not include genes encoding the enzymes required to replicate (the genome can be engineered to be "gutless" - containing only the transgene of interest flanked by the signals required for amplification and packaging of the artificial genome), but these genes may be supplied during production.

A variety of different diseases and conditions associated with one or more genetic deletions, insertions or mutations, may be treated using the method described herein. Examples of such conditions may include, *e.g*., alpha-1-antitrypsin deficiency, liver conditions (*e.g*., biliary atresia, Alagille syndrome, alpha-1 antitrypsin, tyrosinemia, neonatal hepatitis, Wilson disease), metabolic conditions such as biotinidase deficiency, carbohydrate deficient glycoprotein syndrome (CDGS), Crigler-Najjar syndrome, diabetes insipidus, Fabry, galactosemia, glucose-6-phosphate dehydrogenase (G6PD), fatty acid oxidation disorders, glutaric aciduria, hypophosphatemia, Krabbe, lactic acidosis, lysosomal storage diseases, mannosidosis, maple syrup urine, mitochondrial, neuro-metabolic, organic acidemias, PKU, purine, pyruvate dehydrogenase deficiency, urea cycle conditions, vitamin D deficient, and hyperoxaluria. Urea cycle disorders include, *e.g.*, N-acetylglutamate synthase deficiency, carbamoyl phosphate synthetase I deficiency, ornithine transcarbamylase deficiency, "AS deficiency" or citrullinemia, "AL deficiency" or argininosuccinic aciduria, and "arginase deficiency" or argininemia.

Other diseases may also be selected for treatment according to the method described herein. Such diseases include, *e.g*., cystic fibrosis (CF), hemophilia A (associated with defective factor VIII), hemophilia B (associated with defective factor IX), mucopolysaccharidosis (MPS) (*e.g*., Hunter syndrome, Hurler syndrome, Maroteaux-Lamy syndrome, Sanfilippo syndrome, Scheie syndrome, Morquio syndrome, other, MPSI, MPSII, MPSIII, MSIV, MPS 7); ataxia (*e.g*., Friedreich ataxia, spinocerebellar ataxias, ataxia telangiectasia, essential tremor, spastic paraplegia); Charcot-Marie-Tooth (*e.g*., peroneal muscular atrophy, hereditary motor sensory neuropathy), glycogen storage diseases (*e.g*., type I, glucose-6-phosphatase deficiency, Von Gierke), II (alpha glucosidase deficiency, Pompe), III (debrancher enzyme deficiency, Cori), IV (brancher enzyme deficiency, Anderson), V (muscle glycogen phosphorylase deficiency, McArdle), VII (muscle phosphofructokinase deficiency, Tauri), VI (liver phosphorylase deficiency, Hers), IX (liver glycogen phosphorylase kinase deficiency). This list is not exhaustive and other genetic conditions are identified, *e.g*., www.kumc.edu/gec/support; http://www.genome.gov/10001200; and http://www.ncbi.nlm.nih.gov/books/NBK22183/,

Other disorders include a central nervous system (CNS)-related disorder. As used herein, a "CNS-related disorder" is a disease or condition of the central nervous system. Such a disorder may affect the spinal cord, brain, or tissues surrounding the brain and spinal cord. Non-limiting examples of CNS- related disorders, include Parkinson's Disease, Lysosomal Storage Disease, Ischemia, Neuropathic Pain, Amyotrophic lateral sclerosis (ALS) (e.g., linked to a mutation in the gene coding for superoxide dismutase, SOD1), Multiple Sclerosis (MS), and Canavan disease (CD), or a primary or metastatic cancer.

In another embodiment, cells of the retina are targeted, including retinal pigment epithelium (RPE) and photoreceptors, e.g., for treatment of retinitis pigmentosa and/or Leber congenital amaurosis (LCA). Optionally, this treatment may utilize or follow subretinal injection and/or be used in conjunction with the standard of care for the condition.

In one aspect, the method is useful in treating a disorder, comprising: co-administering to a subject having the disorder. The dual AAV system employs (a) a gene editing AAV vector comprising a Cas9 (or Cpfl) gene under control of regulatory sequences which direct its expression in a target cell (*e.g.,* a hepatocyte or lung cell) comprising a targeted gene which has one or more mutations resulting in a disorder; and (b) an AAV targeting vector comprising sgRNA and donor template, wherein the sgRNA comprises at least 20 nucleotides which specifically bind to a selected site in the targeted genes and is 5' (or 3') to a PAM which is specifically recognized by the Cas9 (or Cpfl), and wherein the donor template comprises nucleic acid sequences which replaces at least one of the mutations in the targeted gene and PAM sequences are mutated; wherein the ratio of gene editing AAV vector of (a) to (b) is such that (b) is in excess of (a). Also provided is use of the vector system as described herein to treat a disorder in humans, or in the preparation of a medicament for the treatment of a disorder.

In one embodiment, the method is used in neonates or infants. Alternatively, the method is used in older subjects. In one aspect, the invention provides a method of treating a liver metabolic disorder in neonates, comprising: co-administering to a subject having a liver metabolic disorder. The dual AAV system employs (a) a gene editing AAV vector comprising a Cas9 (or Cpfl) gene under control of regulatory sequences which direct its expression in a hepatocyte comprising a targeted gene which has one or more mutations resulting in a liver metabolic disorder; and (b) an AAV targeting vector comprising sgRNA and donor template, wherein the sgRNA comprises at least 20 nucleotides which specifically bind to a selected site in the targeted genes and is 5' to a protospacer-adjacent motif (PAM) which is specifically recognized by the enzyme, and wherein the donor template comprises nucleic acid sequences which replaces at least one of the mutations in the targeted gene and the PAM corresponding to the sgRNA is mutated; wherein the ratio of gene editing AAV vector of (a) to (b) is such that (b) is in excess of (a). Also provided is the use of (a) a gene editing AAV vector comprising a Cas9 gene under control of regulatory sequences which direct its expression in a hepatocyte comprising a targeted gene which has one or more mutations resulting in a liver metabolic disorder; and (b) an AAV targeting vector comprising sgRNA and donor template, wherein the sgRNA comprises at least 20 nucleotides which specifically bind to a selected site in the targeted genes and which is 5' to a protospacer-adjacent motif (PAM) which is specifically recognized by the Cas9, and wherein the donor template comprises nucleic acid sequences which replaces at least one of the mutations in the targeted gene, to treat a liver metabolic disorder in a neonate subject, wherein the ratio of gene editing AAV vector of (a) to (b) is such that (b) is in excess of (a), or the use of these vectors in the preparation of a medicament for the treatment of a liver metabolic disorder.

In one embodiment, the ratio of editing vector to targeting vector is about 1:3 to about 1:100, inclusive of intervening ratios. For example, the ratio of editing vector to targeting vector may be about 1: 5 to about 1:50, or about 1:10, or about 1:20. Although not as preferred, the ratio may be 1:1 or there may be more targeting vector.

In general, the ratio of AAV vectors is determined based on particle copies (pt) or genome copies (GC), which terms may be used interchangeably herein, for each vector. Suitably, when determining the ratio of two or more AAV vectors to one another (e.g., editing vector to targeting vector), the same method is used to determine the number of each type of vector(s). However, if different methods are determined to be substantially equivalent, different techniques may be used. Suitable method for determining genome copies (GC) have been described and include, *e.g.,* oqPCR or digital droplet PCR (ddPCR) as described in, *e.g.,* M. Lock et al, Hu Gene Therapy Methods, Hum Gene Ther Methods. 2014 Apr;25(2): 115-25. doi: 10.1089/hgtb.2013.131. Epub 2014 Feb 14,

While unlike traditional gene augmentation therapy, gene editing-mediated correction as described herein in neonates may not require readministration. However, optionally, a second or subsequent additional treatments involving co-administration of the dual vector Crispr/enzyme system provided herein may be pursued. For example, where patients are treated as neonates and/or the targeted cells are proliferating cells (e.g., liver cells, epithelial cells, or cancer cells), subsequent treatments may be desired. Such subsequent treatments may follow the first treatment by a month, several months, a year, or several years. Optionally, the subsequent treatment may utilize vectors having different capsids than were utilized for the initial treatment. For example, if initial treatment was by AAV8, a second treatment may utilize rh10. In another example, if initial treatment utilized rh10, subsequent treatment may utilize AAV8. Still other combinations of AAV capsids may be selected by one skilled in the art.

The compositions described herein are designed for delivery to subjects in need thereof by any suitable route or a combination of different routes. For treatment of liver disease, direct or intrahepatic delivery to the liver is desired and may optionally be performed via intravascular delivery, *e.g*., via the portal vein, hepatic vein, bile duct, or by transplant. Alternatively, other routes of administration may be selected (*e.g*., oral, inhalation, intranasal, intratracheal, intraarterial, intraocular, intravenous, intramuscular, and other parental routes). For example, intravenous delivery may be selected for delivery to proliferating, progenitor and/or stem cells. Alternatively, another route of delivery may be selected. The delivery constructs described herein may be delivered in a single composition or multiple compositions. Optionally, two or more different AAV may be delivered [*see, e.g.,* WO 2011/126808 and WO 2013/049493]. In another embodiment, such the dual vector system may contain only a single AAV and a second, different, Cas9-delivery system. For example, Cas9 (or Cpfl) delivery may be mediated by non-viral constructs, *e.g*., "naked DNA", "naked plasmid DNA", RNA, and mRNA; coupled with various delivery compositions and nanoparticles, including, *e.g*., micelles, liposomes, cationic lipid - nucleic acid compositions, poly-glycan compositions and other polymers, lipid and/or cholesterol-based - nucleic acid conjugates, and other constructs such as are described herein. *See, e.g.,* X. Su et al, Mol. Pharmaceutics, 2011, 8 (3), pp 774-787; web publication: March 21, 2011; WO2013/182683, WO 2010/053572 and WO 2012/170930, Such non-viral delivery constructs may be administered by the routes described previously.

The viral vectors, or non-viral DNA or RNA transfer moieties can be formulated with a physiologically acceptable carrier for use in gene transfer and gene therapy applications. In the case of AAV viral vectors, quantification of the genome copies ("GC") may be used as the measure of the dose contained in the formulation. Any method known in the art can be used to determine the genome copy (GC) number of the replication-defective virus compositions of the invention. One method for performing AAV GC number titration is as follows: Purified AAV vector samples are first treated with DNase to eliminate un-encapsidated AAV genome DNA or contaminating plasmid DNA from the production process. The DNase resistant particles are then subjected to heat treatment to release the genome from the capsid. The released genomes are then quantitated by real-time PCR using primer/probe sets targeting specific region of the viral genome (usually poly A signal). The replication-defective virus compositions can be formulated in dosage units to contain an amount of replication-defective virus that is in the range of about 1.0 × 10⁹ GC to about 1.0 × 10¹⁵ GC (to treat an average subject of 70 kg in body weight), and preferably 1.0 × 10¹² GC to 1.0 × 10¹⁴ GC for a human patient. Preferably, the dose of replication-defective virus in the formulation is 1.0 × 10⁹ GC, 5.0 × 10⁹ GC, 1.0 × 10¹⁰ GC, 5.0 × 10¹⁰ GC, 1.0 × 10¹¹ GC, 5.0 × 10¹¹ GC, 1.0 × 10¹² GC, 5.0 × 10¹² GC, or 1.0 × 10¹³ GC, 5.0 × 10¹³ GC, 1.0 × 10¹⁴ GC, 5.0 × 1014 GC, or 1.0 × 10¹⁵ GC.

Production of lentivirus is measured as described herein and expressed as IU per volume (*e.g*., mL). IU is infectious unit, or alternatively transduction units (TU); IU and TU can be used interchangeably as a quantitative measure of the titer of a viral vector particle preparation. The lentiviral vector is typically integrating. The amount of viral particles is at least about 3×10⁶ IU, and can be at least about 1×10⁷ IU, at least about 3×10⁷ IU, at least about 1×10⁸ IU, at least about 3×10⁸ IU, at least about 1×10⁹ IU, or at least about 3×10⁹ IU.

In addition, the dual vector system described herein may involve co-administration of an AAV vector as described herein in combination with a non-AAV vector carrying the enzyme (Cas9 or Cpfl). For example, the enzyme may be delivered via a different vector, or via mRNA or DNA alone, or in combination with AAV vector-mediated delivery of the Cas9. For example, a Cas9 (or Cpfl) sequence may be via a carrier system for expression or delivery in RNA form (*e.g*., mRNA) using one of a number of carrier systems which are known in the art. Such carrier systems include those provided by commercial entities, such as PhaseRx' so-called "SMARTT" technology. These systems utilize block copolymers for delivery to a target host cell. See, *e.g*., US 2011/0286957 entitled, "Multiblock Polymers", published November 24, 2011; US 2011/0281354, published November 17, 2011; EP2620161, published July 31, 2013; and WO 2015/017519, published Feb 5, 2015,. See, also, S. Uchida et al, (Feb 2013) PLoS ONE 8(2): e56220. Still other methods involve generating and injecting synthetic dsRNAs [see Soutschek et al. Nature (2004) 432(7014): 173-8; see also Morrissey et al. Hepatol. (2005) 41(6): 1349-56], local administration to the liver has also been demonstrated by injecting double stranded RNA directly into the circulatory system surrounding the liver using renal vein catheterization. [See Hamar et al. PNAS (2004) 101(41): 14883-8.]. Still other systems involve delivery of dsRNA and particularly siRNA using cationic complexes or liposomal formulations [see, *e.g.,* Landen et al. Cancer Biol. Ther. (2006) 5(12); see also Khoury et al. Arthritis Rheumatol. (2006) 54(6): 1867-77. Other RNA delivery technologies are also available, *e.g*., from Veritas Bio [see, *e.g.,* US 2013/0323001, Dec 23, 2010, "In vivo delivery of double stranded RNA to a target cell" (cytosolic content including RNAs, *e.g*., mRNA, expressed siRNA/shRNA/miRNA, as well as injected/introduced siRNA/shRNA/miRNA, or possibly even transfected DNA present in the cytosol packaged within exovesicles and be transported to distal sites such as the liver)]. Still other systems for in vivo delivery of RNA sequences have been described. See, *e.g.,* US 2012/0195917 (Aug 2, 2012) (5'-cap analogs of RNA to improve stability and increase RNA expression), WO 2013/143555A1, Oct 3, 2013, and/or are commercially available (BioNTech, Germany; Valera (Cambridge, MA); Zata Pharmaceuticals).

DNA and RNA are generally measured in the nanogram (ng) to microgram (µg) amounts of the nucleic acids. In general, for a treatment in a human preferably dosages of the RNA is the range of 1 ng to 700 µg, 1 ng to 500 µg, 1 ng to 300 µg, 1 ng to 200 µg, or 1 ng to 100 µg are formulated and administered. Similar dosage amounts of a DNA molecule (*e.g*., containing a Cas9 or other expression cassette) and not delivered to a subject via a viral vector may be utilized for non-viral DNA delivery constructs.

The above-described recombinant vectors or other constructs may be delivered to host cells according to published methods. The vectors or other moieties are preferably suspended in a physiologically compatible carrier, may be administered to a human or non-human mammalian patient. Suitable carriers may be readily selected by one of skill in the art in view of the indication for which the transfer virus is directed. For example, one suitable carrier includes saline, which may be formulated with a variety of buffering solutions (*e.g*., phosphate buffered saline). Other exemplary carriers include sterile saline, lactose, sucrose, calcium phosphate, gelatin, dextran, agar, pectin, peanut oil, sesame oil, and water. The selection of the carrier is not a limitation of the present invention.

Optionally, the compositions of the invention may contain, in addition to the rAAV and carrier(s), other conventional pharmaceutical ingredients, such as preservatives, or chemical stabilizers. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

The system described herein may be therapeutically useful if a sufficient amount of functional enzyme or protein is generated to improve the patient's condition. In certain embodiments, gene expression levels as low as 5% of healthy patients will provide sufficient therapeutic effect for the patient to be treatable to non-gene therapy approaches. In other embodiments, gene expression levels are at least about 10%, at least about 15% to up to 100% of the normal range (levels) observed in humans (other veterinary subject). For example, by "functional enzyme", is meant a gene which encodes the wild-type enzyme (*e.g.,* OTCase) which provides at least about 50%, at least about 75%, at least about 80%, at least about 90%, or about the same, or greater than 100% of the biological activity level of the wild-type enzyme, or a natural variant or polymorph thereof which is not associated with disease. More particularly, as heterozygous patients may have as low an enzyme functional level as about 50% or lower, effective treatment may not require replacement of enzyme activity to levels within the range of "normal" or non-deficient patients. Similarly, patients having no detectable amounts of enzyme may be rescued by delivering enzyme function to less than 100% activity levels, and may optionally be subject to further treatment subsequently. In certain embodiments, were gene function is being delivered by the donor template, patients may express higher levels than found in "normal", healthy subjects. In still other embodiments, where reduction in gene expression is desired, as much as a 20% reduction to a 50% reduction, or up to about 100% reduction, may provide desired benefits. As described herein, the therapy described herein may be used in conjunction with other treatments, *i.e.,* the standard of care for the subject's (patient's) diagnosis.

A variety of assays exist for measuring OTC expression and activity levels in vitro. *See, e.g.,* X Ye, et al, 1996 Prolonged metabolic correction in adult ornithine transcarbamylase-deficient mice with adenoviral vectors. J Biol Chem 271:3639-3646) or in vivo. For example, OTC enzyme activity can be measured using a liquid chromatography mass spectrometry stable isotope dilution method to detect the formation of citrulline normalized to [1,2,3,4,5-13C5] citrulline (98% 13C). The method is adapted from a previously developed assay for detection of N-acetylglutamate synthase activity [Morizono H, et al, Mammalian N-acetylglutamate synthase. Mol Genet Metab. 2004;81(Suppl 1):S4-11.]. Slivers of fresh frozen liver are weighed and briefly homogenized in buffer containing 10 mM HEPES, 0.5 % Triton X-100, 2.0 mM EDTA and 0.5 mM DTT. Volume of homogenization buffer is adjusted to obtain 50 mg/ml tissue. Enzyme activity is measured using 250 µg liver tissue in 50 mM Tris-acetate, 4 mM ornithine, 5 mM carbamyl phosphate, pH 8.3. Enzyme activity is initiated with the addition of freshly prepared 50 mM carbamyl phosphate dissolved in 50 mM Tris-acetate pH 8.3, allowed to proceed for 5 minutes at 25 °C and quenched by addition of an equal volume of 5 mM13C5-citrulline in 30%TCA. Debris is separated by 5 minutes of microcentrifugation, and the supernatants are transferred to vials for mass spectroscopy. Ten µL of sample are injected into an Agilent 1100 series LC-MS under isocratic conditions with a mobile phase of 93% solvent A (1 ml trifluoroacetic acid in 1 L water):7% solvent B (1ml trifluoroacetic acid in 1L of 1:9 water/acetonitrile). Peaks corresponding to citrulline [176.1 mass:charge ratio (m/z)] and 13C5-citrulline (181.1 m/z) are quantitated, and their ratios are compared to ratios obtained for a standard curve of citrulline run with each assay. Samples are normalized to either total liver tissue or to protein concentration determined using a Bio-Rad protein assay kit (Bio-Rad, Hercules, CA). Other assays, which do not require liver biopsy, may also be used. One such assay is a plasma amino acid assays in which the ratio of glutamine and citrulline is assessed and if glutamine is high (>800 microliters/liter) and citrulline low (*e.g*., single digits), a urea cycle defect is suspected. Plasma ammonia levels can be measured and a concentration of about 100 micromoles per liter is indicative of OTCD. Blood gases can be assessed if a patient is hyperventilating; respiratory alkalosis is frequent in OTCD. Orotic acid in urine, *e.g*., greater than about 20 micromoles per millimole creatine is indicative of OTCD, as is elevated urinary orotate after allopurinol challenge test. Diagnostic criteria for OTCD have been set forth in Tuchman et al, 2008, Urea Cycle Disorders Consortium (UCDC) of the Rare Disease Clinical Research Network (RDCRN). Tuchman M, et al., Consortium of the Rare Diseases Clinical Research Network. Cross-sectional multicenter study of patients with urea cycle disorders in the United States. Mol Genet Metab. 2008;94:397-402, See, also, http://www.ncbi.nlm.nih.gov/books/NBK154378/, which provides a discussion of the present standard of care for OTCD.

It is to be noted that the term "a" or "an" refers to one or more. As such, the terms "a" (or "an"), "one or more," and "at least one" are used interchangeably herein.

The words "comprise", "comprises", and "comprising" are to be interpreted inclusively rather than exclusively. The words "consist", "consisting", and its variants, are to be interpreted exclusively, rather than inclusively. While various embodiments in the specification are presented using "comprising" language, under other circumstances, a related embodiment is also intended to be interpreted and described using "consisting of" or "consisting essentially of" language.

As used herein, the term "about" means a variability of 10 % (±10%) from the reference given, unless otherwise specified.

A "subject" is a mammal, *e.g*., a human, mouse, rat, guinea pig, dog, cat, horse, cow, pig, or non-human primate, such as a monkey, chimpanzee, baboon or gorilla. A patient refers to a human. A veterinary subject refers to a non-human mammal.

As used herein, "disease", "disorder" and "condition" are used interchangeably, to indicate an abnormal state in a subject.

Unless defined otherwise in this specification, technical and scientific terms used herein have the same meaning as commonly understood by one of ordinary skill in the art and by reference to published texts, which provide one skilled in the art with a general guide to many of the terms used in the present application.

The following examples are illustrative only and are not a limitation on the invention described herein.

### EXAMPLES

### Example 1 - Correction of OTC Deficiency using dual AAV CRISPR-Cas9

An X-linked deficiency of the OTC enzyme in humans causes recurrent and life threatening episodes of hyperammonemia [Batshaw, M., et al, Mol Genet Metab, 113: 127-139 (2014); Lichter-Koneti, U, et al., Ornithine Transcarbamylase Deficiency (1993-2013) In: Pagon RA, Adam MP, Ardinger HH, et al., editors. GeneReviews® (http://www.ncbi.nlm.nih.gov/books/NBK154378/)]. In males hemizygous for OTC deficiency, the first metabolic crisis can usually occur in the newborn period and is associated with up to 50% mortality; survivors typically undergo liver transplantation in the first year of life [Ah Mew et al, J Pediatr, 162: 324-329, e321 (2013)]. An animal model of OTC deficiency, the male sparse fur ash (*spf^{ash}*) mouse, has a point mutation at the 3 donor splice site at the end of exon 4 of the OTC gene which leads to abnormal splicing and a 20-fold reduction in OTC mRNA and protein [Hodges, P. E. & Rosenberg, L. E. The spfash mouse: a missense mutation in the ornithine transcarbamylase gene also causes aberrant mRNA splicing. Proc Natl Acad Sci U S A 86, 4142-4146 (1989)]. Affected animals can survive on a chow diet, but develop hyperammonemia that can be lethal when provided on a high protein diet.

The studies in this Example utilized an AAV vector with high liver tropism (i.e., AAV8) to correct the point mutation in newborn *spf^{ash}* mice using Cas9 enzymes from *Streptococcus pyogenes* (SpCas9) [Jinek, M. et al, Science, 337: 816-821 (2012); Cong L., et al, Science, 339: 819-823 (2013); Mali P, et al, Science, 339: 823-826 (2013)] and *Staphylococcus aureus* (SaCas9) [Ran, FA, et al., Nature, 520: 186-191 (2015)]. Protospacer-adjacent motif (PAM) sequences (NNGRRT) in proximity to the *spf^{ash}* mutation of the OTC gene were identified, as were potential 20 nt guide sequences. Three sequences, sgRNA 1-3 (Fig. 1A), were further evaluated following transfection of neomycin-containing plasmids into a mouse MC57G cell line. Low transfection efficiency in this cell line required enrichment of transfected cells by a brief exposure to puromycin. Evidence for double-strand breaks (DSBs) at the desired site was demonstrated using the SURVEYOR assay with two of the three guide RNAs (FIG 4A). Due to the absence of indel formation with sgRNA3, this guide sequence was not pursued further. The remaining candidates, sgRNA1 and sgRNA2, were similar in both DSB induction efficiency (FIG 4A) and proximity to the *spf^{ash}* mutation (FIG 1A). Due to its location within exon 4, sgRNA2 was avoided; DSB induction within an exon can lead to non-homologous end joining (NHEJ) without homology directed repair (HDR), which could ablate the residual OTC activity characteristic of the *spf^{ash}* mutation, thereby reducing residual ureagenesis. As a result, intronic sgRNA1 was selected for further experimentation. The sgRNA1 guide was then transfected with SaCas9 plus a donor DNA with approximately 0.9 kb of sequence flanking each side of the mutation in which the corresponding PAM sequence was mutated and an Agel site was included to facilitate detection of HDR. High efficiency HDR was achieved with this combination of SaCas9, donor, and sgRNA1 (FIG 4B).

A two-vector approach was necessary to incorporate all components into AAV (FIG IB). Vector 1 expresses the SaCas9 gene from a liver specific promoter called TBG (subsequently referred to as AAV8.SaCas9), while vector 2 contains sgRNA1 sequence expressed from a U6 promoter and the 1.8 kb donor OTC DNA sequence (referred to as AAV8.sgRNA1.donor). In all experiments, *spf^{ash}* pups were injected IV on postnatal day 2 with mixtures of vector 1 and vector 2 and subsequently evaluated for indel formation and functional correction of the *spf^{ash}* mutation (Fig. 1C).

To generate a dual AAV vector system for *in vivo* OTC gene correction by SaCas9, we constructed two AAV cis-plasmids: 1) the hSaCas9 was subcloned from pX330.hSaCas9 into an AAV backbone plasmid containing the full length TBG promoter (two copies of enhancer elements of the α microglobulin/bikunin gene followed by a liver-specific TBG promoter) and the bovine growth hormone polyadenylation sequence; 2) the 1.8-kb OTC donor template was cloned into the pAAV backbone and the U6-OTC sgRNA sequence was inserted into the *Afl*II site (Fig. 1C), yielding AAV8.sgRNA1donor. The PAM sequence on the donor template AAV. sgRNA1 donor was mutated (Table 1) to prevent re-cleavage by Cas9 after HDR, and an *AgeI* site was added to facilitate detection of HDR. The untargeted AAV8.control.donor differs from the targeted AAV8.sgRNA1 donor by eliminating the protospacer from the U6-OTC sgRNA cassette. Puromycin-resistant gene was cloned into pX330.hSaCas9-derived plasmids for selection of transfected cells following *in vitro* transient transfection. All plasmid constructs were verified by sequencing.

### A. Vector construction

To edit the murine OTC locus with hSpCas9 or hSaCas9, 20-nt target sequences, *e.g*., the hSpCas9 or hSaCas9 target illustrated in in the table below and FIG 1 were selected to precede a 5'-NGG protospacer-adjacent motif (PAM) or 5'-NNGRRT PAM sequence. Fig. 1 shows SaCas9 target sites.

**Table 2**

| Target sequence | PAM Signature Sequence | Source of Cas9 |
|---|---|---|
| AGTTTGAAATAAACTTTGGA (SEQ ID NO: 5) | AGG | SpCas9 |
| GAAAAGTTTTACAAACTGAG (SEQ ID NO: 6) | CGG | SpCas9 |
| TCAGAGTTTGAAATAAACTT (SEQ ID NO: 7) | TGG | SpCas9 |
| TCTCTTTTAAACTAACCCAT (SEQ ID NO: 8) | CAGAGT | SaCas9 |
| CACAAGACATTCACTTGGGT (SEQ ID NO: 9) | GTGAAT | SaCas9 |
| AAAGTTTATTTCAAACTCTG (SEQ ID NO: 10) | ATGGGT | SaCas9 |

Three target sequences were selected and individually cloned into the pX330 plasmid (Addgene), which co-expresses the sgRNA and humanized SpCas9 (hSpCas9). The OTC donor template plasmid was constructed by amplifying a 1.8-kb fragment flanking the G/A mutation site in *spf^{ash}* mouse using genomic DNA isolated from a C57BL/6 mouse as the template. The *AgeI* restriction site was subsequently introduced into donor template with the In-Fusion^{®} HD Cloning System (Clontech). To generate a dual AAV vector system for *in vivo* OTC gene editing (hSpCas9), two AAV cis-plasmids were constructed: 1) the hSpCas9 was subcloned from pX330 into a AAV backbone plasmid containing two copies of shortened enhancer elements of α microglobulin/bikunin gene [http://www.ncbi.nlm.nih.gov/gene/259, accessed 04/24/2015] followed by a shortened liver-specific TBG promoter (TBG-S1) and a minimal polyadenylation signal (PA75) (signal pattern: AATAAA) [GB Accession number CN480585, https://fasterdb.lyon.unicancer.fr]; 2) the 1.8-kb OTC donor template was cloned into pAAV backbone and U6-OTC sgRNA sequence was inserted into the *AflII* site.

The smaller size of Cas9 from *Staphylococcus aureus* (SaCas9) made it desirable for packaging into the AAV vector. FLAG-tagged SaCas9 was codon-optimized according to codon usage in human (hSaCas9) and pX330.hSaCas9 was constructed by replacing the hSpCas9 and sgRNA scaffold in pX330 with hSaCas9 and SaCas9 sgRNA scaffold. Three 20-nt target sequences preceding a 5'NNGRRT PAM sequence were selected for OTC gene editing.

Illustrative plasmid sequences are provided in the attached Sequence Listing and include, pAAV.ABPS2.TBG-S1.hSpCas9; pAAV.TBG.hSaCas9.bGH; pAAV.U6.control.mOTC.T1.9(hSaCas9); pAAV.U6.control.mOTC.T1.8 (hSpCas9); pAAV.U6.OTC\sgRNA1.mOTC.T1.8.MfeI\(hSaCas9)\; pAAV.U6.OTC\sgRNA1.mOTC.T1.8.MfeI (hSpCas9); pAAV.U6.OTC\sgRNA1.mOTC.T1.8.TBG.hOTCco.BGH(hSaCas9); pAAV.U6.OTC\sgRNA2.mOTC.T1.8.M2\(hSaCas9)\; and pAAV.U6.mOTC\sgRNA4.mOTC.T1.8.MfeI (hSpCas9)\.

### B. In vitro validation of OTC sgRNAs and donor template

MC57G cells were transiently transfected with OTC targeted Cas9 plasmid (250 ng) with increasing amounts of Agel tagged OTC donor plasmid (250, 500, 1000ng) followed by 4-day puromycin enrichment and SURVEYOR nuclease assay. SpCas9 sgRNA3 and SaCas9 sgRNA 1 showed the highest efficiency in inducing indels in the targeted loci and therefore were chosen for subsequent studies.

Increasing the amount of donor template did not result in increasing levels of HDR *in vitro.* Co-transfection of untargeted Cas9 plasmid (250 ng) and the Agel tagged OTC donor template (1000 ng) did not result in detectable HDR. Arrows denote AgeI-sensitive cleavage products resulting from HDR. Lanes with no quantification had no detectable Indels or HDR.

### C. Cell culture and transfection

MC57G cells (ATCC) were maintained in DMEM medium supplemented with 10% FBS and cultured at 37°C with 5% CO₂. Cell lines were used directly upon receipt from ATCC and were not authenticated or tested for mycoplasma contamination. For *in vitro* target and/or donor template testing, plasmids were transfected into MC57G cells using Lipofectamine^{®} LTX with Plus^{™} reagent (Life Technology) per manufacturer's recommendations. Transfected cells were under puromycin (4 µg mL⁻¹) selection for 4 days to enrich transfected cells.

### D. Genomic DNA extraction and SURVEYOR^{®} assay

Genomic DNA from transfected MC57G cells was extracted using the QuickExtract DNA extraction solution (Epicentre Biotechnologies). The efficiency of individual sgRNA was tested by the SURVEYOR nuclease assay (Transgenomics) using PCR primers listed in Table 3, below, which provides primers and sequences for construction and analysis of the donor template and sgRNA plasmids.

**TABLE 3**

| ID | Primer Sequence (5'->3') | SEQ ID NO: | Note |
|---|---|---|---|
| WT Sequence | | 11 | Part of OTC exon 4 and intron 4 |
| *spf^{ash}* sequence | | 12 | *spf^{ash}* G->A mutation |
| OTC donor sequence (SpCas9, 892-983 bp) | | 13 | PAM mutation |
| OTC donor sequence (SaCas9, 892-989 bp) | | 14 | PAM mutation; Agel Restriction site insertion |
| OTC sgRNA1_Fwd | CACCGTCTCTTTTAAACTAACCCAT | 15 | OTC target sequence 1 |
| OTC sgRNA1_Rev | AAACATGGGTTAGTTTAAAAGAGAC | 16 | OTC target sequence 1 |
| OTC sgRNA2_Fwd | CACCGCACAAGACATTCACTTGGGT | 17 | OTC target sequence 2 |
| OTC sgRNA2_Rev | AAACACCCAAGTGAATGTCTTGTGC | 18 | OTC target sequence 2 |
| OTC sgRNA3_Fwd | CACCGAAAGTTTATTTCAAACTCTG | 19 | OTC target sequence 3 |
| OTC sgRNA3_Rev | AAACCAGAGTTTGAAATAAACTTTC | 20 | OTC target sequence 3 |
| HDR-Fwd | TGGAGCAA TTCTGCACA TGGA | 21 | OTC PCR for RFLP analysis |
| HDR-Rev | CTTACTGAACATGGCAGTTTCCC | 22 | OTC PCR for RFLP analysis |
| OTC_PointM_ F | GGCTATGCTTGGGAATGTCCT | 23 | OTC PCR for Surveyor assay |
| OTC PointM _ R | GCTACAGAATGAAAGAGAGGCG | 24 | OTC PCR for Surveyor assay |
| mOTC hSpCas9 sgRNA1_Fwd | CACCGAGTTTGAAATAAACTTTGGA | 25 | mOTC target sequence 1 for hSpCas9 |
| mOTC hSpCas9 sgRNA1_Rev | AAACTCCAAAGTTTATTTCAAACTC | 26 | |
| mOTC hSpCas9 sgRNA2 _Fwd | CACCGAAAAGTTTTACAAACTGAG | 27 | mOTC target sequence 2 for hSpCas9 |
| mOTC hSpCas9 sgRNA2 _Rev | AAACCTCAGTTTGTAAAACTTTTC | 28 | |
| mOTC hSpCas9 sgRNA3_Fwd | CACCGTCAGAGTTTGAAATAAACTT | 29 | mOTC target sequence 3 for hSpCas9 |
| mOTC hSpCas9 sgRNA3_Rev | AAACAAGTTTATTTCAAACTCTGAC | 30 | |
| mOTC hSaCas9 sgRNA1_Fwd | CACCGTCTCTTTTAAACTAACCCAT | 31 | mOTC target sequence 1 for hSaCas9 |
| mOTC hSaCas9 sgRNA1_Rev | AAACATGGGTTAGTTTAAAAGAGAC | 32 | |
| mOTC hSaCas9 sgRNA2 _Fwd | CACCGACAAGACATTCACTTGGGT | 33 | mOTC target sequence 2 for hSaCas9 |
| mOTC hSaCas9 sgRNA2_Rev | AAACACCCAAGTGAATGTCTTGTGC | 34 | |
| mOTC hSaCas9 sgRNA3 _Fwd | CACCGAAAGTTTATTTCAAACTCTG | 35 | mOTC target sequence 3 for hSaCas9 |
| mOTC hSaCas9 sgRNA3 _Rev | AAACCAGAGTTTGAAATAAACTTTC | 36 | |

### E. AAV8 vector production

All vectors used in this study were packaged with AAV serotype 8 capsid in 293 cells by polyethylenimine (PEI)-based triple transfections, concentrated from the supernatant tangential flow filtration (TFF), and further purified by iodixanol gradient ultracentrifugation as previously described (Lock M *et al.,* 2010). The genome titer (GC/ml⁻¹) of AAV vectors were determined by quantitative PCR (qPCR). All vectors used in this study passed the endotoxin assay using QCL-1000 Chromogenic LAL test kit (Cambrex Bio Science).

### F. Animals

*spf^{ash}* mice were maintained at the Animal Facility at the Translational Research Laboratories at the University of Pennsylvania as described previously [ML Batshaw, et al, Mol Genet Metab, 113: 127-130 (2014)]. All animal procedures were performed in accordance with protocols approved by the Institutional Animal Care and Use Committee (IACUC) of the University of Pennsylvania. Mating cages were monitored daily for birth. Newborn (postnatal day 2, p2) male pups received a temporal vein injection of the mixture of two vectors at the intended doses for each in a volume of 50 µl, as described [Daly, TM, et al., Methods Mol Biol, 246: 195-199 (2004)]. Untreated WT, *spf^{ash}* heterozygous (Het), and *spf^{ash}* hemizygous mice served as controls. Mice were sacrificed at 1, 3, or 8 weeks after vector treatment, and liver samples were harvested for analyses. Mice were genotyped at weaning or at the time of necropsy to confirm genotype.

For testing the efficacy of OTC correction, a high protein diet (40% protein, Animal Specialties & Provisions) was given to 7-week-old mice for 7 days. After this time, plasma was collected for measurement of plasma NH₃ using the Sigma Ammonia Assay Kit. The remaining samples were sent to Antech Diagnostics for measurements of ALT, AST, and total bilirubin.

Note that the entire litter of newborn male pups was injected with either the test or control vectors, and no specific randomization method was used. The following assays were performed in a blinded fashion in which the investigator was unaware of the nature of the vectors or vector dose: vector injection, OTC and Cas9 (FLAG) immunostaining and quantification, histopathology analysis on liver, OTC enzyme activity assay, and gene expression analysis and RT-qPCR.

The adult gene editing experiments were conducted in 8- to 10-week-old male *spf^{ash}* mice. Animals in low-dose groups received a tail vein injection of AAV8.SaCas9 (1×10¹¹GC) and AAV8.sgRNA1.donor (1×10¹² GC) or untargeted vectors at the same doses, and they were sacrificed at 3 weeks after injection for analyses. Animals in high-dose groups received a tail vein injection of AAV8.SaCas9 (1×10¹² GC) and AAV8.sgRNA1.donor (5×10¹² GC) or untargeted vectors at the same doses, and they were sacrificed at 2 weeks after injection for analyses.

### G. OTC immunostaining

Immunofluorescence for OTC expression was performed on frozen liver sections. Cryosections (8 µm) were air dried and fixed in 4% paraformaldehyde (all solutions in phosphate-buffered saline) for 10min. Sections were then permeabilized and blocked in 0.2% Triton containing 1% donkey serum for 30min. A rabbit anti-OTC antibody [Augustin, L., et al, Pediatr Res, 48: 842-846 (2000)] diluted 1:1000 in 1% donkey serum was used to incubate the sections for 1 h. After washing, the sections were stained with tetramethylrhodamine (TRITC)-conjugated donkey anti-rabbit antibodies (Vector Labs) in 1% donkey serum for 30 min, washed and mounted with Vectashield (Vector Labs).

Some sections were additionally stained with a monoclonal antibody against glutamine synthetase (BD Biosciences, clone 6, Cat# 610517) as a marker for pericentral hepatocytes followed by fluorescein isothiocyanate (FITC)-labeled donkey anti-mouse antibodies (Jackson Immunoresearch Laboratories, Cat# 715-095-150). Double staining was performed by mixing the two primary and secondary antibodies, respectively, and following the above protocol. Other sections were counterstained with fluorescein-labeled tomato lectin (Lycopersicon esculentum lectin, LEL; Vector Laboratories, Cat# FL-1171) by adding LEL to the secondary antibody solution at a dilution of 1:500.

Cas9 expression was detected on sections from paraffin-embedded livers via immunostaining for FLAG tag using monoclonal antibody M2 (Sigma, Cat# F1804). Paraffin sections were processed according to standard protocols with an antigen retrieval step (boiling for 6 min in 10 mM citrate buffer, pH 6.0). Staining was performed using a mouse-on-mouse (MOM) kit (Vector Laboratories) according to the manufacturer's instructions.

To quantify percentages of OTC-expressing hepatocytes, 10 random images were taken with a 10x objective from each liver section stained for OTC expression. In some cases, where only a small liver section was available, only 5 pictures were taken. Using ImageJ software (Rasband W. S., National Institutes of Health, USA; http://rsb.info.nih.gov/ij/), images were thresholded for OTC-positive area (i.e. the OTC-positive area was selected) and the percentage of the OTC-positive area was determined for each image. In a second measurement the images were thresholded for "empty" area (e.g. veins and sinusoids) to determine the percentage of the area not occupied by liver tissue. This was possible as a result of the presence of weak background fluorescence of the liver tissue. The final percentage of OTC-positive liver tissue (i.e. OTC-positive hepatocytes) was then calculated per adjusted area (total area minus empty area), and the values were averaged for each liver.

To determine the percentage of Cas9-positive hepatocytes, two sections from the each liver were analyzed, one stained for Cas9 (via FLAG tag), the other section stained with hematoxylin to label all nuclei. Three images from every section were taken with an 10x objective and the number of either Cas9-positive or hematoxylin stained hepatocyte nuclei was determined using ImageJ's "Analyze Particles" tool which allows to select and count stained hepatocyte nuclei. Hematoxylin stained nuclei from other cell types could be excluded based on size and circularity parameters. The percentage of Cas9-positive nuclei was then calculated based on the total number of hepatocyte nuclei visible in the hematoxylin-stained sections.

For histochemical staining of OTC activity, sliced liver tissue (2 mm) was fixed, embedded, sectioned (8 µm) and mounted onto slides for histochemical staining of OTC enzyme activity as previously described [Ye, X., et al, J Biol Chem., 271: 3639-3646 (1996)]. Hematoxylin and eosin (H&E) staining was performed on sections from paraffin-embedded liver samples processed and stained according to standard protocols. Sections were analyzed for any abnormalities compared to livers from untreated animals.

### H. OTC enzyme activity assay

OTC enzyme activity was assayed in liver lysates as described previously with modifications [Morizono, H, et al., Biochem J., 322 (Pt2): 625-631 (1997)]. Whole-liver fragments were frozen in liquid nitrogen, and stored at -80 °C until OTC measurements were performed. A homogenate of 50 mg liver tissue per mL was prepared in 50mM Tris acetate buffer pH 7.5, with a Polytron homogenizer (Kinematica AG). A total of 250 µg of liver tissue was used per assay tube, and assays were performed in triplicate. Protein concentration was determined on the remaining liver homogenate using the Bio-Rad Protein assay kit (Bio-Rad) according to the manufacturer's instructions.

### I. Western blot analysis

Western blot was performed on liver lysate as described previously [Wang, L., et al, Gene Ther, 19: 404-410 (2012) ]. OTC protein was detected by a custom rabbit polyclonal antibody (1:10,000 dilution)[Augustin, L., et al, Pediatr Res, 48: 842-846 (2000)]. Mouse anti-FLAG M2 antibody (1:2000 dilution, Sigma) and mouse anti-α-tubulin antibody (1:500 dilution, Santa Cruz) were used to detect Cas9 and α-tubulin. Mouse anti-actin antibody (1:1,000 dilution, Cell Signaling Technology, Cat# 8457L) was used to detect actin Blots imaged with ChemiDoc MP system with ImageLab 4.1 software (Bio-Rad).

### J. Gene expression analysis, RT-PCR and RT- qPCR

RNA was purified using Trizol (Life Technology) and reverse-transcribed using a High-Capacity cDNA Reverse Transcription Kit (Applied Biosystems). Real-time PCR (qPCR) reactions to measure murine OTC, SaCas9 and GAPDH were performed using gene specific primers (Applied Biosystems). Data were normalized to GAPDH.

### K. On-target and off-target mutagenesis analyses

HDR-mediated targeted modifications were confirmed by restriction-fragment length polymorphism (RFLP) analysis, as described previously [Ran, F., et al, Nat Protocol, 8: 228 - 2308 (2013)]. The HDR-Fwd and HDR-Rev primers were designed to anneal outside of the region of homology between the donor template and targeted genomic region. The PCR products were purified and digested with Agel restriction enzyme. To further determine the OTC Intron 4 on-target site, the genomic region was amplified by nested PCR. Briefly, the genomic DNA was first amplified by the HDR-Fwd and HDR-Rev primers (Table 3) using Q5^{®} High-Fidelity DNA Polymerase (New England Biolabs) and gel purified to remove the residual AAV8.sgRNA1.donor in the genomic DNA. Then nested PCR was performed by using the purified 1st round PCR amplicon. Libraries were made from 250 ng of the 2nd PCR products using NEBNext^{®} Ultra^{™} DNA Library Prep Kit for Illumina (NEB) and sequenced on Illumina MiSeq (2×250 base pair (bp) paired end or 2×300 bp paired end, Genewiz). Data were processed according to standard Illumina sequencing analysis procedures. Processed reads were mapped to the PCR amplicons as reference sequences using Burrows-Wheeler Aligner with custom scripts. Reads that did not map to reference were discarded. Insertions and/or deletions were determined by comparison of reads against reference using custom scripts. The most likely off-target sites were determined using the algorithm described in www.benchling.com, referred to as OT1 through OT16 (Table 4 below). Primers spanning these sites (see Table immediately following) were used to amplify relevant sequences by nested PCR. Purified PCR fragments were then subjected to deep sequencing as described above.

**Table 4: PCR primer sequences for detecting potential off-target effects by deep sequencing.**

| Primer Name | Sequence (5'->3') | SEQ ID NO: | Note |
|---|---|---|---|
| | | | |
| OTC_OT1F1 | CTGGTGCCTTTTTCTATCGCC | 37 | Primers for OT1 |
| OTC_OT1R1 | CCAAGAGCAACTACAATGGCTT | 38 | |
| OTC_OT1F2 | GCATTTTCATGAGCATTCCA | 39 | |
| OTC_OT1R2 | CATGTTGTGCCTGCATCTCT | 40 | |
| OTC_OT2F1 | GCAGACTCCAAGATGCAAGAC | 41 | Primers for OT2 |
| OTC_OT2R1 | GATGTTGTTCCACCCGCATCT | 42 | |
| OTC_OT2F2 | CACTGAGCCAAGTCACTGGA | 43 | |
| OTC_OT2R2 | AGGGACAAAACCAAACAGCA | 44 | |
| OTC_OT3F1 | TGGCCTTCTAAAGCAACCAA | 45 | Primers |
| OTC_OT3R1 | CCGTCTCCCAGATCACATGAC | 46 | for OT3 |
| OTC_OT3F2 | ATAACTCATAATCTATGCATGGCACAA | 47 | |
| OTC_OT3R2 | TTTGATCATGGTGTTTATCAGAGC | 48 | |
| OTC_OT4F1 | GTCCCCGACAAACCAAGCTA | 49 | Primers for OT4 |
| OTC_OT4R1 | TGAACTGGCAGTATGCAGGG | 50 | |
| OTC_OT4F2 | AACATGGTTTCTGCCCTCAG | 51 | |
| OTC_OT4R2 | GGACCATGCCGAACTCTTAC | 52 | |
| OTC_OT5F1 | TTGAGACCTAGCTCATGCCC | 53 | Primers for OT5 |
| OTC_OT5R1 | TAACGCAGAACTGGCACAGG | 54 | |
| OTC_OT5F2 | CTCTCCCATGGAGAAAGCTG | 55 | |
| OTC_OT5R2 | GAATGTGGCATTGGCTTTTT | 56 | |
| OTC_OT6F1 | GAGAGAGCCAATCTGCCCAT | 57 | Primers for OT6 |
| OTC_OT6R1 | CACCGGAAACGTGTGAGAGA | 58 | |
| OTC_OT6F2 | ACTTCCCATGATCCCATTGA | 59 | |
| OTC_OT6R2 | AGCTTCCCTCCAAGTGTCCT | 60 | |
| OTC_OT7F1 | GATGGGCATAAGCCCGAAGTA | 61 | Primers for OT7 |
| OTC_OT7R1 | TAAGGCCCAGTGTTGTTGTGT | 62 | |
| OTC_OT7F2 | GTAGCAGGGGCTCTGTGAAG | 63 | |
| OTC_OT7R2 | TGGCCTGAAATACCCAGAAC | 64 | |
| OTC_OT8F1 | GTTGAATTCGCGTGTCCAGG | 65 | Primers for OT8 |
| OTC_OT8R1 | TCCCATGGCGAGAATGTCAC | 66 | |
| OTC_OT8F2 | CCCTGTAGGAAACACAGAGGA | 67 | |
| OTC_OT8R2 | TGCTTTGGATGTTGATTCTAAA | 68 | |
| OTC_OT9F1 | GGCAAAGGACTAGCTTGCAC | 69 | Primers for OT9 |
| OTC_OT9R1 | GGGTGCTATGAGGACCAGTG | 70 | |
| OTC_OT9F2 | CAGTGGTGTGTGGAGAGCTG | 71 | |
| OTC_OT9R2 | AGAGAGAGCGCTTGACTTTGA | 72 | |
| OTC_OT10F1 | CTTTGACTCCCGGCGAAAGA | 73 | Primers |
| Primer Name | Sequence (5'->3') | SEQ ID NO: | Note |
| OTC_OT10R1 | TTGTCCATCCGGGTCATTGC | 74 | for |
| OTC_OT10F2 | AAGTCCTTCTTGCCCAACTT | 75 OT10 | |
| OTC_OT10R2 | CAGCCCCAATGCATTTTT | 76 | |
| OTC_OT11F1 | ATTACAGGTCCTGGTTGGGC | 77 | Primers for OT11 |
| OTC_OT11R1 | ACTGAGCCTGGTAGAGCCTT | 78 | |
| OTC_OT11F2 | GGAAGGTGAAGGAAGGAAGG | 79 | |
| OTC_OT11R2 | TTTTCTAGGAATTCAGGACATACA | 80 | |
| OTC_OT12F | TCATGGTCCTTAAAATTTTTGC | 81 | Primers for OT12 |
| OTC_OT12R | TCCAGGTATGCAAAGTGGAT | 82 | |
| OTC_OT13F1 | TTCAGTTGTACTTTGGATGCTCTGA | 83 | Primers for OT13 |
| OTC_OT13R1 | OT10 CATCTGAATAGCAGCAGGCG | 84 | |
| OTC_OT13F2 | TAGCACAGCCCAAATGACTT | 85 | |
| OTC_OT13R2 | TCATGAAACCCCATAATCAGAA | 86 | |
| OTC_OT14F1 | AGTGGGTCATCCTTTGTTACCC | 87 | Primers for OT14 |
| OTC_OT14R1 | TGCCAGTTATCAGCCAAGCA | 88 | |
| OTC_OT14F2 | CCCAGGAACTTAACTCAGGTG | 89 | |
| OTC_OT14R2 | TGCCATTTGACCTCATAAGTCT | 90 | |
| OTC_OT15F1 | TTCAGCCCCCTTGAGTGTTTA | 91 | Primers for OT15 |
| OTC_OT15R1 | GTCTCTGAGCACAAAGAGACGA | 92 | |
| OTC_OT15F2 | TTGCCTGTCCCAACTAGAGC | 93 | |
| OTC_OT15R2 | GGCCCAAGAATGCACATTTA | 94 | |
| OTC_OT16F1 | CCACACACTGGCTAGGACTG | 95 | Primers for OT16 |
| OTC_OT16R1 | ACTGGCAGCACTTGAGACAA | 96 | |
| OTC_OT16F2 | GATGGCATGCTGTGGTTTTT | 97 | |
| OTC_OT16R2 | CAATGCTTCCACACAGAACC | 98 | |

Frequencies of on-target and off-target indels and on-target correction of the *spf^{ash}* mutation were determined as follows. MiSeq reads were analyzed using custom scripts to identify indels by matching reads against reference, with indels involving any portion of the sequence within 15 nt upstream or downstream of the predicted CRISPR-Cas9 cleavage site (3 nt downstream of the PAM, within the protospacer) considered to be possible off-target effects. Reads for which there was any 18-nt sequence with more than 2 mismatches with the corresponding 18-nt portion of the reference sequence, either upstream or downstream of a candidate indel, were discarded as errors. All candidate indels for the OT1 through OT16 sites were manually curated for confirmation. For the *OTC* intron 4 on-target site, a read was counted as having "Perfect HDR" if on the antisense strand there was a perfect match with a 51-nt sequence from the donor, starting with the donor-specific 'CACCAA' at the location of the PAM, through the donor-specific *Age*I insert 'ACCGGT', and ending with the SNV 'C' at the *spf^{ash} OTC* mutation site. A read was counted as being a "Read with a ′G‴ if it either (1) met the criterion for "Perfect HDR" or (2) had the SNV 'G' on the sense strand in the expected *spf^{ash} OTC* mutation site 54 nt upstream of the predicted CRISPR-Cas9 cleavage site (accounting for the size of the donor-specific *Age*I insert 'ACCGGT'), with up to two mismatches with the 18-nt intronic portion of the reference sequence adjacent to the *spf^{ash} OTC* mutation site. A read was counted as having "Partial HDR" if it did not meet the criteria for "Perfect HDR" and "Read with a ′G‴ and if there was a perfect match with an 18-nt sequence from the donor, starting with the donor-specific 'CACCAA' at the 3' end of the target site and ending with the donor-specific *Age*I insert 'ACCGGT'.

### L. Statistical analysis

Test and control vectors were evaluated in at least 3 mice per group at each time point to ensure reproducibility. Sample sizes are noted in figure legends. All animals with successful temporal vein injection were included in the study analysis. Those with unsuccessful injection were excluded. Injection success was determined according to vector genome copies in liver via qPCR, where animals with vector genome copies <10% of the mean value of the dosing group at the same time point were considered to be unsuccessful.

Statistical analysis was performed with GraphPad Prism 6.03 for Windows. The Dunn's multiple comparisons test was used to compare a number of variables with a single control. The Mann-Whitney test was used to determine differences between two groups. The Mantel-Cox test was used to test the survival distributions for differences. Group averages were presented as mean ± S.E.M.

### M. RFLP knock-in and targeting assays

Genomic DNA of MC57G cells co-transfected with pX330 and OTC donor template was extracted using the QuickExtract^{™} DNA extraction solution (Epicentre Biotechnologies). RFLP assays were performed to detect homology direct repair (HDR). Briefly, the genomic DNA was amplified by using the HDR-Fwd and HDR-Rev primers. The HDR-Fwd and HDR-Rev primers are designed to anneal outside of the region of homology between the OTC donor template and targeted genomic region. The PCR products (30 cycles, 67 °C annealing and 1 min extension at 72 °C) were purified by QIAQuick PCR purification kit (Qiagen) and digested with AgeI. The digested product was loaded on a 4-20% gradient polyacrylamide TBE gel and stained with SYBR Gold dye. The cleavage products was imaged and quantified as described above for the SURVEYOR^{®} assay. All PCR reactions were performed using Phusion^{®} High-fidelity DNA polymerase (New England BioLabs) in conjunction with HF Buffer and 3% dimethylsulphoxide.

A complete analysis of both the SpCas9 and SaCas9 vector systems in *spf^{ash}* animals in which samples were obtained 1, 3 and 8 weeks following vector infusion was performed. Controls included samples from wild type litter mates, and *spfash* mice that were either untreated or administered AAV8.Cas9 and AAV8.donor without guide RNA (called untargeted). Untreated and untargeted *spf^{ash}* animals are referred to as *spf^{ash}* controls, while *spf^{ash}* mice infused with AAV8.SaCas9 plus AAV8.sgRNA1.donor are referred to as treated animals. Pilot experiments elucidated optimal conditions of vector infusion with respect to doses and ratios of the two vectors (FIG 5); all subsequent experiments in newborns were conducted with 5×10¹¹ GC of AAV8.sgRNA1.donor (or AAV8.control.donor) and 5×10¹⁰ GC of AAV8.SaCas9.

To assess *in vivo* indel formation and HDR, liver DNAs were recovered from mice at 3 weeks (n=3) and 8 weeks (n=3) after neonatal vector treatment, followed by amplification of the OTC target region by nested PCR and deep sequencing; similar analyses were conducted on DNAs from an untreated *spf^{ash}* mouse to assess background due to sequencing errors. Table 5 below summarizes the data. Following gene correction, indels were detected in 31% (26.5% - 35.5%) of OTC alleles from the 6 treated animals (Table 5). More detailed studies in two treated mice indicated that over 90% of the deletions were less than 20 bp and only 1% extended into the adjacent exon. HDR-based correction of the G-to-A mutation was observed in 10% (6.7% - 20.1%) of OTC alleles from 6 treated animals. Analysis of amplified DNA between the G-to-A mutation and the donor-specific, altered PAM located 51 nt into the adjacent intron showed that approximately 83% of corrected alleles contained only donor derived-sequences between these two landmarks (reads with perfect HDR), while 3.5% of total OTC alleles had evidence of incomplete HDR events (reads with partial HDR). HDR-mediated targeted modifications were also estimated by the presence of a restriction-fragment length polymorphism (RFLP) introduced into the donor DNA in three animals harvested at each of the three time points. The average rate of HDR was 2.6% at 1 week, 18.5% at 3 weeks, and 14.3% at 8 weeks, confirming the high rate of HDR observed by deep sequencing (FIG 6A).

**Table 5. Summary of the frequencies of indels, correction of OTC spf^{ash} mutation, and HDR in animals treated with the dual AAV genetargeting vectors.**

| ID | Treatment | Time after treatment (week) | Indel (%) | *OTC* reads with a 'G' (%) | Reads with Perfect HDR (%) | Reads with Partial HDR (%) |
|---|---|---|---|---|---|---|
| 4001 | Neonatal, sgRNA1 | 3 | 26.5 | 20.1 | 17.2 | 7.6 |
| 4003 | Neonatal, sgRNA1 | 3 | 35.5 | 7.2 | 6.4 | 2.7 |
| 307 | Neonatal, sgRNA1 | 3 | 30.5 | 8.9 | 7.2 | 2.9 |
| 835 | Neonatal, sgRNA1 | 8 | 26.7 | 6.8 | 5.5 | 2.2 |
| 836 | Neonatal, sgRNA1 | 8 | 29.6 | 10.8 | 8.9 | 3.3 |
| 844 | Neonatal, sgRNA1 | 8 | 34.4 | 6.7 | 5.4 | 2.4 |
| 630 | Adult, untargeted, low dose | 3 | 0.3 | 0.02 | 0.01 | 0.01 |
| 637 | Adult, sgRNA1, low dose | 3 | 50.3 | 0.3 | 0.2 | 0.1 |
| 640 | Adult, sgRNA1, low dose | 3 | 45.0 | 0.3 | 0.3 | 0.1 |
| 641 | Adult, sgRNA 1, low dose | 3 | 38.5 | 0.2 | 0.1 | 0.04 |
| 658 | Adult, untargeted, high dose | 2 | 0.1 | 0.03 | 0.02 | 0.01 |
| 648 | Adult, sgRNA1, high dose | 2 | 43.6 | 1.8 | 1.5 | 0.3 |
| 649 | Adult, sgRNA1, high dose | 2 | 48.5 | 2.1 | 1.7 | 0.4 |
| 653 | Adult, sgRNA1, high dose | 2 | 34.0 | 1.3 | 1.1 | 0.2 |
| | Untreated *spf^{ash}* | n/a | 0.04 | 0.002 | 0.001 | 0.004 |

The algorithm described in www.benchling.com identified 49 potential off-target sites for sgRNA1; the top 15 sites most likely to create DSBs were amplified by PCR and subjected to deep sequencing (See following Table 6). The background from DNA of untreated animals due to sequencing error varied between sites, although it was usually a fraction of a percent. Samples from treated animals did not demonstrate evidence of indels that were above this background.

**Table 6. Full list of off-target sites.**

| ID | Sequence | SEQ ID NO: | PAM | Score | On-target | Chromosome |
|---|---|---|---|---|---|---|
| sgRNA1 | TCTCTTTTAAACTAACCCAT | 99 | CAGAG | 100.00 | TRUE | X |
| OT1 | TATCTTTTCAACTAACCCAA | 100 | TAGGA | 1.14 | FALSE | chr17 |
| OT2 | TTTCTTTTATACTAGCCCAT | 101 | AGGGG | 0.86 | FALSE | chrX |
| OT3 | TCTTTTTTTAAATAACCCAT | 102 | TAGAA | 0.86 | FALSE | chr8 |
| OT4 | TGGCATTTAAACTAACCCAA | 103 | CTGGG | 0.85 | FALSE | chr5 |
| OT5 | TCTTCATGAAACTAACCCAT | 104 | CTGAA | 0.83 | FALSE | chr19 |
| OT6 | ATTTTTTTAAACAAACCCAT | 105 | CTGGA | 0.66 | FALSE | chr2 |
| OT7 | GATCTTGTAATCTAACCCAT | 106 | GTGAA | 0.63 | FALSE | chr6 |
| OT8 | CCTTTTATAATCTAACCCAT | 107 | GTGAA | 0.62 | FALSE | chr9 |
| OT9 | TCTGTTTTAAAGTAACCCTT | 108 | CAGGG | 0.59 | FALSE | chr1 |
| OT10 | TTGCTTTTCAACTAACCCAA | 109 | GTGGG | 0.54 | FALSE | chr8 |
| OT11 | AATATTTTAAACTATCCCAT | 110 | GTGAG | 0.48 | FALSE | chr12 |
| OT12 | TTACTTTAAAACTATCCCAT | 111 | CTGAA | 0.48 | FALSE | chrX |
| OT13 | ACACTTTTAAAATAACCCAG | 112 | ATGAA | 0.47 | FALSE | chr8 |
| OT14 | AATCTTCTAAACCAACCCAT | 113 | TGGAG | 0.46 | FALSE | chr6 |
| OT15 | TCTTTATTAGACTAACCCAG | 114 | CTGAG | 0.46 | FALSE | chrX |
| OT16 | TTTCATTTAACATAACCCAT | 115 | ATGGA | 0.46 | FALSE | chr15 |
| OT17 | TCTTTTTAGAAGTAACCCAT | 116 | GTGAA | 0.46 | FALSE | chr6 |
| OT18 | TCCCTTTTTCACTAACCCAC | 117 | CAGAG | 0.46 | FALSE | chr5 |
| OT19 | TCTTTGTAAAATTAACCCAT | 118 | CAGAG | 0.46 | FALSE | chr18 |
| OT20 | TTTATTTTAAAGTAACCCAC | 119 | ATGAA | 0.45 | FALSE | chr17 |
| OT21 | TATCTTTCAAAATAACCCAC | 120 | AGGGA | 0.43 | FALSE | chr1 |
| OT22 | TATCTTGGAAACTAACCCCT | 121 | GTGGA | 0.42 | FALSE | chr7 |
| OT23 | CTTCTTTTAAACAAACCCAG | 122 | AAGGA | 0.39 | FALSE | chr8 |
| OT24 | AATCTTTTATACTAACCAAT | 123 | AAGAA | 0.39 | FALSE | chr12 |
| OT25 | TATTTTTTAAATTAACTCAT | 124 | TGGGG | 0.38 | FALSE | chr6 |
| OT26 | TTTATTTTAAAATAACACAT | 125 | TGGGA | 0.38 | FALSE | chr15 |
| OT27 | TCTCTTTCAAACTATCCCAA | 126 | GGGAA | 0.37 | FALSE | chr11 |
| OT28 | TATCTTTAAAAATAACACAT | 127 | GAGAA | 0.36 | FALSE | chrX |
| OT29 | TTTCTTTAAAAATAACACAT | 128 | GAGGG | 0.36 | FALSE | chr8 |
| OT30 | GTTCTTTTAAAAAACCCAT | 129 | AAGAA | 0.35 | FALSE | chr12 |
| OT31 | TCTGATTTAAAATAACACAT | 130 | TTGGA | 0.35 | FALSE | chr8 |
| OT32 | CCTCTTATAACCTAACCCAC | 131 | TGGGA | 0.35 | FALSE | chr6 |
| OT33 | CCTCTTGTAAGCTAACCCAC | 132 | ATGGA | 0.35 | FALSE | chr3 |
| OT34 | CCTATTTTAAAGTAACCCTT | 133 | TTGGA | 0.34 | FALSE | chr9 |
| OT35 | CATCTTGTAAACTAGCCCAT | 134 | TAGAA | 0.34 | FALSE | chr19 |
| OT36 | ACTCTTTTACAATAACACAT | 135 | TAGAA | 0.34 | FALSE | chr18 |
| OT37 | TCTCCTTTAGCCTAACTCAT | 136 | AAGAG | 0.33 | FALSE | chr19 |
| OT38 | CCTCTTTAAAAATAACCCCT | 137 | TAGAA | 0.33 | FALSE | chr2 |
| OT39 | TATCTTTGAAATAAACCCAT | 138 | GGGAA | 0.32 | FALSE | chr2 |
| OT40 | TATATTTAAAACTAAGCCAT | 139 | CTGAA | 0.32 | FALSE | chr13 |
| OT41 | TGACTTTAAAACTAAGCCAT | 140 | GTGAA | 0.32 | FALSE | chr11 |
| OT42 | CTTCTTTTCAACTATCCCAT | 141 | TTGGA | 0.31 | FALSE | chr17 |
| OT43 | GCTCTTATAAATTAACCCAG | 142 | AAGAA | 0.31 | FALSE | chr14 |
| OT44 | TTTCTTTTAAATTAAGCCAT | 143 | CTGAG | 0.31 | FALSE | chr10 |
| OT45 | TCTCTTTGTCACAAACCCAT | 144 | GTGAG | 0.31 | FALSE | chr2 |
| OT46 | TTTCTTCTAAGTTAACCCAT | 145 | TTGAG | 0.31 | FALSE | chr3 |
| OT47 | TCACTTCCAAACTACCCCAT | 146 | AAGGG | 0.30 | FALSE | chr9 |
| OT48 | TATCTTATAAAATAACCCAG | 147 | TGGGA | 0.30 | FALSE | chr14 |
| OT49 | TCAATTTTTAACTATCCCAT | 148 | TTGAG | 0.28 | FALSE | chr1 |

[These target sites are listed in order of likelihood. MLE, A maximum-likelihood estimate for the fraction of reads with true indels at the target region was applied to the off-target sequencing samples based on the computed indel rates for the Cas9/sgRNA-1 treated samples and untreated controls, as described in Hsu et. al. Nat Biotech, 31: 827-832 (2013). Data not available for OT4 due to poor sequencing quality.]

Liver homogenates were evaluated for expression of OTC by Western blot. OTC protein in most treated animals was higher than in *spf^{ash}* controls, but did not reach the level found in wild type mice (FIG 6B). Tissue sections of liver were analyzed by immunohistochemistry for OTC expression (FIG 2C-2E). Fig. 2C shows representative fluorescent micrographs of OTC expression, which were quantified in Fig. 2B for % correction using morphometric analyses. No signal (<1%) was observed in the *spf^{ash}* controls, while analysis of heterozygotes showed the predicted mosaicism (Fig. 2C). Morphometry indicated 10 to 100 fold higher numbers of OTC expressing cells in treated groups than found in the *spf^{ash}* control groups (Fig. 2B; 15% (6.8% - 24.4% at 3 weeks and 13% (7.5% - 20.1% at 8 weeks). Treated animals showed patches of OTC expressing cells (Fig. 2D) that localized within all portions of the portal axis except around central veins, which was visualized by staining for glutamine synthetase. This is the predicted distribution of endogenous OTC [MA Dingemanse, et al, Hepatology, 24: 407-411 (1996)]. A higher magnification histological view demonstrated clusters of OTC expressing hepatocytes consistent with correction followed by clonal expansion in the context of the growing liver (Fig. 2E). Direct measurements of OTC enzyme activity from liver homogenates and OTC mRNA from total cellular RNA from liver revealed similarly high levels of correction in treated animals, resulting in 20% (13.4% - 33.7% (n = 5) and 16% (11.0% - 25.4%; n =8) normal OTC activity at 3 and 8 weeks respectively (Fig. 2F), and 13% (8.6% - 21.8%, n = 5) and 9% (5.0% - 16.8% n = 8) of normal OTC mRNA at 3 and 8 weeks (Fig. 2G). Despite the decrease in OTC⁺ hepatocytes, OTC enzyme activity, and OTC mRNA expression from 3 to 8 weeks, none of these differences were statistically significant (Fig. 2b; p=0.4828, Fig. 2e; p=0.2723, Fig. 2f; p=0.1475, respectively). Liver homogenates were also evaluated for expression of OTC by Western blot. OTC protein levels in most treated animals were higher than in *spf^{ash}* controls but did not reach the levels found in wild-type mice. Overall, there was good correlation in the estimates of correction based on histology, protein, and mRNA within individual animals.

One concern about using AAV to deliver SaCas9 is its propensity to achieve stable transgene expression which is not necessary to accomplish editing and may in fact contribute to immune and/or genome toxicity. Western blot analysis showed high level SaCas9 protein at 1 week that declined to undetectable levels by 8 weeks (FIG 6B). Furthermore, immunohistochemistry revealed nuclear-localized SaCas9 protein in 21% of hepatocytes at one week, which declined to undetectable levels (< 0.1% hepatocytes) by 8 weeks (Fig. 3A). SaCas9 mRNA declined 43-fold during this 8 week period, to very low but still detectable levels (Fig. 3B). A 25-fold reduction in SaCas9 DNA during this same time interval indicates that elimination of vector genomes in the setting of the proliferating newborn liver is a primary contributor to the desired decline of SaCas9 expression (Fig. 3C).

In assessing the impact of gene correction on the clinical manifestations of OTC deficiency, we evaluated the tolerance of *spf^{ash}* mice to a one-week course of a high protein diet. As expected, measurement of blood ammonia at the end of the diet revealed a statistically significant elevation from 83 ± 9 µM (n = 13) in wild type controls to 312 ± 30 µM (n = 16) in the *spf^{ash}* controls (Fig. 3D; p<0.0001). Substantial variation in blood ammonias were found in untreated *spf^{ash}* animals after the 7-day high protein diet, which is consistent with findings in OTC deficient patients who show large fluctuations in ammonia over relatively short periods of time [D. Moscioni et al, Mol Ther, 14: 25-33 (2006)]. There was no significant difference between untreated *spf^{ash}* and untargeted *spf^{ash}* controls (Fig. 3D; p=0.83); conversely, a statistically significant 40% reduction in ammonia was observed between untreated *spf^{ash}* and treated *spf^{ash}* animals (Fig. 3D; p=0.0014). Importantly, treated *spf^{ash}* mice also demonstrated a statistically significant improvement in survival over untreated or untargeted *spf^{ash}* (Fig. 3E; p=0.03). During the course of the protein diet, 30% of both untreated *spf^{ash}* (n=20) and untargeted *spf^{ash}* (n=13) developed clinical signs of hyperammonia and died or had to be euthanized, while all of the wild type mice (n=13) and treated *spf^{ash}* mice (n=13) survived (Fig. 3E).

The surprisingly high level of correction in this study is likely due to high expression of SaCas9 with abundant donor DNA in the context of dividing cells. Infusion of AAV8 vectors into newborn monkeys demonstrated the same high peak levels of transduction and gene transfer (i.e., 92% hepatocytes expressing lacZ and 32 vector genomes per cell, respectively) as achieved in these murine studies (i.e., 21% SaCas9-expressing hepatocytes and 52 vector genomes per cell, respectively) with similar kinetics of decline, which is encouraging in terms of translation to larger species including humans.

Issues of safety relate primarily to the expression of Cas9 in the context of an sgRNA that could create off-target DSBs with carcinogenic sequelae. More extensive characterization of these potential toxicities is necessary before clinical translation can be considered, although we could not detect off-target indels at the level of sensitivity achieved by deep sequencing of likely off-target sites. Cas9 could also elicit pathologic immune responses, as has been observed in gene replacement therapies in which the transgene is a foreign protein. However, systemic delivery of AAV in a newborn helps mitigate potential immunologic adverse events for several reasons. First, expression of the prokaryotic SaCas9 protein is transient because the non-integrated vector is lost during hepatocyte proliferation. Furthermore, exposure of newborn rhesus macaques to AAV-encoded proteins has been shown to induce tolerance to these proteins thereby circumventing toxicity caused by destructive adaptive immune responses. Detailed histological analyses of liver and transaminase levels in SaCas9-treated *spf^{ash}* mice harvested at the end of the high protein diet challenge failed to reveal any pathology or toxicity (FIG 7).

### Example 2 - MPSI

### A. MPSI mouse

For use in this study, PAM sequences (NNGRRT) in proximity to the MPSI W392X mutation of the MPSI gene were identified as potential 20-nt protospacer sequences. Three sequences, sgRNA1-3 (FIG 10), were further evaluated following transfection of puromycin-containing plasmids into a mouse MC57G cell line. Low transfection efficiency in this cell line required enrichment of transfected cells by a brief exposure to puromycin. Evidence for double-strand breaks (DSBs) and the formation of indels at the desired site was demonstrated using the SURVEYOR assay.

The AAV donor plasmids are constructed by cloning in the sgRNA under the control of U6 promoter and respective donor template with approximately 1 kb of sequence flanking each side of the mutation and the corresponding PAM sequence in the donor template is mutated to reduce re-cleavage after HDR.

Initial in vivo assessment was performed in neonatal MPSWX pups targeting the liver by co-injection of AAV8 vector expressing SaCas9 under the control of liver-specific TBG promoter (AAV8.TBG.SaCas9) and AAV8.sgRNA.donor. Eight weeks after vector treatment, liver DNA were isolated for indel analysis by Surveyor assay and high frequency of indels were generated by both sgRNA2 and sgRNA3.

Western blot on tissue lysate from wild type and MPSWX mouse showed that brain is the dominant tissue that expresses IDUA enzyme as compared to heart, liver, spleen and kidney. For *in vivo* gene correction, in order to target brain, AAV9 vector expressing SaCas9 under the control of an ubiquitous promoter CBh was made. The sgRNA and donor template vectors are also packaged with AAV9 capsid for broad *in vivo* transduction and crossing the blood-brain barrier.. For *in vivo* assessment, AAV9.SaCas9 and AAV9.sgRNA.donor vectors will be co-injected into neonatal MPSI pups via temporal vein or intracerebroventricular (ICV) delivery. The correction will be characterized in treated mice: biochemistry (IDUA protein and activity), histology (GAG storage), DNA, RNA, and off-target analysis.

### B. MPSI dog

Donor template sequences are provided in the following table.

**Table 7**

| ID | Sequence(5'->3') | SEQ ID NO: | Note |
|---|---|---|---|
| dIDUA_sgRNA1_Fwd | CACCGTTCTGATGAGGGCTCCGCGG | 149 | hSpCas9 |
| dIDUA_sgRNA1_Rev | AAACCCGCGGAGCCCTCATCAGAAC | 150 | |
| dIDUA_sgRNA2_Fwd | CACCGCACCTGGTGCTCGTGGACG | 151 | |
| dlDUA_sgRNA2_Rev | AAACCGTCCACGAGCACCAGGTGC | 152 | |
| dIDUA_sgRNA3_Fwd | CACCGGGCCGCGTCCACGAGCACC | 153 | |
| dlDUA_sgRNA3_Rev | AAACGGTGCTCGTGGACGCGGCCC | 154 | |
| dIDUA_sgRNA4_Fwd | CACCGTCCACGAGCACCAGGTGCG | 155 | |
| dlDUA_sgRNA4_Rev | AAACCGCACCTGGTGCTCGTGGAC | 156 | |
| dIDUA_sgRNA5_Fwd | CACCGTGGACGCGGCCCGCGCGCTG | 157 | |
| dlDUA_sgRNA5_Rev | AAACCAGCGCGCGGGCCGCGTCCAC | 158 | |
| dIDUA_sgRNA6_Fwd | CACCGCTTCTGATGAGGGCTCCGC | 159 | |
| dlDUA_sgRNA6_Rev | AAACGCGGAGCCCTCATCAGAAGC | 160 | |

The sgRNA candidates listed in the table above will be validated on MDCK cells (canine cell line). Site-directed mutagenesis will be performed on the PAM sequence on the donor template corresponding to the selected sgRNA. The AAV donor plasmids are constructed by cloning in the sgRNA and respective donor template and used for AAV vector production. The dual AAV vector system is injected into neonatal MPSI puppies. The correction is characterized in the dogs by biochemistry, histology, DNA, RNA, and off-target analysis.

### Example 3 - hCFTR - ΔF508 human airway cells

sgRNA for SaCas9 have been selected. The sgRNA primers will be closed into pX330.SaCas9. The donor template will then be PCR cloned.

**Table 8**

| ID | Sequence(5'->3' | SEQ ID NO: | Note |
|---|---|---|---|
| hCFTR_sgRNA1_Fwd | | 161 | hSpCas9 |
| hCFTR_sgRNA1_Rev | | 162 | |
| hCFTR_sgRNA2_Fwd | | 163 | |
| hCFTR_sgRNA2_Rev | | 164 | |
| hCFTR_sgRNA3_Fwd | | 165 | |
| hCFTR_sgRNA3_Rev | | 166 | |
| hCFTR_sgRNA4_Fwd | | 167 | |
| hCFTR_sgRNA4_Rev | | 168 | |

In order to validate the sgRNAs in vitro, pX330.SaCas9-sgRNA will be transfected into 293 cells. DNA will then be isolated for Indel analysis. The AAV donor plasmids are constructed by cloning in the sgRNA and respective donor template. The resulting plasmids are used to construct AAV vector (AAV6.2). The dual AAV6.2 viral vector system is used for *in vitro* transduction on ΔF508 human airway cells and the correction in these airway cells is characterized. For *in vivo* assessment, the testing system is DF508 CFTR mouse. Vectors can be delivered systemically to neonatal mice; or to adult mouse lung. AAV serotypes can be AAV6.2 or AAV9.

### Example 4 - Correction of FIX KO phenotype in mice.

SaCas9-mediated insertion of a therapeutic gene. PAM sequences (NNGRRT) in exon 2 of the murine factor IX gene were identified as potential 20-nt protospacer sequences for use in this study. sgRNA candidates were validated in vitro for Indels following the transfection into mouse cell line and sgRNA3 showed the highest efficiency in indel generation. AAV donor vector contains U6.sgRNA, donor arms, and.hFIXco-Padua.bGH (exon 2-8) were constructed. AAV8 vectors were produced and injected into neonatal FIX-KO pups and adult FIX-KO mice. Expression of Cas9 will be characterized, and the correction of the phenotype will be determined by measuring FIX expression, and performing histological examinations. DNA and RNA species will also be characterized, and off-target analyses will be performed.

**Table 9**

| ID | Sequence(5'->3') | SEQ ID NO: | Note |
|---|---|---|---|
| mFIXsgRNA 1_Fwd | | 169 | mFIX target sequence 1 for hSaCas9 |
| mFIXsgRNA 1_Rev | | 170 | |
| mFIXsgRNA 2_Fwd | | 171 | mFIX target sequence 2 for hSaCas9 |
| mFIXsgRNA 2_Rev | | 172 | |
| mFIXsgRNA 3_Fwd | | 173 | mFIX target sequence 3 for hSaCas9 |
| mFIXsgRNA 3_Rev | | 174 | |
| mFIX_Point M_F | | 175 | FIX PCR primers |
| mFIX_Point M_R | | 176 | |

### Example 5- Insertion of therapeutic gene in rhesus macaques.

sgRNA candidates have been identified and cloned into pX330.SaCas9 plasmid, and donor template is being de-novo synthesized. *In vitro* validation of the sgRNA candidates will be performed in LLC-MK2 cell line at the optimal transfection conditions. Cells will be transfected with pX330-sgRNA plasmid in the presence of Cas9. DNA will be purified and assessed for the presence of Indels. Based on Indel assessments, preferred sgRNA candidates will be identified and characterized in rhesus macaques. Selected sgRNA will be used for construction of the AAV donor plasmid in which hFIXco-Padua is flanked by HDR arms. Newborn monkeys will be injected systemically using dual AAV system, and gene insertion will be characterized by measuring hFIX expression, and by measuring activated partial thromboplastin time (APPT).

**Table 10**

| ID | Sequence(5'->3' | SEQ ID NO: | Note |
|---|---|---|---|
| rhFIXsgRNA1_Fwd | | 177 | rhFIX target seqence 1 for hSaCas9 |
| rhFIXsgRNA 1_Rev | | 178 | |
| rhFIXsgRNA2_Fwd | | 179 | rhFIX target seqence 2 for hSaCas9 |
| rhFIXsgRNA2_Rev | | 180 | |
| rhFIXsgRNA3_Fwd | | 181 | rhFIX target seqence 3 for hSaCas9 |
| rhFIXsgRNA3_Rev | | 182 | |

### Example 6 - Gene Splicing Using dual AAV Cpf1 CRISPR System

A U6-gRNA scaffold-terminator was cloned into AsCpf1 and LbCpf1 (plasmids containing Cpfl obtained from Addgene (a non-profit plasmid repository, www.addgene.org), following which the plasmids with DNMT1 target sequences [L. Swiech, et al, Nature biotechnology, 33: 102-106 (2015), e-published 19 October 2014] as a positive control were tested in 293 cells. The Surveyor data showed both constructs functioned as designed. Next, Cpfl protospacer sequences near the OTC *spf^{ash}* mutation will be verified in vitro by Surveyor assay. The most efficient sgRNAs will be cloned into pAAV donor plasmids with the corresponding PAM sequence mutated. AAV8 vectors expressing AsCpf1 and LbCpf1 driven by ABP2.TBG-S 1 as enhancer/promoter for liver applications (OTC and mFIX-KO as first models) have been generated (FIGs 11 and 12).

### Example 7 - Gene Inactivation Using AAV SaCas9 and PCSK9 sgRNA

Seven PCSK9 sgRNAs that target both monkey and human PCSK9 exons were selected (see Table below) and cloned into pX330.SaCas9 plasmid. Plasmids were transfected into monkey cell line LLC-MK2 and 293 cells. DNA was isolated and target regions were amplified by PCR for Surveyor assay. sgRNA3 showed highest efficiency in both monkey and human cell lines and was selected as the top candidate.

**Table 11**

| ID | Sequence (5'->3') | SEQ ID NO: | Note |
|---|---|---|---|
| EGFP-sgRNAF | CACCGTCGTGCTGCTTCATGTGGT | 183 | Control |
| EGFP-sgRNAR | AAACACCACATGAAGCAGCACGAC | 184 | |
| PCSK9_sgRNA1F | CACCGATGCTCTGGGCAAAGACAG | 185 | Exon3 |
| PCSK9_sgRNA1R | AAACCTGTCTTTGCCCAGAGCATC | 186 | |
| PCSK9_sgRNA2F | CACCGATCTCCTAGACACCAGCATA | 187 | Exon4 |
| PCSK9_sgRNA2R | AAACTATGCTGGTGTCTAGGAGATC | 188 | |
| PCSK9_sgRNA3F | CACCGAAGTTGGTCCCCAAAGTCCC | 189 | Exon7 |
| PCSK9_sgRNA3R | AAACGGGACTTTGGGGACCAACTTC | 190 | |
| PCSK9_sgRNA4F | CACCGCAGCACCTGCTTTGTGTCA | 191 | Exon7 |
| PCSK9_sgRNA4R | AAACTGACACAAAGCAGGTGCTGC | 192 | |
| PCSK9_sgRNA5F | CACCGAGTCTCTGCCTCAACTCGGC | 193 | Exon8 |
| PCSK9_sgRNA5R | AAACGCCGAGTTGAGGCAGAGACTC | 194 | |
| PCSK9 _sgRNA6F | CACCGATGACATCTTTGGCAGAGAA | 195 | Exon8 |
| PCSK9_sgRNA6R | AAACTTCTCTGCCAAAGATGTCATC | 196 | |
| PCSK9_sgRNA7F | CACCGCCTGGTTCCCTGAGGACCA | 197 | Exon8 |
| PCSK9_sgRNA7R | AAACTGGTCCTCAGGGAACCAGGC | 198 | |

sgRNA3 was cloned into an AAV plasmid contains SaCas9 driven by ABP2.TBG-S1, a liver-specific enhancer/promoter (FIG 13A). AAV8 vector was produced and intravenously delivered to a rhesus monkey at the dose of 3×10¹³ GC/kg. Two weeks following vector treatment, serum PCSK9 levels were reduced by 40% as compared to day 0 (FIG 13B). Serum cholesterol, HDL, LDL and triglyceride levels are currently being monitored. DNA and RNA analysis will be performed on liver biopsy samples.

This application contains sequences and a sequence listing, which is hereby incorporated by reference.

Although the foregoing invention has been described in some detail by way of illustration and example for purposes of clarity of understanding, it will be readily apparent to those of ordinary skill in the art in light of the teachings of this invention that certain changes and modifications can be made thereto without departing from the scope of the appended claims.

**Table 12**

| (Sequence Listing Free Text) | | |
|---|---|---|
| The following information is provided for sequences containing free text under numeric identifier <223>. | | |
| SEQ ID NO: (containing free text) | Free text under <223> | |
| 1 | <220> | |
| | <221> | misc_feature |
| | <222> | (4)..(23) |
| | <223> | sgRNA2 |
| | <220> | |
| | <221> | misc_feature |
| | <222> | (24)..(29) |
| | <223> | PAM |
| | <220> | |
| | <221> | misc_feature |
| | <222> | (52)..(52) |
| | <223> | spfash mutation site G->A |
| | <220> | |
| | <221> | misc_feature |
| | <222> | (77)..(96) |
| | <223> | sgRNA3 |
| | <220> | |
| | <221> | misc_feature |
| | <222> | (91)..(96) |
| | <223> | PAM (reverse complement sequence) |
| | <220> | |
| | <221> | misc_feature |
| | <222> | (97)..(102) |
| | <223> | PAM |
| | <220> | |
| | <221> | misc_feature |
| | <222> | (97)..(116) |
| | <223> | sgRNA1 (reverse complement sequence) |

| SEQ ID NO: (containinq free text) | Free text under <223> | |
|---|---|---|
| 2 | <220> | |
| | <221> | misc_feature |
| | <222> | (3)..(21) |
| | <223> | sgRNA1 |
| | <220> | |
| | <221> | misc_feature |
| | <222> | (22)..(27) |
| | <223> | PAM |
| | <220> | |
| | <221> | misc_feature |
| | <222> | (98)..(103) |
| | <223> | PAM (reverse complement sequence) |
| | <220> | |
| | <221> | misc_feature |
| | <222> | (102)..(104) |
| | <223> | W392X mutation:TGG->TGA |
| | <220> | |
| | <221> | misc_feature |
| | <222> | (104)..(123) |
| | <223> | sgRNA3 (reverse complement sequence) |
| | <220> | |
| | <221> | misc_feature |
| | <222> | (130)..(149) |
| | <223> | sgRNA2 |
| | <220> | |
| | <221> | misc_feature |
| | <222> | (150)..(155) |
| | <223> | PAM |
| 3 | TBG variant TBG-S 1 | |
| 4 | novel enhancer ABPS2 | |
| 5 | target sequence | |
| 6 | target sequence | |
| 7 | target sequence | |
| 8 | target sequence | |
| 9 | target sequence | |
| 10 | target sequence | |
| 12 | spfash G->A mutation sequence | |
| 13 | OTC donor sequence (SpCas9, 892-983 bp) | |

| SEQ ID NO: (containinq free text) | Free text under <223> | |
|---|---|---|
| 14 | OTC donor sequence (SaCas9, 892-989 bp) | |
| 15 | OTC sgRNA1_Fwd | |
| 16 | OTC sgRNA1_Rev | |
| 17 | OTC sgRNA2 Fwd | |
| 18 | OTC sgRNA2 Rev | |
| 19 | OTC sgRNA3 Fwd | |
| 20 | OTC sgRNA3 Rev | |
| 21 | HDR-Fwd | |
| 22 | HDR-Rev | |
| 23 | OTC_PointM_F | |
| 24 | OTC PointM R | |
| 25 | mOTC hSpCas9 sgRNA1 Fwd | |
| 26 | mOTC hSpCas9 sgRNA1 Rev | |
| 27 | mOTC hSpCas9 sgRNA2 Fwd | |
| 28 | mOTC hSpCas9 sgRNA2_Rev | |
| 29 | mOTC hSpCas9 sgRNA3 Fwd | |
| 30 | mOTC hSpCas9 sgRNA3 Rev | |
| 31 | mOTC hSaCas9 sgRNA1 Fwd | |
| 32 | mOTC hSaCas9 sgRNA1 Rev | |
| 33 | mOTC hSaCas9 sgRNA2 Fwd | |
| 34 | mOTC hSaCas9 sgRNA2 Rev | |
| 35 | mOTC hSaCas9 sgRNA3 Fwd | |
| 36 | mOTC hSaCas9 sgRNA3 Rev | |
| 37 | OTC OT1F1 | |
| 38 | OTC OT1R1 | |
| 39 | OTC OT1F2 | |
| 40 | OTC OT1R2 | |
| 41 | OTC OT2F1 | |
| 42 | OTC OT2R1 | |
| 43 | OTC OT2F2 | |
| 44 | OTC OT2R2 | |
| 45 | OTC OT3F1 | |
| 46 | OTC OT3R1 | |
| 47 | OTC_OT3F2 | |
| 48 | OTC OT3R2 | |
| 49 | OTC OT4F1 | |
| 50 | OTC OT4R1 | |
| 51 | OTC OT4F2 | |
| 52 | OTC OT4R2 | |
| 53 | OTC OT5F1 | |
| 54 | OTC OT5R1 | |
| 55 | OTC OT5F2 | |
| 56 | OTC OT5R2 | |
| 57 | OTC OT6F1 | |
| 58 | OTC OT6R1 | |
| 59 | OTC OT6F2 | |
| 60 | OTC OT6R2 | |
| 61 | OTC OT7F1 | |
| 62 | OTC OT7R1 | |
| 63 | OTC OT7F2 | |
| 64 | OTC OT7R2 | |
| 65 | OTC_OT8F1 | |
| 66 | OTC OT8R1 | |
| 67 | OTC OT8F2 | |
| 68 | OTC OT8R2 | |
| 69 | OTC OT9F 1 | |
| 70 | OTC OT9R1 | |
| 71 | OTC OT9F2 | |
| 72 | OTC OT9R2 | |
| 73 | OTC OT10F1 | |
| 74 | OTC OT10R1 | |
| 75 | OTC OT10F2 | |
| 76 | OTC OT10R2 | |
| 77 | OTC OT11F1 | |
| 78 | OTC OT11R1 | |
| 79 | OTC OT11F2 | |
| 80 | OTC OT11R2 | |
| 81 | OTC_OT12F | |
| 82 | OTC OT12R | |
| 83 | OTC OT13F1 | |
| 84 | OTC OT13R1 | |
| 85 | OTC OT13F2 | |
| 86 | OTC OT13R2 | |
| 87 | OTC OT14F1 | |
| 88 | OTC OT14R1 | |
| 89 | OTC OT14F2 | |
| 90 | OTC OT14R2 | |
| 91 | OTC OT15F1 | |
| 92 | OTC_OT15R1 | |
| 93 | OTC OT15F2 | |
| 94 | OTC OT15R2 | |
| 95 | OTC OT16F1 | |
| 96 | OTC_OT16R1 | |
| 97 | OTC OT16F2 | |
| 98 | OTC OT16R2 | |
| 99 | sgRNA1 | |
| 100 | OT1 | |
| 101 | OT2 | |
| 102 | OT3 | |
| 103 | OT4 | |
| 104 | OT5 | |
| 105 | OT6 | |
| 106 | OT7 | |
| 107 | OT8 | |
| 108 | OT9 | |
| 109 | OT10 | |
| 110 | OT11 | |
| 111 | OT12 | |
| 112 | OT13 | |
| 113 | OT14 | |
| 114 | OT15 | |
| 115 | OT16 | |
| 116 | OT17 | |
| 117 | OT18 | |
| 118 | OT19 | |
| 119 | OT20 | |
| 120 | OT21 | |
| 121 | OT22 | |
| 122 | OT23 | |
| 123 | OT24 | |
| 124 | OT25 | |
| 125 | OT26 | |
| 126 | OT27 | |
| 127 | OT28 | |
| 128 | OT29 | |
| 129 | OT30 | |
| 130 | OT31 | |
| 131 | OT32 | |
| 132 | OT33 | |
| 133 | OT34 | |
| 134 | OT35 | |
| 135 | OT36 | |
| 136 | OT37 | |
| 137 | OT38 | |
| 138 | OT39 | |
| 139 | OT40 | |
| 140 | OT41 | |
| 141 | OT42 | |
| 142 | OT43 | |
| 143 | OT44 | |
| 144 | OT45 | |
| 145 | OT46 | |
| 146 | OT47 | |
| 147 | OT48 | |
| 148 | OT49 | |
| 149 | dIDUA_sgRNA1_Fwd | |
| 150 | dIDUA_sgRNA1_Rev | |
| 151 | dIDUA_sgRNA2_Fwd | |
| 152 | dIDUA_sgRNA2_Rev | |
| 153 | dIDUA_sgRNA3_Fwd | |
| 154 | dIDUA_sgRNA3_Rev | |
| 155 | dIDUA_sgRNA4_Fwd | |
| 156 | dIDUA_sgRNA4_Rev | |
| 157 | dIDUA_sgRNA5_Fwd | |
| 158 | dIDUA_sgRNA5_Rev | |
| 159 | dIDUA_sgRNA6_Fwd | |
| 160 | dIDUA_sgRNA6_Rev | |
| 161 | hCFTR sgRNA1 Fwd | |
| 162 | hCFTR sgRNA1 Rev | |
| 163 | hCFTR sgRNA2 Fwd | |
| 164 | hCFTR_sgRNA2 Rev | |
| 165 | hCFTR_sgRNA3 Fwd | |
| 166 | hCFTR_sgRNA3 Rev | |
| 167 | hCFTR_sgRNA4_Fwd | |
| 168 | hCFTR_sgRNA4_Rev | |
| 169 | mFIXsgRNA1_Fwd | |
| 170 | mFIXsgRNA1_Rev | |
| 171 | mFIXsgRNA2_Fwd | |
| 172 | mFIXsgRNA2_Rev | |
| 173 | mFIXsgRNA3_Fwd | |
| 174 | mFIXsgRNA3_Rev | |
| 175 | mFIX_PointM_F | |
| 176 | mFIX PointM R | |
| 177 | rhFIXsgRNA1_Fwd | |
| 178 | rhFIXsgRNA1_Rev | |
| 179 | rhFIXsgRNA2_Fwd | |
| 180 | rhFIXsgRNA2_Rev | |
| 181 | rhFIXsgRNA3_Fwd | |
| 182 | rhFIXsgRNA3_Rev | |
| 183 | EGFP-sgRNAF | |
| 184 | EGFP-sgRNAR | |
| 185 | PCSK9 sgRNA1F | |
| 186 | PCSK9 sgRNA1R | |
| 187 | PCSK9 sgRNA2F | |
| 188 | PCSK9 sgRNA2R | |
| 189 | PCSK9 sgRNA3F | |
| 190 | PCSK9 sgRNA3R | |
| 191 | PCSK9 sgRNA4F | |
| 192 | PCSK9 sgRNA4R | |
| 193 | PCSK9_sgRNA5F | |
| 194 | PCSK9 sgRNA5R | |
| 195 | PCSK9_sgRNA6F | |
| 196 | PCSK9 sgRNA6R | |
| 197 | PCSK9 sgRNA7F | |
| 198 | PCSK9 sgRNA7R | |

## Claims

1. A dual vector system comprising:
(a) a gene editing AAV vector comprising a Cas9 gene under control of regulatory sequences which direct expression of the Cas9 in a target cell comprising a targeted gene which has one or more mutations resulting in a disorder;
(b) a targeting AAV vector comprising one or more sgRNA and a donor template, wherein the one or more sgRNA individually comprise at least 20 nucleotides which specifically bind to a selected site in the targeted genes and which selected site is 5' to a protospacer-adjacent motif (PAM) which is specifically recognized by the Cas9, and wherein the donor template comprises nucleic acid sequences which replaces at least one of the mutations in the targeted gene;
wherein the ratio of the gene editing AAV vector (a) to the targeting AAV vector (b) is such that the targeting AAV vector (b) is in excess of the gene editing AAV vector (a), as determined based on vector genome copies of (a) and (b), and wherein the target cells are hepatocytes or liver cells.

2. The dual vector system according to claim 1, wherein the ratio of the gene editing AAV vector (a) to the targeting AAV vector (b) is 1:3 to 1:100, or 1:10, as determined based on genome copies of (a) and (b).

3. The dual vector system according to claim 1, wherein the sgRNA of the targeting AAV vector (b) comprises 24 nucleotides to 28 nucleotides.

4. The dual vector system according to claim 1, wherein the donor template of the targeting AAV vector (b) is a full-length gene encoding a functioning protein or enzyme, or wherein the donor template of the targeting AAV vector (b) encodes a partial protein or enzyme and is designed to complement a defective gene and to allow production of a functioning protein in the target cell.

5. The dual vector system according to claim 1, wherein the sgRNA of the targeting AAV vector (b) is designed to target an intron of the targeted gene, such that the mutation is corrected by the donor.

6. The dual vector system according to claim 1, wherein the sgRNA of the targeting AAV vector (b) is designed to target a gene, such that the donor template is inserted upstream of the gene defect.

7. The dual vector system according to claim 1, wherein the targeting AAV vector (b) comprises more than one sgRNA.

8. The dual vector system according to claim 1, wherein the gene editing AAV vector of (a) and the targeting AAV vector of (b) have the same AAV capsid.

9. The dual vector system according to claim 8, wherein the AAV capsid is selected from AAV8, AAV9, rhl0, AAV6.2, AAV3B, hu37, and/or rh64.

10. The dual vector system according to claim 1, wherein Cas9 is selected from *Staphylococcus aureus* or *Streptococcus pyogenes* Cas9.

11. The dual vector system according to claim 1, wherein the Cas9 gene is under the control of a tissue-specific promoter, optionally wherein the tissue-specific promoter is a liver-specific promoter, optionally wherein the liver-specific promoter is a human thyroxin-binding globulin (TBG) promoter.

12. The dual vector system according to claim 1, wherein the Cas9 gene is under the control of a constitutive promoter.

13. The dual vector system according to any of claims 1 to 12 for use in a method of treating a disorder in humans.

14. A dual vector system for use in a method of treating a liver metabolic disorder in neonates, comprising:
(a) a gene editing AAV vector comprising a Cas9 gene under control of regulatory sequences which direct expression of the Cas9 in a hepatocyte comprising a targeted gene which has one or more mutations resulting in a liver metabolic disorder; and
(b) a targeting AAV vector comprising sgRNA and a donor template, wherein the sgRNA comprises at least 20 nucleotides which specifically bind to a selected site in the targeted genes and which selected site is 5' to a protospacer-adjacent motif (PAM) which is specifically recognized by the Cas9, and wherein the donor template comprises nucleic acid sequences which replaces at least one of the mutations in the targeted gene;
wherein the ratio of genome copies of the gene editing AAV vector (a) to genome copies of the targeting AAV vector (b) is such that the genome copies of the targeting AAV vector (b) is in excess of the gene editing AAV vector (a).

15. The dual vector system according to claim 14, wherein the gene editing AAV vector (a) and the targeting AAV vector (b) are delivered essentially simultaneously via the same route.

16. The dual vector system according to claim 14, wherein the gene editing AAV vector (a) is suspended in a vehicle for injection at a concentration of 2 × 10¹¹ GC/mL to 2 × 10¹² GC/mL, and/or wherein the targeting AAV vector (b) is suspended in a vehicle for injection at a concentration of about 2 × 10¹² GC/mL to about 1 × 10¹³ GC/mL.

17. The dual vector system according to claim 14, wherein the liver metabolic disorder is ornithine transcarbamylase (OTC), wherein an OTC gene has a mutation.

18. The dual vector system according to claim 17, wherein the sgRNA of the targeting AAV vector (b) is targeted to a G/A mutation site in exon4 of the OTC gene, located at nucleotide 243 based on the numbering of the wild-type OTC gene.

19. A dual vector system comprising:
(a) a gene editing AAV vector comprising a Cas9 gene under control of regulatory sequences which direct expression of the Cas9 in a liver cell or hepatocyte comprising a targeted gene which has one or more mutations resulting in a disorder;
(b) a targeting AAV vector comprising one or more sgRNA and a donor template, wherein the one or more sgRNA individually comprise at least 20 nucleotides which specifically bind to a selected site in the targeted gene, and which selected site is 5' to a protospacer-adjacent motif (PAM) which is specifically recognized by the Cas9, and wherein the donor template comprises a full-length gene encoding a functioning protein or enzyme or a partial protein or enzyme and is designed to complement the targeted gene of (a) to allow production of a functioning protein in the target cell,
wherein the ratio of the gene editing AAV vector (a) to the targeting AAV vector (b) is such that the targeting AAV vector (b) is in excess of the gene editing AAV vector (a), as determined based on genome copies of (a) and (b).

20. The vector system according to claim 19, wherein the ratio of the gene editing AAV vector (a) to the targeting AAV vector (b) is 1:3 to 1:100, or 1:10, as determined based on genome copies of (a) and (b).

21. The vector system according to claim 19, wherein the sgRNA of the targeting AAV vector (b) comprises 24 nucleotides to 28 nucleotides.

## Patentansprüche

1. Duales Vektorsystem, umfassend:
(a) einen Gen-Editing-AAV-Vektor, der ein Cas9-Gen unter der Kontrolle von Regulatorsequenzen, die die Expression des Cas9 in einer ein anvisiertes Gen, das eine oder mehrere zu einer Störung führende Mutationen aufweist, umfassenden Zielzelle steuern, umfasst;
(b) einen Targeting-AAV-Vektor, der eine oder mehrere sgRNA und eine Donor-Matrize umfasst, wobei die eine oder mehreren sgRNA jeweils wenigstens 20 Nukleotide umfassen, die an eine ausgewählte Stelle in den anvisierten Genen spezifisch binden, wobei die ausgewählte Stelle 5' zu einem PAM (Protospacer-Adjacent Motif) liegt, das vom Cas9 spezifisch erkannt wird, und wobei die Donor-Matrize Nukleinsäuresequenzen umfasst, wodurch wenigstens eine der Mutationen im anvisierten Gen ersetzt wird;
wobei das Verhältnis des Gen-Editing-AAV-Vektors (a) zum Targeting-AAV-Vektor (b) solcher Art ist, dass der Targeting-AAV-Vektor (b) im Überschuss zum Gen-Editing-AAV-Vektor (a) vorliegt, wie bezogen auf Vektorgenomkopien von (a) und (b) bestimmt, und wobei es sich bei den Zielzellen um Hepatozyten oder Leberzellen handelt.

2. Duales Vektorsystem nach Anspruch 1, wobei das Verhältnis des Gen-Editing-AAV-Vektors (a) zum Targeting-AAV-Vektor (b) bei 1:3 bis 1:100 oder 1:10 liegt, wie bezogen auf Genomkopien von (a) und (b) bestimmt.

3. Duales Vektorsystem nach Anspruch 1, wobei die sgRNA des Targeting-AAV-Vektors (b) 24 Nukleotide bis 28 Nukleotide umfasst.

4. Duales Vektorsystem nach Anspruch 1, wobei es sich bei der Donor-Matrize des Targeting-AAV-Vektors (b) um ein Volllängen-Gen handelt, das ein funktionsfähiges Protein oder Enzym codiert, oder wobei die Donor-Matrize des Targeting-AAV-Vektors (b) ein Teilprotein bzw. -enzym codiert und zur Komplementierung eines defekten Gens und zur Ermöglichung der Produktion eines funktionsfähigen Proteins in der Zielzelle vorgesehen ist.

5. Duales Vektorsystem nach Anspruch 1, wobei die sgRNA des Targeting-AAV-Vektors (b) zum Anvisieren eines Introns des anvisierten Gens vorgesehen ist, so dass die Mutation durch den Donor korrigiert wird.

6. Duales Vektorsystem nach Anspruch 1, wobei die sgRNA des Targeting-AAV-Vektors (b) zum Anvisieren eines Gens vorgesehen ist, so dass die Donor-Matrize stromaufwärts vom Gendefekt inseriert wird.

7. Duales Vektorsystem nach Anspruch 1, wobei der Targeting-AAV-Vektor (b) mehr als eine sgRNA umfasst.

8. Duales Vektorsystem nach Anspruch 1, wobei der Gen-Editing-AAV-Vektor unter (a) und der Targeting-AAV-Vektor unter (b) das gleiche AAV-Capsid aufweisen.

9. Duales Vektorsystem nach Anspruch 8, wobei das AAV-Capsid ausgewählt ist aus AAV8, AAV9, rh10, AAV6.2, AAV3B, hu37 und/oder rh64.

10. Duales Vektorsystem nach Anspruch 1, wobei Cas9 ausgewählt ist aus *Staphylococcus aureaus-* oder *Staphylococcus pyogenes*-Cas9.

11. Duales Vektorsystem nach Anspruch 1, wobei das Cas9-Gen unter der Kontrolle eines gewebespezifischen Promotors steht, gegebenenfalls wobei es sich bei dem gewebespezifischen Promotor um einen leberspezifischen Promotor handelt, gegebenenfalls wobei es sich bei dem leberspezifischen Promotor um einen menschlichen TBG(Thyroxin-Binding Globulin)-Promotor handelt.

12. Duales Vektorsystem nach Anspruch 1, wobei das Cas9-Gen unter der Kontrolle eines konstitutiven Promotors steht.

13. Duales Vektorsystem nach einem der Ansprüche 1 bis 12 zur Verwendung bei einem Verfahren zur Behandlung einer Störung beim Menschen.

14. Duales Vektorsystem zur Verwendung bei einem Verfahren zur Behandlung einer Leberstoffwechselstörung bei Neugeborenen, umfassend:
(a) einen Gen-Editing-AAV-Vektor, der ein Cas9-Gen unter der Kontrolle von Regulatorsequenzen, die die Expression des Cas9 in einem ein anvisiertes Gen, das eine oder mehrere zu einer Leberstoffwechselstörung führende Mutationen aufweist, umfassenden Hepatozyten steuern, umfasst; und
(b) einen Targeting-AAV-Vektor, der sgRNA und eine Donor-Matrize umfasst, wobei die sgRNA wenigstens 20 Nukleotide umfasst, die an eine ausgewählte Stelle in den anvisierten Genen spezifisch binden, wobei die ausgewählte Stelle 5' zu einem PAM (Protospacer-Adjacent Motif) liegt, das vom Cas9 spezifisch erkannt wird, und wobei die Donor-Matrize Nukleinsäuresequenzen umfasst, wodurch wenigstens eine der Mutationen im anvisierten Gen ersetzt wird;
wobei das Verhältnis von Genomkopien des Gen-Editing-AAV-Vektors (a) zu Genomkopien des Targeting-AAV-Vektors (b) solcher Art ist, dass die Genomkopien des Targeting-AAV-Vektors (b) im Überschuss zum Gen-Editing-AAV-Vektor (a) vorliegen.

15. Duales Vektorsystem nach Anspruch 14, wobei der Gen-Editing-AAV-Vektor (a) und der Targeting-AAV-Vektor (b) im Wesentlichen gleichzeitig über den gleichen Weg zugeführt werden.

16. Duales Vektorsystem nach Anspruch 14, wobei der Gen-Editing-AAV-Vektor (a) in einem Vehikel zur Injektion in einer Konzentration von 2 × 10¹¹ GC/ml bis 2 × 10¹² GC/ml suspendiert ist und/oder wobei der Targeting-AAV-Vektor (b) in einem Vehikel zur Injektion in einer Konzentration von etwa 2 × 10¹² GC/ml bis etwa 1 × 10¹³ GC/ml suspendiert ist.

17. Duales Vektorsystem nach Anspruch 14, wobei es sich bei der Leberstoffwechselstörung um Ornithin-Transcarbamylase (OTC) handelt, wobei ein OTC-Gen eine Mutation aufweist.

18. Duales Vektorsystem nach Anspruch 17, wobei die sgRNA des Targeting-AAV-Vektors (b) auf eine G/A-Mutationsstelle in Exon4 des OTC-Gens gelenkt wird, die sich an Nukleotid 243 bezogen auf die Nummerierung des Wildtyp-OTC-Gens befindet.

19. Duales Vektorsystem, umfassend:
(a) einen Gen-Editing-AAV-Vektor, der ein Cas9-Gen unter der Kontrolle von Regulatorsequenzen umfasst, die die Expression des Cas9 in einer Leberzelle bzw. einem Hepatozyten steuern, die bzw. der ein anvisiertes Gen, das eine oder mehrere zu einer Störung führende Mutationen aufweist, umfasst;
(b) einen Targeting-AAV-Vektor, der eine oder mehrere sgRNA und eine Donor-Matrize umfasst, wobei die eine oder mehreren sgRNA jeweils wenigstens 20 Nukleotide umfassen, die an eine ausgewählte Stelle im anvisierten Gen spezifisch binden, wobei die ausgewählte Stelle 5' zu einem PAM (Protospacer-Adjacent Motif) liegt, das vom Cas9 spezifisch erkannt wird, und wobei die Donor-Matrize ein Gen in voller Länge, das ein funktionsfähiges Protein oder Enzym oder ein Teilprotein oder -enzym codiert, umfasst und zur Komplementierung des anvisierten Gens unter (a) vorgesehen ist, so dass die Produktion eines funktionsfähigen Proteins in der Zielzelle ermöglicht wird,
wobei das Verhältnis des Gen-Editing-AAV-Vektors (a) zum Targeting-AAV-Vektor (b) solcher Art ist, dass der Targeting-AAV-Vektor (b) im Überschuss zum Gen-Editing-AAV-Vektor (a) vorliegt, wie bezogen auf Genomkopien von (a) und (b) bestimmt.

20. Vektorsystem nach Anspruch 19, wobei das Verhältnis des Gen-Editing-AAV-Vektors (a) zum Targeting-AAV-Vektor (b) bei 1:3 bis 1:100 oder 1:10 liegt, wie bezogen auf Genomkopien von (a) und (b) bestimmt.

21. Vektorsystem nach Anspruch 19, wobei die sgRNA des Targeting-AAV-Vektors (b) 24 Nukleotide bis 28 Nukleotide umfasst.

## Revendications

1. Système de vecteur double comprenant :
(a) un vecteur AAV d'édition génique comprenant un gène Cas9 sous le contrôle de séquences régulatrices qui dirigent l'expression de la Cas9 dans une cellule cible comprenant un gène ciblé qui possède une ou plusieurs mutations entraînant un trouble ;
(b) un vecteur AAV de ciblage comprenant un ou plusieurs ARNsg et une matrice donneuse, le ou les ARNsg comprenant individuellement au moins 20 nucléotides qui se lient spécifiquement à un site choisi dans les gènes ciblés et lequel site choisi est en 5' par rapport à un motif de reconnaissance du proto-espaceur (PAM) qui est spécifiquement reconnu par la Cas9, et la matrice donneuse comprenant des séquences d'acides nucléiques qui remplacent au moins l'une des mutations dans le gène ciblé ;
le rapport du vecteur AAV d'édition génique (a) sur le vecteur AAV de ciblage (b) étant tel que le vecteur AAV de ciblage (b) est en excès par rapport au vecteur AAV d'édition génique (a), tel que déterminé sur la base de copies du génome du vecteur de (a) et (b), et les cellules cibles étant des hépatocytes ou des cellules hépatiques.

2. Système de vecteur double selon la revendication 1, le rapport du vecteur AAV d'édition génique (a) sur le vecteur AAV de ciblage (b) étant de 1 : 3 à 1 : 100, ou 1 : 10, tel que déterminé sur la base de copies du génome de (a) et (b).

3. Système de vecteur double selon la revendication 1, l'ARNsg du vecteur AAV de ciblage (b) comprenant 24 nucléotides à 28 nucléotides.

4. Système de vecteur double selon la revendication 1, la matrice donneuse du vecteur AAV de ciblage (b) étant un gène pleine longueur codant pour une protéine ou enzyme fonctionnelle, ou la matrice donneuse du vecteur AAV de ciblage (b) codant pour une protéine ou enzyme partielle et étant conçue pour complémenter un gène défectueux et pour permettre la production d'une protéine fonctionnelle dans la cellule cible.

5. Système de vecteur double selon la revendication 1, l'ARNsg du vecteur AAV de ciblage (b) étant conçu pour cibler un intron du gène ciblé, de sorte que la mutation soit corrigée par le donneur.

6. Système de vecteur double selon la revendication 1, l'ARNsg du vecteur AAV de ciblage (b) étant conçu pour cibler un gène, de sorte que la matrice donneuse soit insérée en amont du gène défectueux.

7. Système de vecteur double selon la revendication 1, le vecteur AAV de ciblage (b) comprenant plus d'un ARNsg.

8. Système de vecteur double selon la revendication 1, le vecteur AAV d'édition génique de (a) et le vecteur AAV de ciblage de (b) possédant la même capside AAV.

9. Système de vecteur double selon la revendication 8, la capside AAV étant choisie parmi AAV8, AAV9, rh10, AAV6.2, AAV3B, hu37, et/ou rh64.

10. Système de vecteur double selon la revendication 1, la Cas9 étant choisie parmi une Cas9 de *Staphylococcus aureus* ou de *Streptococcus pyogenes.*

11. Système de vecteur double selon la revendication 1, le gène Cas9 étant sous le contrôle d'un promoteur spécifique à un tissu, éventuellement le promoteur spécifique à un tissu étant un promoteur spécifique au foie, éventuellement le promoteur spécifique au foie étant un promoteur de globuline liant la thyroxine (TBG) humaine.

12. Système de vecteur double selon la revendication 1, le gène Cas9 étant sous le contrôle d'un promoteur constitutif.

13. Système de vecteur double selon l'une quelconque des revendications 1 à 12 pour une utilisation dans un procédé de traitement d'un trouble chez des êtres humains.

14. Système de vecteur double pour une utilisation dans un procédé de traitement d'un trouble métabolique du foie chez des nouveau-nés, comprenant :
(a) un vecteur AAV d'édition génique comprenant un gène Cas9 sous le contrôle de séquences régulatrices qui dirigent l'expression de la Cas9 dans un hépatocyte comprenant un gène ciblé qui possède une ou plusieurs mutations entraînant un trouble métabolique du foie ; et
(b) un vecteur AAV de ciblage comprenant un ARNsg et une matrice donneuse, l'ARNsg comprenant au moins 20 nucléotides qui se lient spécifiquement à un site choisi dans les gènes ciblés et lequel site choisi est en 5' par rapport à un motif de reconnaissance du proto-espaceur (PAM) qui est spécifiquement reconnu par la Cas9, et la matrice donneuse comprenant des séquences d'acides nucléiques qui remplacent au moins l'une des mutations dans le gène ciblé ;
le rapport de copies du génome du vecteur AAV d'édition génique (a) sur les copies du génome du vecteur AAV de ciblage (b) étant tel que les copies du génome du vecteur AAV de ciblage (b) sont en excès par rapport au vecteur AAV d'édition génique (a).

15. Système de vecteur double selon la revendication 14, le vecteur AAV d'édition génique (a) et le vecteur AAV de ciblage (b) étant administrés essentiellement simultanément via la même voie.

16. Système de vecteur double selon la revendication 14, le vecteur AAV d'édition génique (a) étant mis en suspension dans un véhicule pour injection à une concentration de 2 × 10¹¹ GC/mL à 2 × 10¹² GC/mL, et/ou le vecteur AAV de ciblage (b) étant mis en suspension dans un véhicule pour injection à une concentration d'environ 2 × 10¹² GC/mL à environ 1 × 10¹³ GC/mL.

17. Système de vecteur double selon la revendication 14, le trouble métabolique du foie étant l'ornithine transcarbamylase (OTC), un gène OTC ayant une mutation.

18. Système de vecteur double selon la revendication 17, l'ARNsg du vecteur AAV de ciblage (b) étant ciblé vers un site de mutation G/A dans l'exon4 du gène OTC, situé au niveau du nucléotide 243 sur la base de la numérotation du gène OTC de type sauvage.

19. Système de vecteur double comprenant :
(a) un vecteur AAV d'édition génique comprenant un gène Cas9 sous le contrôle de séquences régulatrices qui dirigent l'expression de la Cas9 dans une cellule hépatique ou un hépatocyte comprenant un gène ciblé qui possède une ou plusieurs mutations entraînant un trouble ;
(b) un vecteur AAV de ciblage comprenant un ou plusieurs ARNsg et une matrice donneuse, le ou les ARNsg comprenant individuellement au moins 20 nucléotides qui se lient spécifiquement à un site choisi dans le gène ciblé, et lequel site choisi est en 5' par rapport à un motif de reconnaissance du proto-espaceur (PAM) qui est spécifiquement reconnu par la Cas9, et la matrice donneuse comprenant un gène pleine longueur codant pour une protéine ou enzyme fonctionnelle ou une protéine ou enzyme partielle et étant conçue pour complémenter le gène ciblé de (a) pour permettre la production d'une protéine fonctionnelle dans la cellule cible,
le rapport du vecteur AAV d'édition génique (a) sur le vecteur AAV de ciblage (b) étant tel que le vecteur AAV de ciblage (b) est en excès par rapport au vecteur AAV d'édition génique (a), tel que déterminé sur la base de copies du génome de (a) et (b).

20. Système de vecteur selon la revendication 19, le rapport du vecteur AAV d'édition génique (a) sur le vecteur AAV de ciblage (b) étant de 1 : 3 à 1 : 100, ou 1 : 10, tel que déterminé sur la base de copies du génome de (a) et (b).

21. Système de vecteur selon la revendication 19, l'ARNsg du vecteur AAV de ciblage (b) comprenant 24 nucléotides à 28 nucléotides.


### SEQUENCE LISTING

<110> The Trustees of the University of Pennsylvania
<120> DUAL AAV VECTOR SYSTEM FOR CRISPR/Cas9 MEDIATED CORRECTION OF HUMAN DISEASE
<130> UPN-15-7506PCT
<150> US 62/153,470
   <151> 2015-04-27
<150> US 62/183,825
   <151> 2015-06-24
<150> US 62/254,225
   <151> 2015-11-12
<150> US 62/287,511
   <151> 2016-01-27
<160> 198
<170> PatentIn version 3.5
<210> 1
   <211> 120
   <212> DNA
   <213> Mus musculus
<220>
   <221> misc_feature
   <222> (4)..(23)
   <223> sgRNA2
<220>
   <221> misc_feature
   <222> (24)..(29)
   <223> PAM
<220>
   <221> misc_feature
   <222> (52)..(52)
   <223> spfash mutation site G->A
<220>
   <221> misc_feature
   <222> (77)..(96)
   <223> sgRNA3
<220>
   <221> misc_feature
   <222> (91)..(96)
   <223> PAM (reverse complement sequence)
<220>
   <221> misc_feature
   <222> (97)..(102)
   <223> PAM
<220>
   <221> misc_feature
   <222> (97)..(116)
   <223> sgRNA1 (reverse complement sequence)
<400> 1
   ccacacaaga cattcacttg ggtgtgaatg aaagtctcac agacaccgct cggtttgtaa 60
   aacttttctt ccttccaaag tttatttcaa actctgatgg gttagtttaa aagagaagat 120
<210> 2
   <211> 157
   <212> DNA
   <213> Mus musculus
<220>
   <221> misc_feature
   <222> (3)..(21)
   <223> sgRNA1
<220>
   <221> misc_feature
   <222> (22)..(27)
   <223> PAM
<220>
   <221> misc_feature
   <222> (98)..(103)
   <223> PAM (reverse complement sequence)
<220>
   <221> misc_feature
   <222> (102)..(104)
   <223> W392X mutation:TGG->TGA
<220>
   <221> misc_feature
   <222> (104)..(123)
   <223> sgRNA3 (reverse complement sequence)
<220>
   <221> misc_feature
   <222> (130)..(149)
   <223> sgRNA2
<220>
   <221> misc_feature
   <222> (150)..(155)
   <223> PAM
<400> 2
   caaatggtgg gagctagata ttagggtagg aagccagatg ctaggtatga gagagccaac 60
   agcctcagcc ctctgcttgg cttatagatg gagaacaact ctgggcagag gtctcaaagg 120
   ctggggctgt gttggacagc aatcatacag tgggtgt 157
<210> 3
   <211> 176
   <212> DNA
   <213> Artificial Sequence
<220>
   <223> TBG variant TBG-S1
<400> 3
   actcaaagtt caaaccttat cattttttgc tttgttcctc ttggccttgg ttttgtacat 60
   cagctttgaa aataccatcc cagggttaat gctggggtta atttataact aagagtgctc     120
   tagttttgca atacaggaca tgctataaaa atggaaagat gttgctttct gagaga 176
<210> 4
   <211> 80
   <212> DNA
   <213> Artificial Sequence
<220>
   <223> novel enhancer ABPS2
<400> 4
   gttaattttt aaactgtttg ctctggttaa taatctcagg aggttaattt ttaaactgtt 60
   tgctctggtt aataatctca 80
<210> 5
   <211> 20
   <212> DNA
   <213> Artificial Sequence
<220>
   <223> target sequence
<400> 5
   agtttgaaat aaactttgga 20
<210> 6
   <211> 20
   <212> DNA
   <213> Artificial Sequence
<220>
   <223> target sequence
<400> 6
   gaaaagtttt acaaactgag 20
<210> 7
   <211> 20
   <212> DNA
   <213> Artificial Sequence
<220>
   <223> target sequence
<400> 7
   tcagagtttg aaataaactt 20
<210> 8
   <211> 20
   <212> DNA
   <213> Artificial Sequence
<220>
   <223> target sequence
<400> 8
   tctcttttaa actaacccat 20
<210> 9
   <211> 20
   <212> DNA
   <213> Artificial Sequence
<220>
   <223> target sequence
<400> 9
   cacaagacat tcacttgggt 20
<210> 10
   <211> 20
   <212> DNA
   <213> Artificial Sequence
<220>
   <223> target sequence
<400> 10
   aaagtttatt tcaaactctg 20
<210> 11
   <211> 92
   <212> DNA
   <213> Mus musculus
<400> 11
   gaaagtctca cagacaccgc tcggtttgta aaacttttct tccttccaaa gtttatttca 60
   aactctgatg ggttagttta aaagagaaga tg 92
<210> 12
   <211> 92
   <212> DNA
   <213> Artificial Sequence
<220>
   <223> spfash G->A mutation sequence
<400> 12
   gaaagtctca cagacaccgc tcagtttgta aaacttttct tccttccaaa gtttatttca 60
   aactctgatg ggttagttta aaagagaaga tg 92
<210> 13
   <211> 91
   <212> DNA
   <213> Artificial Sequence
<220>
   <223> OTC donor sequence (SpCas9, 892-983 bp)
<400> 13
   aaagtctcac agacaccgct cggtttgtaa aacttttctt ccttgcaaag tttatttcaa 60
   actctgatgg gttagtttaa aagagaagat g 91
<210> 14
   <211> 98
   <212> DNA
   <213> Artificial Sequence
<220>
   <223> OTC donor sequence (SaCas9, 892-989 bp)
<400> 14
   gaaagtctca cagacaccgc tcggtttgta aaacttttct tccttccaaa gtttaccggt 60
   atttcaattg gtgatgggtt agtttaaaag agaagatg 98
<210> 15
   <211> 25
   <212> DNA
   <213> Artificial Sequence
<220>
   <223> OTC sgRNA1_Fwd
<400> 15
   caccgtctct tttaaactaa cccat 25
<210> 16
   <211> 25
   <212> DNA
   <213> Artificial Sequence
<220>
   <223> OTC sgRNA1_Rev
<400> 16
   aaacatgggt tagtttaaaa gagac 25
<210> 17
   <211> 25
   <212> DNA
   <213> Artificial Sequence
<220>
   <223> OTC sgRNA2_Fwd
<400> 17
   caccgcacaa gacattcact tgggt 25
<210> 18
   <211> 25
   <212> DNA
   <213> Artificial Sequence
<220>
   <223> OTC sgRNA2_Rev
<400> 18
   aaacacccaa gtgaatgtct tgtgc 25
<210> 19
   <211> 25
   <212> DNA
   <213> Artificial Sequence
<220>
   <223> OTC sgRNA3_Fwd
<400> 19
   caccgaaagt ttatttcaaa ctctg 25
<210> 20
   <211> 25
   <212> DNA
   <213> Artificial Sequence
<220>
   <223> OTC sgRNA3_Rev
<400> 20
   aaaccagagt ttgaaataaa ctttc 25
<210> 21
   <211> 21
   <212> DNA
   <213> Artificial Sequence
<220>
   <223> HDR-Fwd
<400> 21
   tggagcaatt ctgcacatgg a 21
<210> 22
   <211> 23
   <212> DNA
   <213> Artificial Sequence
<220>
   <223> HDR-Rev
<400> 22
   cttactgaac atggcagttt ccc 23
<210> 23
   <211> 21
   <212> DNA
   <213> Artificial Sequence
<220>
   <223> OTC_PointM_F
<400> 23
   ggctatgctt gggaatgtcc t 21
<210> 24
   <211> 22
   <212> DNA
   <213> Artificial Sequence
<220>
   <223> OTC_PointM_R
<400> 24
   gctacagaat gaaagagagg cg 22
<210> 25
   <211> 25
   <212> DNA
   <213> Artificial Sequence
<220>
   <223> mOTC hSpCas9 sgRNA1_Fwd
<400> 25
   caccgagttt gaaataaact ttgga 25
<210> 26
   <211> 25
   <212> DNA
   <213> Artificial Sequence
<220>
   <223> mOTC hSpCas9 sgRNA1_Rev
<400> 26
   aaactccaaa gtttatttca aactc 25
<210> 27
   <211> 24
   <212> DNA
   <213> Artificial Sequence
<220>
   <223> mOTC hSpCas9 sgRNA2_Fwd
<400> 27
   caccgaaaag ttttacaaac tgag 24
<210> 28
   <211> 24
   <212> DNA
   <213> Artificial Sequence
<220>
   <223> mOTC hSpCas9 sgRNA2_Rev
<400> 28
   aaacctcagt ttgtaaaact tttc 24
<210> 29
   <211> 25
   <212> DNA
   <213> Artificial Sequence
<220>
   <223> mOTC hSpCas9 sgRNA3_Fwd
<400> 29
   caccgtcaga gtttgaaata aactt 25
<210> 30
   <211> 25
   <212> DNA
   <213> Artificial Sequence
<220>
   <223> mOTC hSpCas9 sgRNA3_Rev
<400> 30
   aaacaagttt atttcaaact ctgac 25
<210> 31
   <211> 25
   <212> DNA
   <213> Artificial Sequence
<220>
   <223> mOTC hSaCas9 sgRNA1_Fwd
<400> 31
   caccgtctct tttaaactaa cccat 25
<210> 32
   <211> 25
   <212> DNA
   <213> Artificial Sequence
<220>
   <223> mOTC hSaCas9 sgRNA1_Rev
<400> 32
   aaacatgggt tagtttaaaa gagac 25
<210> 33
   <211> 25
   <212> DNA
   <213> Artificial Sequence
<220>
   <223> mOTC hSaCas9 sgRNA2_Fwd
<400> 33
   caccgcacaa gacattcact tgggt 25
<210> 34
   <211> 25
   <212> DNA
   <213> Artificial Sequence
<220>
   <223> mOTC hSaCas9 sgRNA2_Rev
<400> 34
   aaacacccaa gtgaatgtct tgtgc 25
<210> 35
   <211> 25
   <212> DNA
   <213> Artificial Sequence
<220>
   <223> mOTC hSaCas9 sgRNA3_Fwd
<400> 35
   caccgaaagt ttatttcaaa ctctg 25
<210> 36
   <211> 25
   <212> DNA
   <213> Artificial Sequence
<220>
   <223> mOTC hSaCas9 sgRNA3_Rev
<400> 36
   aaaccagagt ttgaaataaa ctttc 25
<210> 37
   <211> 21
   <212> DNA
   <213> Artificial Sequence
<220>
   <223> OTC_OT1F1
<400> 37
   ctggtgcctt tttctatcgc c 21
<210> 38
   <211> 22
   <212> DNA
   <213> Artificial Sequence
<220>
   <223> OTC_OT1R1
<400> 38
   ccaagagcaa ctacaatggc tt 22
<210> 39
   <211> 20
   <212> DNA
   <213> Artificial Sequence
<220>
   <223> OTC_OT1F2
<400> 39
   gcattttcat gagcattcca 20
<210> 40
   <211> 20
   <212> DNA
   <213> Artificial Sequence
<220>
   <223> OTC_OT1R2
<400> 40
   catgttgtgc ctgcatctct 20
<210> 41
   <211> 21
   <212> DNA
   <213> Artificial Sequence
<220>
   <223> OTC_OT2F1
<400> 41
   gcagactcca agatgcaaga c 21
<210> 42
   <211> 21
   <212> DNA
   <213> Artificial Sequence
<220>
   <223> OTC_OT2R1
<400> 42
   gatgttgttc cacccgcatc t 21
<210> 43
   <211> 20
   <212> DNA
   <213> Artificial Sequence
<220>
   <223> OTC_OT2F2
<400> 43
   cactgagcca agtcactgga 20
<210> 44
   <211> 20
   <212> DNA
   <213> Artificial Sequence
<220>
   <223> OTC_OT2R2
<400> 44
   agggacaaaa ccaaacagca 20
<210> 45
   <211> 20
   <212> DNA
   <213> Artificial Sequence
<220>
   <223> OTC_OT3F1
<400> 45
   tggccttcta aagcaaccaa 20
<210> 46
   <211> 21
   <212> DNA
   <213> Artificial Sequence
<220>
   <223> OTC_OT3R1
<400> 46
   ccgtctccca gatcacatga c 21
<210> 47
   <211> 27
   <212> DNA
   <213> Artificial Sequence
<220>
   <223> OTC_OT3F2
<400> 47
   ataactcata atctatgcat ggcacaa 27
<210> 48
   <211> 24
   <212> DNA
   <213> Artificial Sequence
<220>
   <223> OTC_OT3R2
<400> 48
   tttgatcatg gtgtttatca gagc 24
<210> 49
   <211> 20
   <212> DNA
   <213> Artificial Sequence
<220>
   <223> OTC_OT4F1
<400> 49
   gtccccgaca aaccaagcta 20
<210> 50
   <211> 20
   <212> DNA
   <213> Artificial Sequence
<220>
   <223> OTC_OT4R1
<400> 50
   tgaactggca gtatgcaggg 20
<210> 51
   <211> 20
   <212> DNA
   <213> Artificial Sequence
<220>
   <223> OTC_OT4F2
<400> 51
   aacatggttt ctgccctcag 20
<210> 52
   <211> 20
   <212> DNA
   <213> Artificial Sequence
<220>
   <223> OTC_OT4R2
<400> 52
   ggaccatgcc gaactcttac 20
<210> 53
   <211> 20
   <212> DNA
   <213> Artificial Sequence
<220>
   <223> OTC_OT5F1
<400> 53
   ttgagaccta gctcatgccc 20
<210> 54
   <211> 20
   <212> DNA
   <213> Artificial Sequence
<220>
   <223> OTC_OT5R1
<400> 54
   taacgcagaa ctggcacagg 20
<210> 55
   <211> 20
   <212> DNA
   <213> Artificial Sequence
<220>
   <223> OTC_OT5F2
<400> 55
   ctctcccatg gagaaagctg 20
<210> 56
   <211> 20
   <212> DNA
   <213> Artificial Sequence
<220>
   <223> OTC_OT5R2
<400> 56
   gaatgtggca ttggcttttt 20
<210> 57
   <211> 20
   <212> DNA
   <213> Artificial Sequence
<220>
   <223> OTC_OT6F1
<400> 57
   gagagagcca atctgcccat 20
<210> 58
   <211> 20
   <212> DNA
   <213> Artificial Sequence
<220>
   <223> OTC_OT6R1
<400> 58
   caccggaaac gtgtgagaga 20
<210> 59
   <211> 20
   <212> DNA
   <213> Artificial Sequence
<220>
   <223> OTC_OT6F2
<400> 59
   acttcccatg atcccattga 20
<210> 60
   <211> 20
   <212> DNA
   <213> Artificial Sequence
<220>
   <223> OTC_OT6R2
<400> 60
   agcttccctc caagtgtcct 20
<210> 61
   <211> 21
   <212> DNA
   <213> Artificial Sequence
<220>
   <223> OTC_OT7F1
<400> 61
   gatgggcata agcccgaagt a 21
<210> 62
   <211> 21
   <212> DNA
   <213> Artificial Sequence
<220>
   <223> OTC_OT7R1
<400> 62
   taaggcccag tgttgttgtg t 21
<210> 63
   <211> 20
   <212> DNA
   <213> Artificial Sequence
<220>
   <223> OTC_OT7F2
<400> 63
   gtagcagggg ctctgtgaag 20
<210> 64
   <211> 20
   <212> DNA
   <213> Artificial Sequence
<220>
   <223> OTC_OT7R2
<400> 64
   tggcctgaaa tacccagaac 20
<210> 65
   <211> 20
   <212> DNA
   <213> Artificial Sequence
<220>
   <223> OTC_OT8F1
<400> 65
   gttgaattcg cgtgtccagg 20
<210> 66
   <211> 20
   <212> DNA
   <213> Artificial Sequence
<220>
   <223> OTC_OT8R1
<400> 66
   tcccatggcg agaatgtcac 20
<210> 67
   <211> 21
   <212> DNA
   <213> Artificial Sequence
<220>
   <223> OTC_OT8F2
<400> 67
   ccctgtagga aacacagagg a 21
<210> 68
   <211> 22
   <212> DNA
   <213> Artificial Sequence
<220>
   <223> OTC_OT8R2
<400> 68
   tgctttggat gttgattcta aa 22
<210> 69
   <211> 20
   <212> DNA
   <213> Artificial Sequence
<220>
   <223> OTC_OT9F1
<400> 69
   ggcaaaggac tagcttgcac 20
<210> 70
   <211> 20
   <212> DNA
   <213> Artificial Sequence
<220>
   <223> OTC_OT9R1
<400> 70
   gggtgctatg aggaccagtg 20
<210> 71
   <211> 20
   <212> DNA
   <213> Artificial Sequence
<220>
   <223> OTC_OT9F2
<400> 71
   cagtggtgtg tggagagctg 20
<210> 72
   <211> 21
   <212> DNA
   <213> Artificial Sequence
<220>
   <223> OTC_OT9R2
<400> 72
   agagagagcg cttgactttg a 21
<210> 73
   <211> 20
   <212> DNA
   <213> Artificial Sequence
<220>
   <223> OTC_OT10F1
<400> 73
   ctttgactcc cggcgaaaga 20
<210> 74
   <211> 20
   <212> DNA
   <213> Artificial Sequence
<220>
   <223> OTC_OT10R1
<400> 74
   ttgtccatcc gggtcattgc 20
<210> 75
   <211> 20
   <212> DNA
   <213> Artificial Sequence
<220>
   <223> OTC_OT10F2
<400> 75
   aagtccttct tgcccaactt 20
<210> 76
   <211> 18
   <212> DNA
   <213> Artificial Sequence
<220>
   <223> OTC_OT10R2
<400> 76
   cagccccaat gcattttt 18
<210> 77
   <211> 20
   <212> DNA
   <213> Artificial Sequence
<220>
   <223> OTC_OT11F1
<400> 77
   attacaggtc ctggttgggc 20
<210> 78
   <211> 20
   <212> DNA
   <213> Artificial Sequence
<220>
   <223> OTC_OT11R1
<400> 78
   actgagcctg gtagagcctt 20
<210> 79
   <211> 20
   <212> DNA
   <213> Artificial Sequence
<220>
   <223> OTC_OT11F2
<400> 79
   ggaaggtgaa ggaaggaagg 20
<210> 80
   <211> 24
   <212> DNA
   <213> Artificial Sequence
<220>
   <223> OTC_OT11R2
<400> 80
   ttttctagga attcaggaca taca 24
<210> 81
   <211> 22
   <212> DNA
   <213> Artificial Sequence
<220>
   <223> OTC_OT12F
<400> 81
   tcatggtcct taaaattttt gc 22
<210> 82
   <211> 20
   <212> DNA
   <213> Artificial Sequence
<220>
   <223> OTC_OT12R
<400> 82
   tccaggtatg caaagtggat 20
<210> 83
   <211> 25
   <212> DNA
   <213> Artificial Sequence
<220>
   <223> OTC_OT13F1
<400> 83
   ttcagttgta ctttggatgc tctga 25
<210> 84
   <211> 20
   <212> DNA
   <213> Artificial Sequence
<220>
   <223> OTC_OT13R1
<400> 84
   catctgaata gcagcaggcg 20
<210> 85
   <211> 20
   <212> DNA
   <213> Artificial Sequence
<220>
   <223> OTC_OT13F2
<400> 85
   tagcacagcc caaatgactt 20
<210> 86
   <211> 22
   <212> DNA
   <213> Artificial Sequence
<220>
   <223> OTC_OT13R2
<400> 86
   tcatgaaacc ccataatcag aa 22
<210> 87
   <211> 22
   <212> DNA
   <213> Artificial Sequence
<220>
   <223> OTC_OT14F1
<400> 87
   agtgggtcat cctttgttac cc 22
<210> 88
   <211> 20
   <212> DNA
   <213> Artificial Sequence
<220>
   <223> OTC_OT14R1
<400> 88
   tgccagttat cagccaagca 20
<210> 89
   <211> 21
   <212> DNA
   <213> Artificial Sequence
<220>
   <223> OTC_OT14F2
<400> 89
   cccaggaact taactcaggt g 21
<210> 90
   <211> 22
   <212> DNA
   <213> Artificial Sequence
<220>
   <223> OTC_OT14R2
<400> 90
   tgccatttga cctcataagt ct 22
<210> 91
   <211> 21
   <212> DNA
   <213> Artificial Sequence
<220>
   <223> OTC_OT15F1
<400> 91
   ttcagccccc ttgagtgttt a 21
<210> 92
   <211> 22
   <212> DNA
   <213> Artificial Sequence
<220>
   <223> OTC_OT15R1
<400> 92
   gtctctgagc acaaagagac ga 22
<210> 93
   <211> 20
   <212> DNA
   <213> Artificial Sequence
<220>
   <223> OTC_OT15F2
<400> 93
   ttgcctgtcc caactagagc 20
<210> 94
   <211> 20
   <212> DNA
   <213> Artificial Sequence
<220>
   <223> OTC_OT15R2
<400> 94
   ggcccaagaa tgcacattta 20
<210> 95
   <211> 20
   <212> DNA
   <213> Artificial Sequence
<220>
   <223> OTC_OT16F1
<400> 95
   ccacacactg gctaggactg 20
<210> 96
   <211> 20
   <212> DNA
   <213> Artificial Sequence
<220>
   <223> OTC_OT16R1
<400> 96
   actggcagca cttgagacaa 20
<210> 97
   <211> 20
   <212> DNA
   <213> Artificial Sequence
<220>
   <223> OTC_OT16F2
<400> 97
   gatggcatgc tgtggttttt 20
<210> 98
   <211> 20
   <212> DNA
   <213> Artificial Sequence
<220>
   <223> OTC_OT16R2
<400> 98
   caatgcttcc acacagaacc 20
<210> 99
   <211> 20
   <212> DNA
   <213> Artificial Sequence
<220>
   <223> sgRNA1
<400> 99
   tctcttttaa actaacccat 20
<210> 100
   <211> 20
   <212> DNA
   <213> Artificial Sequence
<220>
   <223> OT1
<400> 100
   tatcttttca actaacccaa 20
<210> 101
   <211> 20
   <212> DNA
   <213> Artificial Sequence
<220>
   <223> OT2
<400> 101
   tttcttttat actagcccat 20
<210> 102
   <211> 20
   <212> DNA
   <213> Artificial Sequence
<220>
   <223> OT3
<400> 102
   tcttttttta aataacccat 20
<210> 103
   <211> 20
   <212> DNA
   <213> Artificial Sequence
<220>
   <223> OT4
<400> 103
   tggcatttaa actaacccaa 20
<210> 104
   <211> 20
   <212> DNA
   <213> Artificial Sequence
<220>
   <223> OT5
<400> 104
   tcttcatgaa actaacccat 20
<210> 105
   <211> 20
   <212> DNA
   <213> Artificial Sequence
<220>
   <223> OT6
<400> 105
   atttttttaa acaaacccat 20
<210> 106
   <211> 20
   <212> DNA
   <213> Artificial Sequence
<220>
   <223> OT7
<400> 106
   gatcttgtaa tctaacccat 20
<210> 107
   <211> 20
   <212> DNA
   <213> Artificial Sequence
<220>
   <223> OT8
<400> 107
   ccttttataa tctaacccat 20
<210> 108
   <211> 20
   <212> DNA
   <213> Artificial Sequence
<220>
   <223> OT9
<400> 108
   tctgttttaa agtaaccctt 20
<210> 109
   <211> 20
   <212> DNA
   <213> Artificial Sequence
<220>
   <223> OT10
<400> 109
   ttgcttttca actaacccaa 20
<210> 110
   <211> 20
   <212> DNA
   <213> Artificial Sequence
<220>
   <223> OT11
<400> 110
   aatattttaa actatcccat 20
<210> 111
   <211> 20
   <212> DNA
   <213> Artificial Sequence
<220>
   <223> OT12
<400> 111
   ttactttaaa actatcccat 20
<210> 112
   <211> 20
   <212> DNA
   <213> Artificial Sequence
<220>
   <223> OT13
<400> 112
   acacttttaa aataacccag 20
<210> 113
   <211> 20
   <212> DNA
   <213> Artificial Sequence
<220>
   <223> OT14
<400> 113
   aatcttctaa accaacccat 20
<210> 114
   <211> 20
   <212> DNA
   <213> Artificial Sequence
<220>
   <223> OT15
<400> 114
   tctttattag actaacccag 20
<210> 115
   <211> 20
   <212> DNA
   <213> Artificial Sequence
<220>
   <223> OT16
<400> 115
   tttcatttaa cataacccat 20
<210> 116
   <211> 20
   <212> DNA
   <213> Artificial Sequence
<220>
   <223> OT17
<400> 116
   tctttttaga agtaacccat 20
<210> 117
   <211> 20
   <212> DNA
   <213> Artificial Sequence
<220>
   <223> OT18
<400> 117
   tccctttttc actaacccac 20
<210> 118
   <211> 20
   <212> DNA
   <213> Artificial Sequence
<220>
   <223> OT19
<400> 118
   tctttgtaaa attaacccat 20
<210> 119
   <211> 20
   <212> DNA
   <213> Artificial Sequence
<220>
   <223> OT20
<400> 119
   tttattttaa agtaacccac 20
<210> 120
   <211> 20
   <212> DNA
   <213> Artificial Sequence
<220>
   <223> OT21
<400> 120
   tatctttcaa aataacccac 20
<210> 121
   <211> 20
   <212> DNA
   <213> Artificial Sequence
<220>
   <223> OT22
<400> 121
   tatcttggaa actaacccct 20
<210> 122
   <211> 20
   <212> DNA
   <213> Artificial Sequence
<220>
   <223> OT23
<400> 122
   cttcttttaa acaaacccag 20
<210> 123
   <211> 20
   <212> DNA
   <213> Artificial Sequence
<220>
   <223> OT24
<400> 123
   aatcttttat actaaccaat 20
<210> 124
   <211> 20
   <212> DNA
   <213> Artificial Sequence
<220>
   <223> OT25
<400> 124
   tattttttaa attaactcat 20
<210> 125
   <211> 20
   <212> DNA
   <213> Artificial Sequence
<220>
   <223> OT26
<400> 125
   tttattttaa aataacacat 20
<210> 126
   <211> 20
   <212> DNA
   <213> Artificial Sequence
<220>
   <223> OT27
<400> 126
   tctctttcaa actatcccaa 20
<210> 127
   <211> 20
   <212> DNA
   <213> Artificial Sequence
<220>
   <223> OT28
<400> 127
   tatctttaaa aataacacat 20
<210> 128
   <211> 20
   <212> DNA
   <213> Artificial Sequence
<220>
   <223> OT29
<400> 128
   tttctttaaa aataacacat 20
<210> 129
   <211> 20
   <212> DNA
   <213> Artificial Sequence
<220>
   <223> OT30
<400> 129
   gttcttttaa aaaaacccat 20
<210> 130
   <211> 20
   <212> DNA
   <213> Artificial Sequence
<220>
   <223> OT31
<400> 130
   tctgatttaa aataacacat 20
<210> 131
   <211> 20
   <212> DNA
   <213> Artificial Sequence
<220>
   <223> OT32
<400> 131
   cctcttataa cctaacccac 20
<210> 132
   <211> 20
   <212> DNA
   <213> Artificial Sequence
<220>
   <223> OT33
<400> 132
   cctcttgtaa gctaacccac 20
<210> 133
   <211> 20
   <212> DNA
   <213> Artificial Sequence
<220>
   <223> OT34
<400> 133
   cctattttaa agtaaccctt 20
<210> 134
   <211> 20
   <212> DNA
   <213> Artificial Sequence
<220>
   <223> OT35
<400> 134
   catcttgtaa actagcccat 20
<210> 135
   <211> 20
   <212> DNA
   <213> Artificial Sequence
<220>
   <223> OT36
<400> 135
   actcttttac aataacacat 20
<210> 136
   <211> 20
   <212> DNA
   <213> Artificial Sequence
<220>
   <223> OT37
<400> 136
   tctcctttag cctaactcat 20
<210> 137
   <211> 20
   <212> DNA
   <213> Artificial Sequence
<220>
   <223> OT38
<400> 137
   cctctttaaa aataacccct 20
<210> 138
   <211> 20
   <212> DNA
   <213> Artificial Sequence
<220>
   <223> OT39
<400> 138
   tatctttgaa ataaacccat 20
<210> 139
   <211> 20
   <212> DNA
   <213> Artificial Sequence
<220>
   <223> OT40
<400> 139
   tatatttaaa actaagccat 20
<210> 140
   <211> 20
   <212> DNA
   <213> Artificial Sequence
<220>
   <223> OT41
<400> 140
   tgactttaaa actaagccat 20
<210> 141
   <211> 20
   <212> DNA
   <213> Artificial Sequence
<220>
   <223> OT42
<400> 141
   cttcttttca actatcccat 20
<210> 142
   <211> 20
   <212> DNA
   <213> Artificial Sequence
<220>
   <223> OT43
<400> 142
   gctcttataa attaacccag 20
<210> 143
   <211> 20
   <212> DNA
   <213> Artificial Sequence
<220>
   <223> OT44
<400> 143
   tttcttttaa attaagccat 20
<210> 144
   <211> 20
   <212> DNA
   <213> Artificial Sequence
<220>
   <223> OT45
<400> 144
   tctctttgtc acaaacccat 20
<210> 145
   <211> 20
   <212> DNA
   <213> Artificial Sequence
<220>
   <223> OT46
<400> 145
   tttcttctaa gttaacccat 20
<210> 146
   <211> 20
   <212> DNA
   <213> Artificial Sequence
<220>
   <223> OT47
<400> 146
   tcacttccaa actaccccat 20
<210> 147
   <211> 20
   <212> DNA
   <213> Artificial Sequence
<220>
   <223> OT48
<400> 147
   tatcttataa aataacccag 20
<210> 148
   <211> 20
   <212> DNA
   <213> Artificial Sequence
<220>
   <223> OT49
<400> 148
   tcaattttta actatcccat 20
<210> 149
   <211> 25
   <212> DNA
   <213> Artificial Sequence
<220>
   <223> dIDUA_sgRNA1_Fwd
<400> 149
   caccgttctg atgagggctc cgcgg 25
<210> 150
   <211> 25
   <212> DNA
   <213> Artificial Sequence
<220>
   <223> dIDUA_sgRNA1_Rev
<400> 150
   aaacccgcgg agccctcatc agaac 25
<210> 151
   <211> 24
   <212> DNA
   <213> Artificial Sequence
<220>
   <223> dIDUA_sgRNA2_Fwd
<400> 151
   caccgcacct ggtgctcgtg gacg 24
<210> 152
   <211> 24
   <212> DNA
   <213> Artificial Sequence
<220>
   <223> dlDUA_sgRNA2_Rev
<400> 152
   aaaccgtcca cgagcaccag gtgc 24
<210> 153
   <211> 24
   <212> DNA
   <213> Artificial Sequence
<220>
   <223> dIDUA_sgRNA3_Fwd
<400> 153
   caccgggccg cgtccacgag cacc 24
<210> 154
   <211> 24
   <212> DNA
   <213> Artificial Sequence
<220>
   <223> dlDUA_sgRNA3_Rev
<400> 154
   aaacggtgct cgtggacgcg gccc 24
<210> 155
   <211> 24
   <212> DNA
   <213> Artificial Sequence
<220>
   <223> dIDUA_sgRNA4_Fwd
<400> 155
   caccgtccac gagcaccagg tgcg 24
<210> 156
   <211> 24
   <212> DNA
   <213> Artificial Sequence
<220>
   <223> dlDUA_sgRNA4_Rev
<400> 156
   aaaccgcacc tggtgctcgt ggac 24
<210> 157
   <211> 25
   <212> DNA
   <213> Artificial Sequence
<220>
   <223> dIDUA_sgRNA5_Fwd
<400> 157
   caccgtggac gcggcccgcg cgctg 25
<210> 158
   <211> 25
   <212> DNA
   <213> Artificial Sequence
<220>
   <223> dlDUA_sgRNA5_Rev
<400> 158
   aaaccagcgc gcgggccgcg tccac 25
<210> 159
   <211> 24
   <212> DNA
   <213> Artificial Sequence
<220>
   <223> dIDUA_sgRNA6_Fwd
<400> 159
   caccgcttct gatgagggct ccgc 24
<210> 160
   <211> 24
   <212> DNA
   <213> Artificial Sequence
<220>
   <223> dlDUA_sgRNA6_Rev
<400> 160
   aaacgcggag ccctcatcag aagc 24
<210> 161
   <211> 25
   <212> DNA
   <213> Artificial Sequence
<220>
   <223> hCFTR_sgRNA1_Fwd
<400> 161
   caccgaatgg tgccaggcat aatcc 25
<210> 162
   <211> 25
   <212> DNA
   <213> Artificial Sequence
<220>
   <223> hCFTR_sgRNA1_Rev
<400> 162
   aaacggatta tgcctggcac cattc 25
<210> 163
   <211> 25
   <212> DNA
   <213> Artificial Sequence
<220>
   <223> hCFTR_sgRNA2_Fwd
<400> 163
   caccgcaaag catgccaact agaag 25
<210> 164
   <211> 25
   <212> DNA
   <213> Artificial Sequence
<220>
   <223> hCFTR_sgRNA2_Rev
<400> 164
   aaaccttcta gttggcatgc tttgc 25
<210> 165
   <211> 25
   <212> DNA
   <213> Artificial Sequence
<220>
   <223> hCFTR_sgRNA3_Fwd
<400> 165
   caccgaccat taaagaaaat atcat 25
<210> 166
   <211> 25
   <212> DNA
   <213> Artificial Sequence
<220>
   <223> hCFTR_sgRNA3_Rev
<400> 166
   aaacatgata ttttctttaa tggtc 25
<210> 167
   <211> 25
   <212> DNA
   <213> Artificial Sequence
<220>
   <223> hCFTR_sgRNA4_Fwd
<400> 167
   caccgagttt cttacctctt ctagt 25
<210> 168
   <211> 25
   <212> DNA
   <213> Artificial Sequence
<220>
   <223> hCFTR_sgRNA4_Rev
<400> 168
   aaacactaga agaggtaaga aactc 25
<210> 169
   <211> 25
   <212> DNA
   <213> Artificial Sequence
<220>
   <223> mFIXsgRNA1_Fwd
<400> 169
   caccgtttag gatatctact cagta 25
<210> 170
   <211> 25
   <212> DNA
   <213> Artificial Sequence
<220>
   <223> mFIXsgRNA1_Rev
<400> 170
   aaactactga gtagatatcc taaac 25
<210> 171
   <211> 25
   <212> DNA
   <213> Artificial Sequence
<220>
   <223> mFIXsgRNA2_Fwd
<400> 171
   caccgacaaa cctgcacatt cggta 25
<210> 172
   <211> 25
   <212> DNA
   <213> Artificial Sequence
<220>
   <223> mFIXsgRNA2_Rev
<400> 172
   aaactaccga atgtgcaggt ttgtc 25
<210> 173
   <211> 24
   <212> DNA
   <213> Artificial Sequence
<220>
   <223> mFIXsgRNA3_Fwd
<400> 173
   caccgcacct gaacaccgtc atgg 24
<210> 174
   <211> 24
   <212> DNA
   <213> Artificial Sequence
<220>
   <223> mFIXsgRNA3_Rev
<400> 174
   aaacccatga cggtgttcag gtgc 24
<210> 175
   <211> 20
   <212> DNA
   <213> Artificial Sequence
<220>
   <223> mFIX_PointM_F
<400> 175
   cgagggagat ggacaacaat 20
<210> 176
   <211> 20
   <212> DNA
   <213> Artificial Sequence
<220>
   <223> mFIX_PointM_R
<400> 176
   gcaccacaag ccctgtaaat 20
<210> 177
   <211> 24
   <212> DNA
   <213> Artificial Sequence
<220>
   <223> rhFIXsgRNA1_Fwd
<400> 177
   caccgcgtgt gaacatgatc atgg 24
<210> 178
   <211> 24
   <212> DNA
   <213> Artificial Sequence
<220>
   <223> rhFIXsgRNA1_Rev
<400> 178
   aaacccatga tcatgttcac acgc 24
<210> 179
   <211> 25
   <212> DNA
   <213> Artificial Sequence
<220>
   <223> rhFIXsgRNA2_Fwd
<400> 179
   caccgtttag gatatctact cagtg 25
<210> 180
   <211> 25
   <212> DNA
   <213> Artificial Sequence
<220>
   <223> rhFIXsgRNA2_Rev
<400> 180
   aaaccactga gtagatatcc taaac 25
<210> 181
   <211> 25
   <212> DNA
   <213> Artificial Sequence
<220>
   <223> rhFIXsgRNA3_Fwd
<400> 181
   caccgacaaa cctgtacatt cagca 25
<210> 182
   <211> 25
   <212> DNA
   <213> Artificial Sequence
<220>
   <223> rhFIXsgRNA3_Rev
<400> 182
   aaactgctga atgtacaggt ttgtc 25
<210> 183
   <211> 24
   <212> DNA
   <213> Artificial Sequence
<220>
   <223> EGFP-sgRNAF
<400> 183
   caccgtcgtg ctgcttcatg tggt 24
<210> 184
   <211> 24
   <212> DNA
   <213> Artificial Sequence
<220>
   <223> EGFP-sgRNAR
<400> 184
   aaacaccaca tgaagcagca cgac 24
<210> 185
   <211> 24
   <212> DNA
   <213> Artificial Sequence
<220>
   <223> PCSK9_sgRNA1F
<400> 185
   caccgatgct ctgggcaaag acag 24
<210> 186
   <211> 24
   <212> DNA
   <213> Artificial Sequence
<220>
   <223> PCSK9_sgRNA1R
<400> 186
   aaacctgtct ttgcccagag catc 24
<210> 187
   <211> 25
   <212> DNA
   <213> Artificial Sequence
<220>
   <223> PCSK9_sgRNA2F
<400> 187
   caccgatctc ctagacacca gcata 25
<210> 188
   <211> 25
   <212> DNA
   <213> Artificial Sequence
<220>
   <223> PCSK9_sgRNA2R
<400> 188
   aaactatgct ggtgtctagg agatc 25
<210> 189
   <211> 25
   <212> DNA
   <213> Artificial Sequence
<220>
   <223> PCSK9_sgRNA3F
<400> 189
   caccgaagtt ggtccccaaa gtccc 25
<210> 190
   <211> 25
   <212> DNA
   <213> Artificial Sequence
<220>
   <223> PCSK9_sgRNA3R
<400> 190
   aaacgggact ttggggacca acttc 25
<210> 191
   <211> 24
   <212> DNA
   <213> Artificial Sequence
<220>
   <223> PCSK9_sgRNA4F
<400> 191
   caccgcagca cctgctttgt gtca 24
<210> 192
   <211> 24
   <212> DNA
   <213> Artificial Sequence
<220>
   <223> PCSK9_sgRNA4R
<400> 192
   aaactgacac aaagcaggtg ctgc 24
<210> 193
   <211> 25
   <212> DNA
   <213> Artificial Sequence
<220>
   <223> PCSK9_sgRNA5F
<400> 193
   caccgagtct ctgcctcaac tcggc 25
<210> 194
   <211> 25
   <212> DNA
   <213> Artificial Sequence
<220>
   <223> PCSK9_sgRNA5R
<400> 194
   aaacgccgag ttgaggcaga gactc 25
<210> 195
   <211> 25
   <212> DNA
   <213> Artificial Sequence
<220>
   <223> PCSK9_sgRNA6F
<400> 195
   caccgatgac atctttggca gagaa 25
<210> 196
   <211> 25
   <212> DNA
   <213> Artificial Sequence
<220>
   <223> PCSK9_sgRNA6R
<400> 196
   aaacttctct gccaaagatg tcatc 25
<210> 197
   <211> 24
   <212> DNA
   <213> Artificial Sequence
<220>
   <223> PCSK9_sgRNA7F
<400> 197
   caccgcctgg ttccctgagg acca 24
<210> 198
   <211> 24
   <212> DNA
   <213> Artificial Sequence
<220>
   <223> PCSK9_sgRNA7R
<400> 198
   aaactggtcc tcagggaacc aggc 24